# EUROPEAN PATENT APPLICATION

(11) **EP 3 696 177 A1**
(43) Date of publication of application: **19.08.2020**
(21) Application number: 19156695.9
(22) Date of filing: 12.02.2019
(51) Int. Cl.: C07D 413/12, A01N 43/76, A01N 43/78, C07D 417/12

(54) **HETEROCYCLIC COMPOUNDS FOR THE CONTROL OF INVERTEBRATE PESTS**

(71) Applicant: BASF SE, 67056 Ludwigshafen am Rhein (DE)
(72) Inventor: KOERBER, Karsten, 67056 Ludwigshafen (DE); GARZA SANCHEZ, Rosario Aleyda, 67056 Ludwigshafen (DE); SCHROEDER, Birte, 67056 Ludwigshafen (DE); HUWYLER, Nikolas, 67056 Ludwigshafen (DE); NARINE, Arun, 67056 Ludwigshafen (DE)
(74) Representative: BASF IP Association

(57) **Abstract**

The invention relates to compounds of formula I 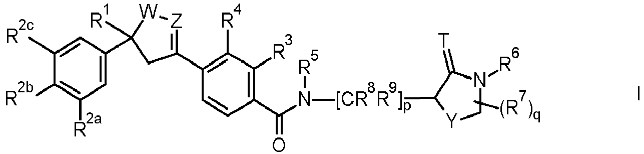 wherein the variables have the meanings as defined in the specification, to compositions comprising them, to active compound combinations comprising them, and to their use for protecting growing plants and animals from attack or infestation by invertebrate pests, furthermore, to seed comprising such compounds.

## Description

The invention relates to compounds of formula I 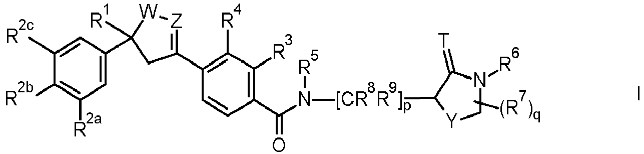 wherein
- W-Z: is -O-N=, -CH₂-N=, or -CH₂-CH=;
- R¹: halomethyl;
- R^{2a}: halogen, halomethyl, or halomethoxy;
- R^{2b}, R^{2c}: are independently H, or as defined for R^{2a};
- R³: is halogen, CN, NO₂, C₁-C₂-alkyl, halomethyl, C₁-C₂-alkoxy, S(O)ₘ-C₁-C₂-alkyl, C₁-C₂-haloalkoxy, or S(O)ₘ-C₁-C₂-haloalkyl;
- R⁴: is H, or as defined for R³; or
- R³ and R⁴: form together with the C-atoms they are bound to a 5-, or 6-membered saturated, partially, or fully unsaturated carbo- or heterocyclic ring;
- R⁵,R⁶ are: independently H, CN, C₁-C₁₀-alkyl, C₃-C₈-cycloalkyl, C₂-C₁₀-alkenyl, C₃-C₈-cycloalkenyl, C₂-C₁₀-alkynyl, OR¹⁰, S(O)ₘR¹⁰, N(R¹⁰)₂, which aliphatic groups are unsubstituted, partially or fully halogenated and/or substituted with one or more R^{a}; phenyl which is unsubstituted or substituted with one or more R^{A}; and 3- to 7-membered saturated, partially or fully unsaturated heterocycle comprising 1, 2 or 3 heteroatoms O, N(O)ₙ or S(O)ₘ as ring members, which heterocycle is unsubstituted or substituted with one or more R^{A},
R¹⁰ is independently H, C₁-C₆-alkyl, C₁-C₆-haloalkyl, C₃-C₈-cycloalkyl, C₃-C₈-cycloalkyl-C₁-C₄-alkyl, C₃-C₈-halocycloalkyl, C₂-C₆-alkenyl, C₂-C₆-haloalkenyl, C₂-C₆-alkynyl, C₂-C₆-haloalkynyl, which groups are unsubstituted or substituted with one or more R^{a},
R^{a} is CN, N₃, NO₂, SCN, SF₅, Si(C₁-C₄-alkyl)₃, OR^{a1}, OSO₂R^{a1}, S(O)ₙR^{a1}, N(R^{a2})R^{a3}, C(=O)N(R^{a2})R^{a3}, C(=S)N(R^{a2})R^{a3}, C(=O)R^{a1}, C(=O)OR^{a1}, CH=NOR^{a1}, C₃-C₈-Cycloalkyl, C₃-C₈-halocycloalkyl, which cyclic moieties may be substituted with R^{a4}; phenyl which is unsubstituted or substituted with one or more R^{A}; and 3- to 7-membered saturated, partially or fully unsaturated heterocycle comprising 1, 2 or 3 heteroatoms O, N(O)ₙ or S(O)ₘ as ring members, which heterocycle is unsubstituted or substituted with one or more R^{A},
m is 0, 1, or 2;
n,p independently are 0, or 1;
R^{a1} H, C₁-C₆-alkyl, C₁-C₆-haloalkyl, C₂-C₄-alkenyl, C₂-C₄-alkynyl, CH₂-CN, C₃-C₆-cycloalkyl, C₃-C₆-halocycloalkyl, C₃-C₆-cycloalkylmethyl, C₃-C₆-halocycloalkylmethyl, phenyl and hetaryl which aromatic rings are unsubstituted or partially or fully substituted with R^{A};
R^{a2} is H, or C₁-C₆-alkyl,
R^{a3} is H, C₁-C₆-alkyl, C₁-C₆-haloalkyl, C₂-C₆-alkenyl, C₂-C₆-haloalkenyl, C₂-C₆-alkynyl, C₂-C₆-haloalkynyl, or C₃-C₆-cycloalkyl, C₃-C₆-halocycloalkyl, C₃-C₆-cycloalkylmethyl, or C₃-C₆-halocycloalkylmethyl which rings are unsubstituted or substituted with a cyano;
R^{a4} is independently OH, CN, C₁-C₆-alkoxy, C₁-C₆-haloalkoxy, S(O)ₘ-C₁-C₆-alkyl, S(O)ₘ-C₁-C₆-haloalkyl, C(=O)N(R^{a2})R^{a3}, C₃-C₆-cycloalkyl, or C₃-C₆-halocycloalkyl which cycles are unsubstituted or substituted with one or more R^{a11}; or phenyl, partially or fully unsaturated heterocycle which rings are unsubstituted or substituted with one or more R^{A};
R^{a11} is independently OH, CN, C₁-C₂-alkyl, or C₁-C₂-haloalkyl;
R^{A} is independently selected from halogen, CN, NO₂, C₁-C₄-alkyl, C₁-C₄-haloalkyl, C2-C4-alkenyl, C2-C4-haloalkenyl, C2-C4-alkynyl, C2-C4-haloalkynyl, C₃-C₆-cycloalkyl, C₃-C₆-halocycloalkyl, C₁-C₄-alkoxy, C₁-C₄-haloalkoxy, S(O)ₘ-C₁-C₄-alkyl, S(O)ₘ-C₁-C₄-haloalkyl, N(R^{a2})R^{a3}, N(R^{a2})C(=O)R^{a3}, C₁-C₄-alkylcarbonyl, C₁-C₄-haloalkylcarbonyl, C(=O)N(R^{a2})R^{a3}; or
two R^{A} present on the same carbon atom of a saturated or partially saturated ring may form together =O or =S; or two R^{A} present on the same S or SO ring member of a heterocyclic ring may together form a group =N(C₁-C₆-alkyl), =NO(C₁-C₆-alkyl), =NN(H)(C₁-C₆-alkyl) or =NN(C₁-C₆-alkyl)₂;
- T: is O, or S;
- Y: is NR¹⁰, O, or S;
- R⁷: independently from one another are halogen, CN, OH, C₁-C₆-alkyl, C₁-C₆-haloalkyl, C₁-C₄-alkoxy C₁-C₄-alkyl, C₁-C₄-alkoxycarbonyl, C₁-C₄-alkyl, C₁-C₆-alkoxy, C₁-C₆-alkylthio, C₁-C₆-alkylamino, di(C₁-C₄-alkyl)amino, C₁-C₆-alkoxycarbonyl, phenyl, or phenyl partially or fully substituted with R^{A};
two R⁷ present on the same carbon atom may together form a group =O, =S, =C(R^{a2})₂, =NH, =N(C₁-C₆-alkyl), =NO(C₁-C₆-alkyl), =NN(H)(C₁-C₆-alkyl), or =NN(C₁-C₆-alkyl)₂;
- R⁸, R⁹: independently are as defined for R¹⁰;
- q: is 0, 1, 2, or 3;
and the N-oxides, stereoisomers and agriculturally or veterinarily acceptable salts thereof.

The invention also provides agricultural compositions comprising at least one compound of formula I, a stereoisomer thereof and/or an agriculturally acceptable salt thereof and at least one liquid and/or solid carrier, especially at least one inert liquid and/or solid agriculturally acceptable carrier.

The invention also provides a veterinary composition comprising at least one compound of formula I, a stereoisomer thereof and/or a veterinarily acceptable salt thereof and at least one liquid and/or solid carrier, especially at least one inert veterinarily liquid and/or solid acceptable carrier.

The invention also provides a method for controlling invertebrate pests which method comprises treating the pests, their food supply, their habitat or their breeding ground or a cultivated plant, plant propagation materials (such as seed), soil, area, material or environment in which the pests are growing or may grow, or the materials, cultivated plants, plant propagation materials (such as seed), soils, surfaces or spaces to be protected from pest attack or infestation with a pesticidally effective amount of a compound of formula I or a salt thereof as defined herein.

The invention also relates to plant propagation material, in particular seed, comprising at least one compound of formula I and/or an agriculturally acceptable salt thereof.

The invention further relates to a method for treating or protecting an animal from infestation or infection by parasites which comprises bringing the animal in contact with a parasiticidally effective amount of a compound of formula I or a veterinarily acceptable salt thereof. Bringing the animal in contact with the compound I, its salt or the veterinary composition of the invention means applying or administering it to the animal.

EP1731512, WO2011/067272, and WO2018/197466 describe structurally closely related active compounds. These compounds are mentioned to be useful for combating invertebrate pests.

Nevertheless, there remains a need for highly effective and versatile agents for combating invertebrate pests. It is therefore an object of the invention to provide compounds having a good pesticidal activity and showing a broad activity spectrum against a large number of different invertebrate pests, especially against difficult to control pests, such as insects.

It has been found that these objects can be achieved by compounds of formula I as depicted and defined below, and by their stereoisomers, salts, tautomers and N-oxides, in particular their agriculturally acceptable salts.

Compounds of formula I can be prepared by reacting an activated carboxylic acid derivative of formula II or the corresponding carboxylic acid Ila with a compound of formula III in an amidation reaction. X in formula II denotes a leaving group, preferably halogen such as e.g. Cl or Br, or C₁-C₆-alkoxy such as OCH₃ or OC₂H₅. Amine III is preferably used as its ammonium salt, wherein Y is an anion, preferably a halogenide such as Cl or Br.

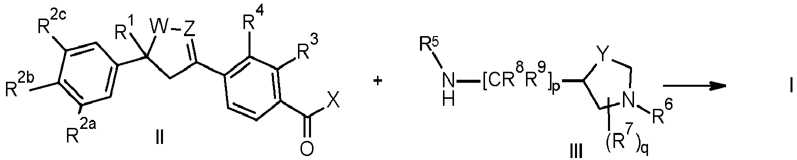

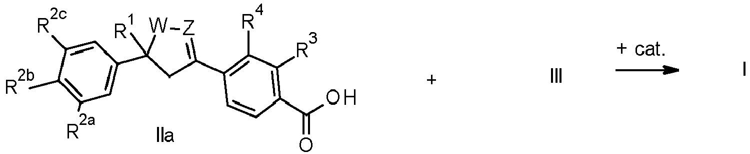

The amidation reaction is usually carried out with the acid chlorides or by prior transformation of carboxylic acids of formula IIa with oxalyl chloride [(COCl)₂] or thionylchloride (SOCl₂) to the corresponding acid chlorides, followed by reaction with the amine of formula III. Suitable reaction conditions are described in literature, e.g. in WO 2004/22536. The reaction is generally carried out in the presence of an organic base such as triethylamine (Et₃N), N,N-diisopropylethylamine (iPr₂NEt), pyridine, substituted pyridines such as collidine or lutidine, or the like. Optionally a nucleophilic catalyst such as 4-(N,N-dimethylamino)pyridine ("DMAP") can be employed in the reaction. Suitable solvents are halogenated hydrocarbons such as, dichloromethane, chloroform, and chlorobenzene, or polar aprotic solvents such as tetrahydrofurane (THF), and N,N-dimethylformamide (DMF), or aromatic hydrocarbons such as benzene, toluene, o-, m-, and p-xylene, or mixtures thereof. The transformation is usually carried out at temperatures from -40 °C to 100 °C, preferably from 0 °C to 30 °C. The starting materials are generally reacted with one another in equimolar amounts. In terms of yield, it may be advantageous to employ an excess of III, based on II.

Alternatively, amidation of the carboxylic acid IIa is carried out in the presence of a coupling reagent. Suitable coupling reagents (activators) are known and are, e.g. selected from carbodiimides, such as N,N-dicyclohexylcarbodiimide (DCC) and N,N-diisopropylcarbodiimide (DCI), benzotriazole derivatives such as 1-[bis(dimethylamino)methylene]-1H-1,2,3-triazolo[4,5-b]pyridinium 3-oxid hexafluorophosphate (HATU), O-(benzotriazol-1-yl)-N,N,N',N'-tetramethyluronium hexafluorophosphate (HBTU), and 1-[bis(dimethylamino)methylen]-5-chlorobenzotriazolium 3-oxide hexafluorophosphate (HCTU), or phosphonium-derived activators, such as (Benzotriazol-1-yloxy)tris(dimethylamino)phosphonium hexafluorophosphate (BOP), (benzotriazol-1-yl-oxytripyrrolidinophosphonium hexafluorophosphate) (Py-BOP), bromotripyrrolidinophosphonium hexafluorophosphate (Py-BrOP). Generally, the activator is used in excess. The benzotriazole and phosphonium coupling reagents are generally used in a basic medium. Preferably, bromotripyrrolidinophosphonium hexafluorophosphate (Py-BrOP) is used as the coupling reagent (activator). Suitable reaction conditions are described in the literature, e.g. in WO2015/128358. The reaction is generally carried out in the presence of a base such as a tertiary amine base like iPr₂NEt, Et₃N. Suitable solvents are, e.g., halogenated hydrocarbons such as dichloromethane, chloroform, and chlorobenzene. The transformation is usually carried out at temperatures from 0 °C to 100 °C, preferably from 10 °C to 40 °C. The starting materials are generally reacted with one another in equimolar amounts. In terms of yield, it may be advantageous to employ an excess of III, based on II.

Introduction of group R⁵ being different from hydrogen is preferably made to formula III compounds. Alternatively, such R⁵ can also be introduced to formula VI, or to formula I compounds wherein R⁵ is H.

Introduction of groups R⁷ being C(S) is preferably achieved from formula III compounds with R⁷ being C(O) by using Lawesson's reagent [cf. Thomson et al., Organic Syntheses, Collective Volumen, 7, 372].

Depending on the nature of Y and (R⁷)_{q} compounds of formula III can be obtained by different reaction sequences. Compounds of formula IIIa are intermediates for formula I compounds.

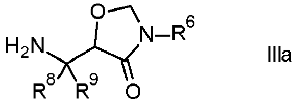

IIIa can be obtained starting with amidation of a compound formula IVa, which is commercially available or can be made by standard methods of organic chemistry, with alkyl amine, e.g. methylamine. 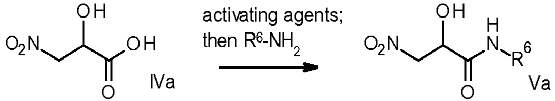 Suitable reaction conditions for the preparation of compounds of formula Va by amidation can be found in the literature [e.g. Jiajia, Chin. J. Med. Chem. 2009, 19, 401-406]. This transformation is usually carried out at temperatures of from -5°C to 10°C, preferably at 0°C, in an inert solvent in the presence of a carboxylic acid activator such as, e.g. thionyl chloride (SOCl₂), 1,1'-carbonyldiimidazole, or carbodiimides, such as DCC, and DCI, benzotriazole derivatives such as HATU, HBTU, and HCTU, or the like, followed, after the appropriate time, by the addition of the amine of formula R⁶-NH₂. Suitable solvents are, e.g. halogenated hydrocarbons such as dichloromethane, chloroform, and chlorobenzene, or polar aprotic solvents such as THF, DMF, and dimethyl sulfoxide (DMSO), or mixtures thereof. Optionally, the reaction can be carried out in the presence of an additional organic base such as, NEt₃, N-ethyl-N,N-diisopropylamine (iPr₂NEt), pyridine, or substituted pyridines such as collidine or lutidine. The transformation is usually carried out at temperatures from -50°C to 50°C, preferably from 0°C to 25°C. The starting materials are generally reacted with one another in equimolar amounts. In terms of yield, it may be advantageous to employ a slight excess of the carboxylic acid activator and R⁶-NH₂, based on IVa.

Suitable reaction conditions for the cyclization of compounds of formula Via can be found in the literature [cf. Polonski, Tetrahedron, 1983, 39, 3139-3143]. The reaction is generally carried out at temperatures of from 25°C to 130°C, preferably at 120°C, in an inert solvent, with para-formaldehyde in the presence of an acid. Para-formaldehyde can be replaced by a either a ketone of formula (R⁷)₂C(O) or an aldehyde of formula R⁷HC(O) for the introduction of R⁷ at the branching point of the N,O-acetal.

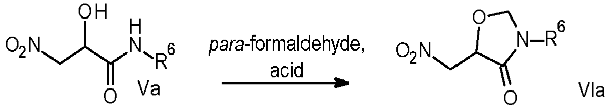

Suitable solvents are aliphatic hydrocarbons such as pentane, hexane, cyclohexane, and petrol ether, aromatic hydrocarbons such as toluene, o-, m-, and p-xylene, ethers such as diethylether, diisopropylether, tert.-butylmethylether (TBME), dioxane, anisole, and THF, preferably toluene. It is also possible to use mixtures of the solvents mentioned.

Suitable acids are organic acids such as formic acid, acetic acid, propionic acid, oxalic acid, toluene sulphonic acid, benzene sulphonic acid, camphor sulphonic acid, citric acid, and trifluoro acetic acid, preferably toluene sulphonic acid. The acids are generally employed in catalytic amounts; however, they can also be used in equimolar amounts, in excess or, if appropriate, as solvent.

Suitable reaction conditions for the reduction of compounds of formula Via can be found in the literature [cf. Xu et al., Synthesis, 2002, 8, 1017-1026].

At this stage R⁸ and R⁹ can be introduced by alkylation of compound VIa. Alkylation of formula Via compounds can be effected by reaction of Via with compounds R⁸-Z and R⁹-Z wherein Z is a nuceophilic leaving group such as halogen like Cl, Br and I, or a sulfonate such as trifluoromethanesulfoate (OTf), or methanesulfonate (OMs).

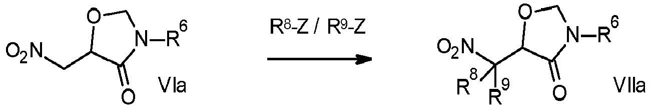

The reaction is generally carried out in the presence of a base such as, e.g., inorganic bases, such as alkali metal and alkaline earth metal hydroxides, such as LiOH, NaOH, KOH, CsOH, and Ca(OH)₂, alkali metal and alkaline earth metal hydrides, such as LiH, NaH, KH, and CaH₂, or alkali metal and alkaline earth metal carbonates, such as Li₂CO₃, K₂CO₃, and Cs₂CO₃, or alkali metal fluorides such as CsF. Suitably, the alkali metal and alkaline earth metal hydroxides and carbonates can be employed as an aqueous solution in a biphasic reaction mixture together with an organic solvent such as halogenated hydrocarbons such as dichloromethane, chloroform, and chlorobenzene. Optionally, if biphasic conditions are used, a phase transfer catalyst can be employed in the reaction such as tetra(*n*-butyl)ammonium iodide (Bi₄NI). Otherwise, if the reaction is performed in the absence of water, suitable organic solvents are, e.g. halogenated hydrocarbons such as dichloromethane, chloroform, and chlorobenzene, or polar aprotic solvents such as THF, acetonitrile, or DMF. The starting materials are generally reacted with one another in equimolar amounts. In terms of yield, it may be advantageous to employ an excess of R⁸-Z or R⁹, based on VIa.

Introduction of R⁷ to the five-membered ring can be performed with an alkylation reaction. Alkylation of formula VIIa compounds can be effected by reaction of VIIa with a compound R⁷-Z, wherein Z is a nuceophilic leaving group such as halogen like Cl, Br and I, or a sulfonate such as OTf, or OMs.

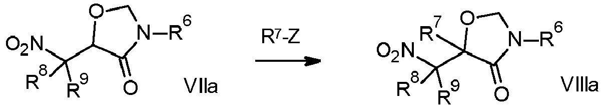

The reaction is generally carried out in the presence of a base, such as inorganic bases, such as alkali metal and alkaline earth metal hydroxides, such as LiOH, NaOH, KOH, CsOH, and Ca(OH)₂, alkali metal and alkaline earth metal hydrides, such as LiH, NaH, KH, and CaH₂, or alkali metal and alkaline earth metal carbonates, such as Li₂CO₃, K₂CO₃, and Cs₂CO₃, or alkali metal fluorides such as CsF. Suitably, the alkali metal and alkaline earth metal hydroxides and carbonates can be employed as an aqueous solution in a biphasic reaction mixture together with an organic solvent such as halogenated hydrocarbons such as dichloromethane, chloroform, and chlorobenzene. Optionally, if biphasic conditions are used, a phase transfer catalyst can be employed in the reaction such as Bu₄NI. Otherwise, if the reaction is performed in the absence of water, suitable organic solvents are, e.g., halogenated hydrocarbons such as dichloromethane, chloroform, and chlorobenzene, or polar aprotic solvents such as THF, acetonitrile, or DMF. The starting materials are generally reacted with one another in equimolar amounts. In terms of yield, it may be advantageous to employ an excess of R⁷-Z, based on VIIa.

This transformation is usually carried out at temperatures of from 0°C to 30°C, preferably at 25°C, in an inert solvent, in the presence of a reducing agent.

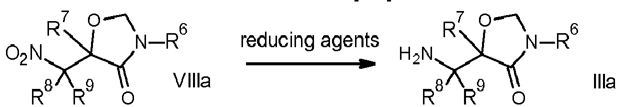

Solvents are alcohols such as methanol, ethanol, n-propanol, isopropanol, n-butanol, and tert.-butanol. It is also possible to use mixtures of the solvents mentioned. Suitable reducing agents are in general Raney-Nickel, Pd/C, or Zn.

5-Amino-thiazolodin-4-ones of formula IIIb 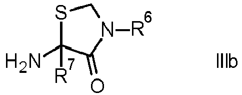 are intermediates for formula I compounds wherein Y is S and p is 0; IIIb can be obtained by a reaction sequence starting with reduction of compound of formula IVb, which is commercially available, or can be made by standard methods of organic chemistry known to a person skilled in the art.

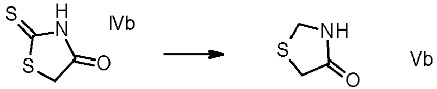

This transformation is usually carried out at temperatures of from 0°C to 120°C, preferably from 60°C to 70°C, in an inert solvent, in the presence of an acid and a reducing agent [cf. Gupta et al., JACS 2017, 139, 15, 5588-5595].

Suitable solvents are alcohols such as methanol, ethanol, n-propanol, isopropanol, n-butanol, and tert.-butanol. It is also possible to use mixtures of the solvents mentioned. Suitable reducing agents are in general Pd/C, Zn, Raney-Nickel. Suitable acids and acidic catalysts are in general organic acids such as formic acid, acetic acid, propionic acid, oxalic acid, toluene sulphonic acid, benzene sulphonic acid, camphor sulphonic acid, citric acid, and trifluoro acetic acid. The acids are generally employed in catalytic amounts; however, they can also be used in equimolar amounts, in excess or, if appropriate, as solvent.

The starting materials are generally reacted with one another in equimolar amounts. In terms of yield, it may be advantageous to employ an excess of the reducing agent, based on IVb.

Alkylation of formula Vb compounds can be effected by reaction of Vb with a compound R⁶-Z wherein Z is a nuceophilic leaving group such as halogen like Cl, Br, and I, or a sulfonate such as OTf, or OMs. Suitable reaction conditions are described in literature [cf. Maddaluno et al., Tetrahedron: Asymmetry 1992, 3, 1239-1242, EP1553088, Kitaori et al., Tetrahedron, 1999, 55, 14381-14390C].

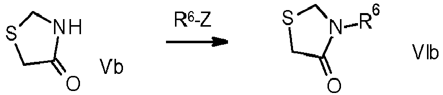

The reaction is generally carried out in the presence of a base such as inorganic bases, such as alkali metal and alkaline earth metal hydroxides, such as LiOH, NaOH, KOH, CsOH, and Ca(OH)₂, alkali metal and alkaline earth metal hydrides, such as LiH, NaH, KH, and CaH₂, or alkali metal and alkaline earth metal carbonates, such as Li₂CO₃, K₂CO₃, and Cs₂CO₃, or alkali metal fluorides such as CsF. Suitably, the alkali metal and alkaline earth metal hydroxides and carbonates can be employed as an aqueous solution in a biphasic reaction mixture together with an organic solvent such as halogenated hydrocarbons such as dichloromethane, chloroform, and chlorobenzene. Optionally, if biphasic conditions are used, a phase transfer catalyst can be employed in the reaction such as Bu₄NI. Otherwise, if the reaction is performed in the absence of water, suitable organic solvents are, e.g., halogenated hydrocarbons such as dichloromethane, chloroform, and chlorobenzene, or polar aprotic solvents such as THF, acetonitrile, or DMF. The starting materials are generally reacted with one another in equimolar amounts. In terms of yield, it may be advantageous to employ an excess of R⁶-Z, based on Vb.

Introduction of the functionality in position 5 of the ring is effected by halogenation to yield the 5-halo-compound of formula Vlb.

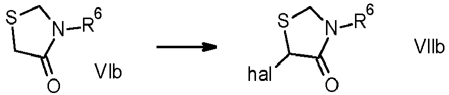

This transformation is usually carried out at temperatures of from 25°C to 150°C, preferably from 100°C to 110°C, in an inert solvent, in the presence of a halogenating agent such as SOCl₂ [cf. US5,856,273]

Suitable solvents are halogenated hydrocarbons such as methylene chloride, chloroform, and chlorobenzene, preferably in methylene chloride. It is also possible to use mixtures of the solvents mentioned. Suitable sources for halogenating agents are SOCl₂, *N*-bromosuccinimid (NBS), or bromine [cf. Dzambaski et al, Tetrahedron, 2013, 69, 9819-9815]. The starting materials are generally reacted with one another in equimolar amounts. In terms of yield, it may be advantageous to employ an excess of the halogenating agent, based on Vllb.

For introduction of R⁷ the five-membered ring can be alkylated. Reformatzki-type alkylation of formula Vllb compounds can be effected by reaction of Vllb with a compound R⁷-Z wherein Z is a nuceophilic leaving group such as halogen like Cl, Br, and I, or a sulfonate such as OTf, or OMs.

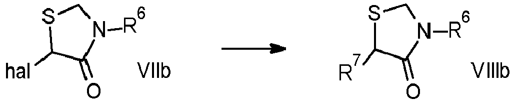

The reaction is generally carried out in the presence of a alkaline earth metall such as, e.g. magnesium (Mg) or a transition metal such as, e.g. zinc (Zn). Suitable organic solvents are aliphatic hydrocarbons such as pentane, hexane, cyclohexane, and petrol ether, aromatic hydrocarbons such as toluene, o-, m-, and p-xylene, halogenated hydrocarbons such as methylene chloride, chloroform, and chlorobenzene, ethers such as diethylether, Diisopropylether, TBME, dioxane, anisole, and THF. The starting materials are generally reacted with one another in equimolar amounts. In terms of yield, it may be advantageous to employ an excess of R⁷-Z, based on VIIb.

Functionalization of compound Vlllb can be effected by halogenation to yield 5-halo-compound of formula IXb.

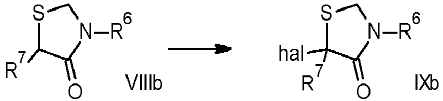

This transformation is usually carried out at temperatures of from 25°C to 150°C, preferably from 100°C to 110°C, in an inert solvent, in the presence of a halogenating agent such as SOCl₂ [cf. US5,856,273]

Suitable solvents are halogenated hydrocarbons such as methylene chloride, chloroform, and chlorobenzene, preferably in methylene chloride. It is also possible to use mixtures of the solvents mentioned. Suitable sources for halogenating agents are SOCl₂, NBS, or bromine [cf. literature above].

The starting materials are generally reacted with one another in equimolar amounts. In terms of yield, it may be advantageous to employ an excess of the halogenating agent, based on VIIIb. Compounds of IXb can be reacted with NaN₃ to yield compounds of formula Xb. If previous described substitution with R⁷ is not desired compound Vllb can be used as starting material instead of IXb.

This transformation is usually carried out at temperatures of from 25°C to 50°C, preferably at 25°C to, in an inert solvent [cf. Zhang et al., Adv. Synth. Catal. 2004, 346, 1335-1354].

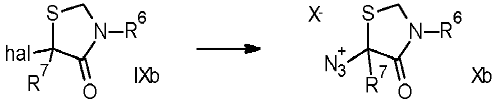

Suitable solvents are alcohols such as methanol, ethanol, *n*-propanol, isopropanol, *n*-butanol, and tert.-butanol, it is also possible to use mixtures of the solvents mentioned. The starting materials are generally reacted with one another in equimolar amounts. In terms of yield, it may be advantageous to employ an excess of NaN₃, based on IXb.

The azido group can be reduced to amino under hydrogenolytic conditions in the presence of a H₂ atmosphere, and a palladium catalyst such as, e.g., palladium on carbon ("Pd/C") [cf. Iding et al., Tetrahedron 2004, 647-653], Pd(OH)₂ on carbon ("Pearlman catalyst") [cf. Maddaluno et al., Tetrahedron: Asymmetry 1992, 3, 1239-1242].

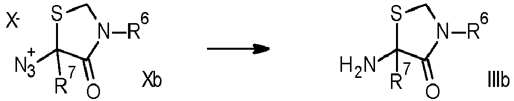

This transformation is usually carried out at temperatures of from 0°C to 80°C, preferably from 20°C to 30°C, in an inert solvent, in the presence of a Pd catalyst [cf. Iding et al., Tetrahedron 2004, 647-653]. Suitable Pd catalysts are Pd/C, Pd(OAc)₂, Pd(OH)₂, Pd(PPh₃)₄. Suitable solvents are alcohols such as methanol, ethanol, n-propanol, isopropanol, n-butanol, and tert.-butanol, it is also possible to use mixtures of the solvents mentioned.

Compounds of formula IIIc are intermediates for formula I compounds.

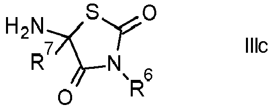

IIIc can be obtained starting with an alkylation of compound formula IVc, which is commercially available or can be made by standard methods of organic chemistry. Alkylation of formula Vc compounds can be effected by reaction of Vc with a compound R⁶-Z wherein Z is a nuceophilic leaving group such as halogen like Cl, Br and I, or a sulfonate such as OTf, or OMs. Suitable reaction conditions are described in literature [cf. Maddaluno et al., Tetrahedron: Asymmetry 1992, 3, 1239-1242, EP1553088, or Kitaori et al., Tetrahedron, 1999, 55, 14381-14390C]. 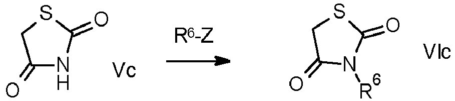 The reaction is generally carried out in the presence of a base such as, e.g., inorganic bases, such as alkali metal and alkaline earth metal hydroxides, such as LiOH, NaOH, KOH, CsOH, and Ca(OH)₂, alkali metal and alkaline earth metal hydrides, such as LiH, NaH, KH, and CaH₂, or alkali metal and alkaline earth metal carbonates, such as Li₂CO₃, K₂CO₃, and Cs₂CO₃, or alkali metal fluorides such as CsF. Suitably, the alkali metal and alkaline earth metal hydroxides and carbonates can be employed as an aqueous solution in a biphasic reaction mixture together with an organic solvent such as halogenated hydrocarbons such as dichloromethane, chloroform, and chlorobenzene. Optionally, if biphasic conditions are used, a phase transfer catalyst can be employed in the reaction such as, e.g. Bu₄NI. Otherwise, if the reaction is performed in the absence of water, suitable organic solvents are, e.g., halogenated hydrocarbons such as dichloromethane, chloroform, and chlorobenzene, or polar aprotic solvents such as THF, acetonitrile, or DMF. The starting materials are generally reacted with one another in equimolar amounts. In terms of yield, it may be advantageous to employ an excess of R⁶-Z, based on Vc.

Introduction of the functionality in position 5 of the ring is effected by halogenation to yield the 5-halo-compound of formula VIIc.

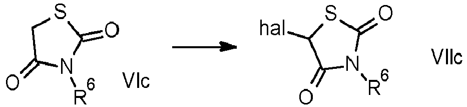

This transformation is usually carried out at temperatures of from 0° C to 100 °C, preferably from 0 °C to 60 °C, in an inert solvent, in the presence of a halogenating agent such as bromine and an acid [cf. WO 2002006258]. Suitable solvents are halogenated hydrocarbons such as methylene chloride, chloroform, and chlorobenzene, preferably methylene chloride. It is also possible to use mixtures of the solvents mentioned. Suitable acids and acidic catalysts are in general organic acids such as formic acid, acetic acid, propionic acid, oxalic acid, toluene sulphonic acid, benzene sulphonic acid, camphor sulphonic acid, citric acid, and trifluoro acetic acid, preferably acetic acid.

The starting materials are generally reacted with one another in equimolar amounts. In terms of yield, it may be advantageous to employ an excess of the halogenating agent, based on VIc.

Nucleophilic substitution of the halide allows introduction of an imine in the 5-position. This transformation is usually carried out at temperatures of from 0°C to 100°C, preferably from 25°C to 75°C, in an inert solvent, in the presence of a suitable nucleophile such as imine Vlllc. Examples for nucleophilic replacement of 5-halo-compounds can be found in literature, e.g. WO2002006258.

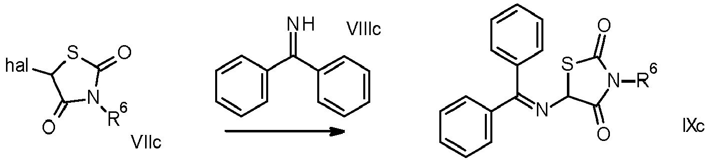

Suitable solvents are, e.g. halogenated hydrocarbons such as dichloromethane, chloroform, and chlorobenzene, or polar aprotic solvents such as THF, DMF, and DMSO, or mixtures thereof.

Introduction of R⁷ to the five-membered ring can be performed by alkylation. Alkylation of formula IXc compounds can be effected by reaction of IXc with a compound R⁷-Z wherein Z is a nuceophilic leaving group such as halogen like Cl, Br and I, or a sulfonate such as OTf, or OMs. Suitable reaction conditions are described in literature mentioned above.

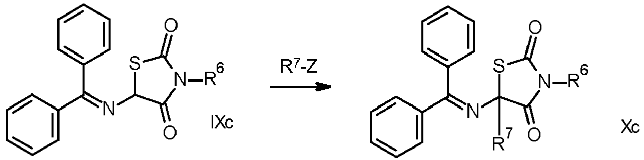

The reaction is generally carried out in the presence of a base such as inorganic bases, such as alkali metal and alkaline earth metal hydroxides, such as LiOH, NaOH, KOH, CsOH, and Ca(OH)₂, alkali metal and alkaline earth metal hydrides, such as LiH, NaH, KH, and CaH₂, or alkali metal and alkaline earth metal carbonates, such as Li₂CO₃, K₂CO₃, and Cs₂CO₃, or alkali metal fluorides such as CsF. Suitably, the alkali metal and alkaline earth metal hydroxides and carbonates can be employed as an aqueous solution in a biphasic reaction mixture together with an organic solvent such as halogenated hydrocarbons such as dichloromethane, chloroform, and chlorobenzene. Optionally, if biphasic conditions are used, a phase transfer catalyst can be employed in the reaction such as, e.g. Bu₄NI. Otherwise, if the reaction is performed in the absence of water, suitable organic solvents are, e.g., halogenated hydrocarbons such as dichloromethane, chloroform, and chlorobenzene, or polar aprotic solvents such as THF, acetonitrile, or DMF. The starting materials are generally reacted with one another in equimolar amounts. In terms of yield, it may be advantageous to employ an excess of R⁷-Z, based on IXc.

Subsequent hydrolysis of tertiary imine IXc to desired 5-amino-thiazolidinone IIIc can be performed under acidic conditions. This transformation is usually carried out at temperature of from 20°C to 45°C, preferably at 25°C, in an inert solvent, in the presence of a strong acid [cf. McLaughlin et al., JOC 2003, 68, 50-54].

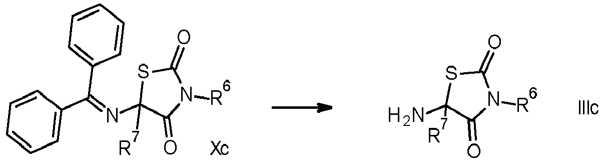

Suitable solvents are ethers such as diethylether, diisopropylether, TBME, dioxane, anisole, and THF, or mixtures thereof.

Suitable acids and acidic catalysts are in general anorganic acids such as HF, HCI, HBr, H₂SO₄, and HClO₄, preferably HCl.

Compounds of formula IIId are intermediates for formula I compounds.

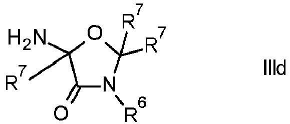

IIId can be obtained starting with a cyclization of compound formula IVd, which is commercially available or can be made by standard methods of organic chemistry. This transformation is usually carried out at temperatures from 20°C to 100°C, preferably at 60°C, in an inert solvent, in the presence of an acid [cf. Kametani et al., Heterocycles 1977, 7, 919-25].

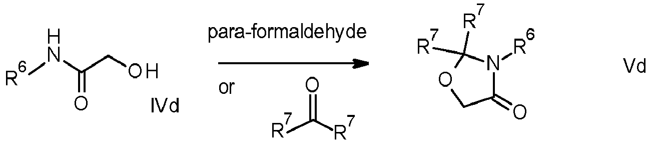

Suitable solvents are aliphatic hydrocarbons such as pentane, hexane, cyclohexane, and petrol ether, aromatic hydrocarbons such as toluene, o-, m-, and p-xylene, ethers such as diethylether, diisopropylether, TBME, dioxane, anisole, and THF, preferably toluene. It is also possible to use mixtures of the solvents mentioned.

Suitable acids are organic acids such as formic acid, acetic acid, propionic acid, oxalic acid, toluene sulphonic acid, benzene sulphonic acid, camphor sulphonic acid, citric acid, and trifluoro acetic acid, preferably toluene sulphonic acid. The acids are generally employed in catalytic amounts; however, they can also be used in equimolar amounts, in excess or, if appropriate, as solvent.

The reaction can either be performed with para-formaldehyde or with either an aldehyde of the type R⁷CH(O) or a ketone of the type (R⁷)₂C(O) if introduction of R⁷ is desired.

Introduction of the functionality in position 5 of the ring is effected by halogenation to yield the 5-halo-compound of formula Vld.

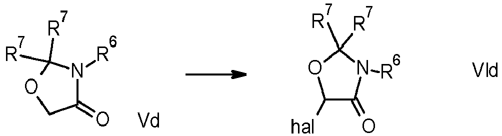

This transformation is usually carried out at temperatures of from 0°C to 100°C, preferably from 0 °C to 60 °C, in an inert solvent, in the presence of a halogenating agent such as bromine and an acid.

Suitable solvents are halogenated hydrocarbons such as methylene chloride, chloroform, and chlorobenzene, preferably in methylene chloride. It is also possible to use mixtures of the solvents mentioned.

The starting materials are generally reacted with one another in equimolar amounts. In terms of yield, it may be advantageous to employ an excess of the halogenating agent, based on Vd. Suitable acids and acidic catalysts are in general organic acids such as formic acid, acetic acid, propionic acid, oxalic acid, toluene sulphonic acid, benzene sulphonic acid, camphor sulphonic acid, citric acid, and trifluoro acetic acid, preferably acetic acid.

Nucleophilic substitution of the halide allows introduction of an imine in the 5-position. This transformation is usually carried out at temperatures of from 0°C to 100°C, preferably from 25°C to 75°C in an inert solvent, in the presence of a suitable nucleophile such as imine Vlllc. 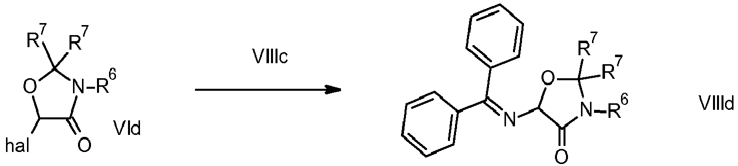 Suitable solvents are, e.g. halogenated hydrocarbons such as dichloromethane, chloroform, and chlorobenzene, or polar aprotic solvents such as THF, DMF, and DMSO, or mixtures thereof.

Introduction of R⁷ to the five-membered ring can be performed with an alkylation reaction. Alkylation to compounds of formula Xd can be effected by reaction of VIIId with a compound R⁷-Z wherein Z is a nuceophilic leaving group such as halogen like Cl, Br and I, or a sulfonate such as OTf, or OMs.

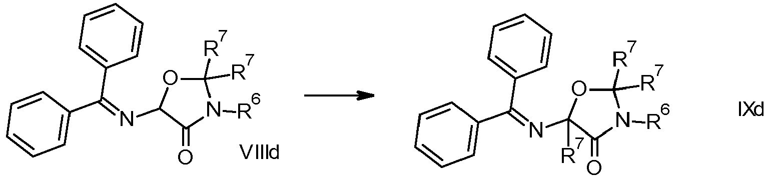

The reaction is generally carried out in the presence of a base such as inorganic bases, such as alkali metal and alkaline earth metal hydroxides, such as LiOH, NaOH, KOH, CsOH, and Ca(OH)₂, alkali metal and alkaline earth metal hydrides, such as LiH, NaH, KH, and CaH₂, or alkali metal and alkaline earth metal carbonates, such as Li₂CO₃, K₂CO₃, and Cs₂CO₃, or alkali metal fluorides such as CsF. Suitably, the alkali metal and alkaline earth metal hydroxides and carbonates can be employed as an aqueous solution in a biphasic reaction mixture together with an organic solvent such as halogenated hydrocarbons such as dichloromethane, chloroform, and chlorobenzene. Optionally, if biphasic conditions are used, a phase transfer catalyst can be employed in the reaction such as, e.g., Bu₄NI. Otherwise, if the reaction is performed in the absence of water, suitable organic solvents are, e.g., halogenated hydrocarbons such as dichloromethane, chloroform, and chlorobenzene, or polar aprotic solvents such as THF, acetonitrile, or DMF. The starting materials are generally reacted with one another in equimolar amounts. In terms of yield, it may be advantageous to employ an excess of R⁷-Z, based on Vllld.

Subsequent hydrolysis of tertiary imine Xd to 5-amino-thiazolidinone IIId can be performed under acidic conditions. This transformation is usually carried out at temperature of from 20°C to 45°C, preferably at 25°C, in an inert solvent, in the presence of a strong acid [cf. Wolf et al. Advanced Synthesis & Catalysis, 2001, 343, 662-674].

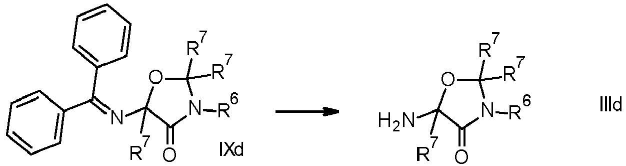

Suitable solvents are ethers such as diethylether, diisopropylether, TBME, dioxane, anisole, and THF, or mixtures thereof. Suitable acids and acidic catalysts are in general anorganic acids such as hydrofluoric acid, hydrochloric acid, hydrobromic acid, sulphuric acid und perchloric acid, preferably hydrochloric acid.

Compounds of formula IIIe are intermediates for formula I compounds.

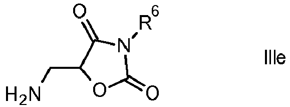

IIIe can be obtained by a reaction sequence starting with an amidation of compound formula IVe, wherein PG is a protective group, such as benzyloxycarbonyl ("Cbz"), benzyl ("Bn"), *tert-*butylcarbonyl ("Boc"), or acetyl ("Ac"). Compound Ve can be made by standard methods of organic chemistry, with alkyl amine e.g. methyl amine.

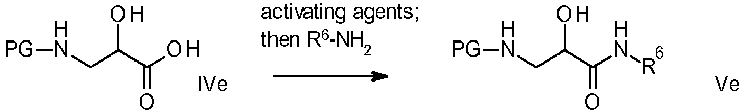

Suitable reaction conditions for the preparation of compounds of formula Ve by amidation can be found in literature [cf. Jiajia Chin. J. Med. Chem. 2009, 19, 401-406]. This transformation is usually carried out at temperatures of from -5°C to 10°C, preferably at 0°C, in an inert solvent in the presence of a carboxylic acid activator such as SOCl₂, 1,1'-carbonyldiimidazole, or carbodiimides, such as DCC and DCI, benzotriazole derivatives such as HATU, HBTU, and HCTU, or the like, followed by the addition of the amine R⁶-NH₂. Suitable solvents are, e.g. halogenated hydrocarbons such as, dichloromethane, chloroform, and chlorobenzene, or polar aprotic solvents such as THF, DMF, and DMSO, or mixtures thereof. Optionally, the reaction can be carried out in the presence of an additional organic base such as, NEt₃, iPr₂NEt, pyridine, or substituted pyridines such as collidine or lutidine. The transformation is usually carried out at temperatures from -50°C to 50°C, preferably from 0°C to 25°C. The starting materials are generally reacted with one another in equimolar amounts. In terms of yield, it may be advantageous to employ a slight excess of the carboxylic acid activator and R⁶-NH₂, based on IVe.

Subsequent cyclization of Ve with an activating reagent such as e.g. *N*,*N*'-disuccinimidyl carbonate (DSC).

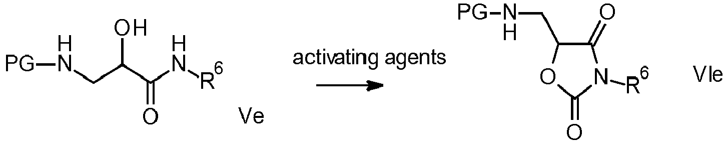

This transformation is usually carried out at temperatures of from 10°C to 60°C, preferably at 25°C, in an inert solvent in the presence of an activating reagent, e.g. as DSC, *N*,*N*-Carbon-yldiimidazol (CDI), thiophosgene, or di-*tert-*butyl decarbonate (Boc₂O). Suitable solvents are, e.g., halogenated hydrocarbons such as dichloromethane, chloroform, and chlorobenzene, or polar aprotic solvents such as THF, DMF, and DMSO, or mixtures thereof.

Next step is a cleavage under hydrogenolytic conditions in the presence of H₂ atmosphere, and a palladium catalyst, e.g. Pd/C [cf. Iding et al., Tetrahedron 2004, 647-653], Pd(OH)₂ on carbon ("Pearlman catalyst") [cf.W. M. Pearlman, THL, 1967, 8, 1663-1664] for deprotection of the amine, which gives access to compounds of formular IIIe.

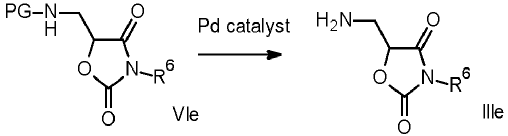

This transformation is usually carried out at temperatures of from 0°C to 80°C, preferably from 20°C to 30°C, in an inert solvent, in the presence of a Pd catalyst [cf. Iding et al., Tetrahedron 2004, 647-653].

Suitable Pd catalysts are Pd/C, Pd(OAc)₂, Pd(OH)₂, and Pd(PPh₃)₄. Suitable solvents are alcohols such as methanol, ethanol, *n*-propanol, isopropanol, *n*-butanol, and tert.-butanol, it is also possible to use mixtures of the solvents mentioned.

Compounds of formula IIIf are intermediates for formula I compounds.

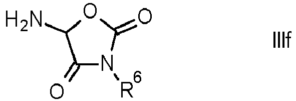

Illf can be obtained starting with a nucleophilic addition reaction of PG-NH₂ to ethyl glyoxylate hydrate, wherein PG is a protective group, such as benzyloxycarbonyl ("Cbz"), benzyl ("Bn"), *tert*-butylcarbonyl ("Boc"), or acetyl ("Ac"). This transformation is usually carried out at temperature of from 0°C to 60°C, preferably at 25°C, in an inert solvent, in the presence of an acid.

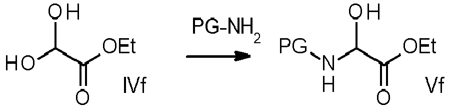

Suitable solvents are ethers such as diethylether, *di*-isopropylether, TBME, dioxane, anisole, and THF, nitrils such as acetonitrile, and propionitrile, moreover DMSO, and dimethylacetamide, preferably acetonitrile. Suitable organic acids such as formic acid, acetic acid, propionic acid, oxalic acid, toluene sulphonic acid, benzene sulphonic acid, camphor sulphonic acid, citric acid, and trifluoro acetic acid. The acids are generally employed in catalytic amounts; however, they can also be used in equimolar amounts, in excess or, if appropriate, as solvent. The starting materials are generally reacted with one another in equimolar amounts. In terms of yield, it may be advantageous to employ an excess of the reducing agent, based on IVf. Next step is the cyclization of compound Vf to Vlf in the presence of an activating reagent under basic conditions.

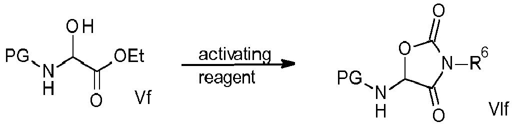

This transformation is usually carried out at temperatures of from 10°C to 60°C, preferably at 25°C, in an inert solvent in the presence of an activating reagent, e.g. as DSC, CDI, thiophosgene, or Boc₂O. Suitable solvents are, e.g. halogenated hydrocarbons such as dichloromethane, chloroform, and chlorobenzene, or polar aprotic solvents such as THF, DMF, and DMSO, or mixtures thereof.

Next step is a cleavage reaction under hydrogenolytic conditions in the presence of H₂ atmosphere, and a Palladium catalyst such as, e.g. Pd/C [cf. H. Iding et al., Tetrahedron 2004, 647-653], Pd(OH)₂ on carbon ("Pearlman catalyst") [cf. W. M. Pearlman, THL, 1967, 8, 1663-1664.] for deprotection, which gives access to compounds of formula IIIf.

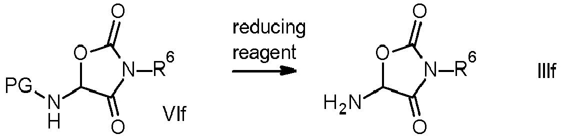

This transformation is usually carried out at temperatures of from 0°C to 80°C, preferably from 20°C to 30°C, in an inert solvent, in the presence of a Pd catalyst [cf. Iding et al., Tetrahedron 2004, 647-653]. Suitable Pd catalysts are Pd/C, Pd(OAc)₂, Pd(OH)₂, Pd(PPh₃)₄. Suitable solvents are alcohols such as methanol, ethanol, *n*-propanol, isopropanol, *n*-butanol, and *tert.*-bu-tanol, it is also possible to use mixtures of the solvents mentioned.

The starting materials required for preparing compounds I are commercially available or known from literature [cf. WO2014/029639; WO2010/72781] or can be prepared in accordance with the literature cited.

As a rule, the compounds of formula I, and their precursors in the synthesis process, can be prepared by the methods described above. If individual compounds cannot be prepared via the above-described routes, they can be prepared by derivatization of other compounds I or the respective precursor or by customary modifications of the synthesis routes described. For example, in individual cases, certain compounds of formula I can advantageously be prepared from other compounds of formula I by derivatization, e.g. by ester hydrolysis, amidation, esterification, ether cleavage, olefination, reduction, oxidation, or by customary modifications of the synthesis routes described.

The reaction mixtures are worked up in the customary manner, e.g. by mixing with water, separating the phases, and, if appropriate, purifying the crude products by chromatography, e.g. on alumina or on silica gel. Some of the intermediates and end products may be obtained in the form of colorless or pale brown viscous oils which are freed or purified from volatile components under reduced pressure and at moderately elevated temperature. If the intermediates and end products are obtained as solids, they may be purified by recrystallization or trituration. However, if the synthesis yields mixtures of isomers, a separation is generally not necessarily required since in some cases the individual isomers can be interconverted during work-up for use or during application (e.g. under the action of light, acids or bases). Such conversions may also take place after use, e.g. in the treatment of plants in the treated plant.

The organic moieties mentioned in the above definitions of the variables are - like the term halogen - collective terms for individual listings of the individual group members. The prefix Cₙ-Cₘ indicates in each case the possible number of carbon atoms in the group.

The term "halogen" denotes in each case fluorine, bromine, chlorine or iodine, in particular fluorine, chlorine or bromine.

The term "alkyl" as used herein and in the alkyl moieties of alkylamino, alkylcarbonyl, alkylthio, alkylsulfinyl, alkylsulfonyl and alkoxyalkyl denotes in each case a straight-chain or branched alkyl group having usually from 1 to 10 carbon atoms, frequently from 1 to 6 carbon atoms, preferably 1 to 4 carbon atoms, more preferably from 1 to 3 carbon atoms. Examples of an alkyl group are methyl (Me), ethyl (Et), n-propyl, iso-propyl, n-butyl, 2-butyl, iso-butyl, tert-butyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl.

The term "haloalkyl" as used herein and in the haloalkyl moieties of haloalkylcarbonyl, haloalkoxycarbonyl, haloalkylthio, haloalkylsulfonyl, haloalkylsulfinyl, haloalkoxy and haloalkoxyalkyl, denotes in each case a straight-chain or branched alkyl group having usually from 1 to 10 carbon atoms, frequently from 1 to 6 carbon atoms, preferably from 1 to 4 carbon atoms, wherein the hydrogen atoms of this group are partially or totally replaced with halogen atoms. Preferred haloalkyl moieties are selected from C₁-C₄-haloalkyl, more preferably from C₁-C₃-haloalkyl or C₁-C₂-haloalkyl, in particular from C₁-C₂-fluoroalkyl such as fluoromethyl, difluoromethyl, trifluoromethyl, 1-fluoroethyl, 2-fluoroethyl, 2,2-difluoroethyl, 2,2,2-trifluoroethyl, and pentafluoroethyl. The term "alkenyl" as used herein denotes in each case a singly unsaturated hydrocarbon radical having usually 2 to 10, frequently 2 to 6, preferably 2 to 4 carbon atoms, e.g. vinyl, allyl (2-propen-1-yl), 1-propen-1-yl, 2-propen-2-yl, methallyl (2-methylprop-2-en-1-yl), 2-buten-1-yl, 3-buten-1-yl, 2-penten-1-yl, 3-penten-1-yl, 4-penten-1-yl, 1-methylbut-2-en-1-yl, 2-ethylprop-2-en-1-yl and the like.

The term "cycloalkyl" as used herein and in the cycloalkyl moieties of cycloalkoxy and cycloalkylthio denotes in each case a monocyclic cycloaliphatic radical having usually from 3 to 10 or from 3 to 6 carbon atoms, such as cyclopropyl (c-Pr), cyclobutyl, cyclopentyl, cyclohexyl, cycloheptyl, cyclooctyl, cyclononyl, and cyclodecyl or cyclopropyl, cyclobutyl, cyclopentyl and cyclohexyl.

The term "cycloalkenyl" as used herein and in the cycloalkenyl moieties of cycloalkenyloxy and cycloalkenylthio denotes in each case a monocyclic singly unsaturated non-aromatic radical having usually from 3 to 10, e.g. 3 or 4 or from 5 to 10 carbon atoms, preferably from 3- to 8 carbon atoms. Exemplary cycloalkenyl groups include cyclopropenyl, cycloheptenyl or cyclooctenyl.

The term "carbocycle" or "carbocyclyl" includes in general a 3- to 12-membered, preferably a 3-to 8-membered or a 5- to 8-membered, more preferably a 5- or 6-membered monocyclic, non-aromatic ring comprising 3 to 12, preferably 3 to 8 or 5 to 8, more preferably 5 or 6 carbon atoms. Preferably, the term "carbocycle" covers cycloalkyl and cycloalkenyl groups as defined above.

The term "heterocycle" or "heterocyclyl" includes in general 3- to 12-membered, preferably 5- or 6-membered, in particular 6-membered monocyclic heterocyclic non-aromatic radicals. The heterocyclic non-aromatic radicals usually comprise 1, 2 or 3 heteroatoms selected from N, O and S as ring members, wherein S-atoms as ring members may be present as S, SO or SO₂. Examples of 5- or 6-membered heterocyclic radicals comprise saturated or unsaturated, non-aromatic heterocyclic rings, such as 2- and 3-azetidinyl, 2- and 3-oxetanyl, 2- and 3-thietanyl, 2- and 3-thietanyl-S-oxid (S-oxothietanyl), 2- and 3-thietanyl-S-dioxid (S-dioxothietanyl), 2- and 3-pyrrolidinyl, 2,3-dihydrofuran-4-yl and -5-yl, 2- and 3-tetrahydrofuranyl, 1,3-dioxolan-2-yl, thiolan-2-yl, S-oxothiolan-2-yl, S-dioxothiolan-2-yl, 4- and 5-oxazolidinyl, 1,3-dioxan-2-yl, 1- and 3-thiopyran-2-yl, S-oxothiopyranyl, and S-dioxothiopyranyl.

With respect to the variables, the particularly preferred embodiments of the intermediates correspond to those of the compounds of the formula I.

In a particular embodiment, the variables of the compounds of the formula I have the following meanings, these meanings, both on their own and in combination with one another, being particular embodiments of the compounds of formula I.

In a preferred embodiment, the compounds I are present in form of a mixture of compounds I.A and I.B, where compound I.A is present in an amount of more than 50% by weight, in particular of at least 70% by weight, specifically of at least 90% by weight, based on the total weight of compounds I.A and I.B.

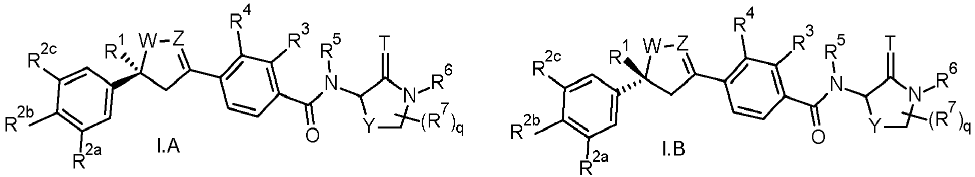

In one particularly preferred embodiment of the invention, the method comprises step of contacting the plant, parts of it, its propagation material, the pests, their food supply, habitat or breeding grounds a pesticidally effective amount of a compound of formula I.A.

Preferably W-Z in formula I is -O-N=; such compounds correspond to formula I.1.

In another embodiment W-Z in formula I is -CH₂-N=; such compounds correspond to formula I.2. In another embodiment W-Z in formula I is -CH₂-CH=; such compounds correspond to formula 1.3.

Preferred embodiments relate to each of following compounds of formula I, wherein the variables are as defined in the outset and the preferred embodiments:

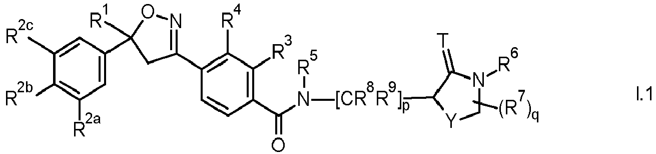

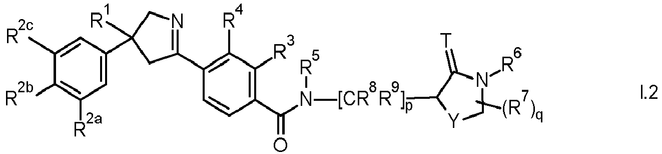

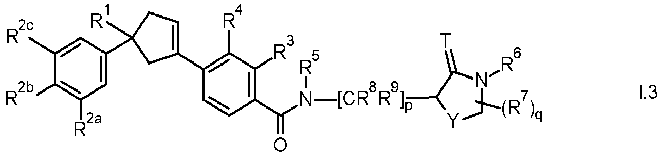

R¹ is preferably CF₃.

R^{2a} is preferably selected from F, Cl, Br, CF₃, and OCF₃.

R^{2b} and R^{2c} are independently preferably selected from H, F, Cl, Br, CF₃, and OCF₃.

Particularly preferred is each one of the following combinations of R^{2a}, R^{2b} and R^{2c} wherein each line of Table A denotes a substitution pattern of the phenyl ring ("A") bearing the R^{2a}, R^{2b} and R^{2c} moieties.

**Table A**

| No. | R^{2a} | R^{2b} | R^{2c} |
|---|---|---|---|
| A-1 | F | F | H |
| A-2 | F | H | F |
| A-3 | F | F | F |
| A-4 | F | Cl | F |
| A-5 | F | Br | F |
| A-6 | F | H | Cl |
| A-7 | F | H | Br |
| A-8 | Cl | F | H |
| A-9 | Cl | H | Cl |
| A-10 | Cl | Cl | Cl |
| A-11 | Cl | F | Cl |
| A-12 | Cl | Br | Cl |
| A-13 | Cl | H | Br |
| A-14 | Br | F | H |
| A-15 | Br | H | Br |
| A-16 | Br | F | Br |
| A-17 | Br | Cl | Br |
| A-18 | CF₃ | H | H |
| A-19 | CF₃ | H | F |
| A-20 | CF₃ | H | Cl |
| A-21 | CF₃ | H | Br |
| A-22 | CF₃ | H | CF₃ |
| A-23 | CF₃ | F | F |
| A-24 | CF₃ | F | Cl |
| A-25 | CF₃ | Cl | Cl |
| A-26 | CF₃ | F | H |
| A-27 | OCF₃ | H | F |
| A-28 | OCF₃ | H | Cl |
| A-29 | OCF₃ | F | H |
| A-30 | OCF₃ | H | CF₃ |
| A-31 | OCF₃ | H | H |

Groups A-8, A-9, and A-11 are more preferred patterns in formula I compounds. A-11 is particularly preferred.

R³ and R⁴ are preferably halogen such as Cl and F, NO₂, CN, SCH₃, OCH₃, CH₃, or fluoromethyl such as CHF₂, or CF₃. More preferably R⁴ is H, and R³ has one of the preferred meanings.

In another embodiment R³ and R⁴ together with the C-atoms they are bound to form a 5- or 6-membered saturated carbocyclic ring. In another embodiment R³ and R⁴ together with the C-atoms they are bound to form a 5- or 6-membered saturated heterocyclic ring, such as 2,3-dihydrofuranyl.

R⁵, R⁸, and R⁹ are independently from one another preferably H.

In one embodiment R⁶ is C₁-C₆-alkyl, C₁-C₄-haloalkyl, C₃-C₆-alkenyl, C₃-C₆-alkynyl, which groups may be substituted with C(=O)OR^{a1}, C(=O)N(R^{a2})R^{a3}, CH=NOR^{a1}, and phenyl, benzyl, which rings are unsubstituted or substituted with halogen, C₁-C₄-alkyl, or C₁-C₄-haloalkyl. Preferably R⁶ is C₁-C₆-alkyl.

R^{a1}, R^{a2}, and R^{a3}, are independently from one another preferably H, and C₁-C₄-alkyl.

Two R⁷ groups on the same atom denote preferably a (thio)carbonyl group.

In a preferred embodiment T denotes O.

In another embodiment T is S.

Preferred embodiments relate to each of following compounds of formula I, wherein the variables are as defined in the outset and the preferred embodiments:

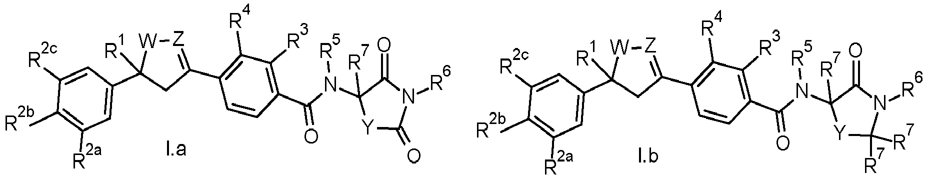

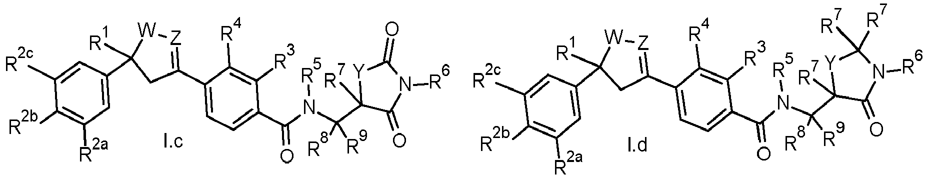

In one embodiment of formula I compounds Y is O.

In another embodiment of formula I compounds Y is S.

Accordingly, preferred embodiments relate to each of following compounds of formula I, wherein the variables are as defined in the outset and the preferred embodiments:

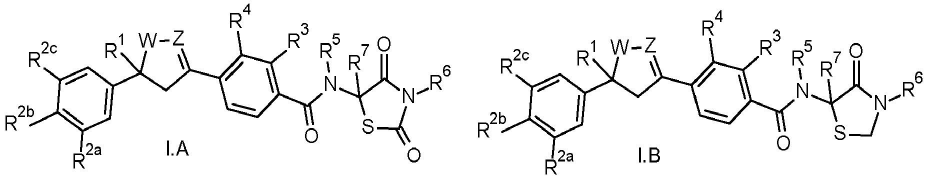

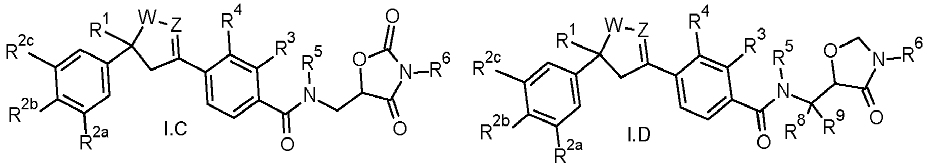

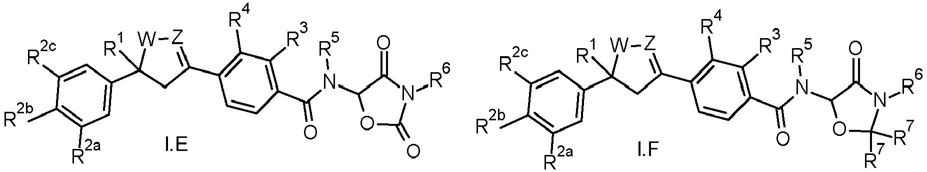

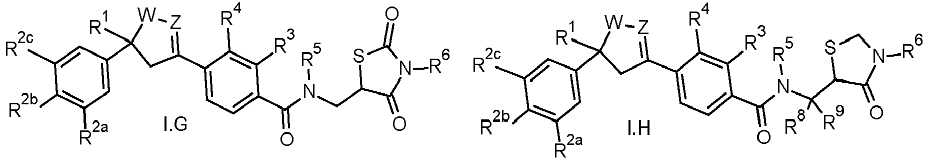

The Y containing ring is preferably thiazolidine-2,4-dion-5-yl, thiazolidin-4-on-5-yl, oxazolidin-4-on-5-yl, oxazolidine-2,4-dion-5-yl, which is substituted with R⁶.

Accordingly, preferred embodiments relate to each of following compounds of formula I, wherein the variables are as defined in the outset and the preferred embodiments:

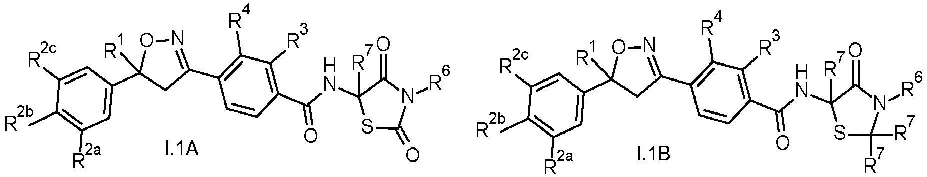

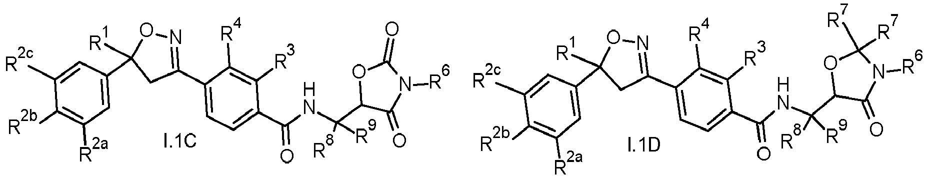

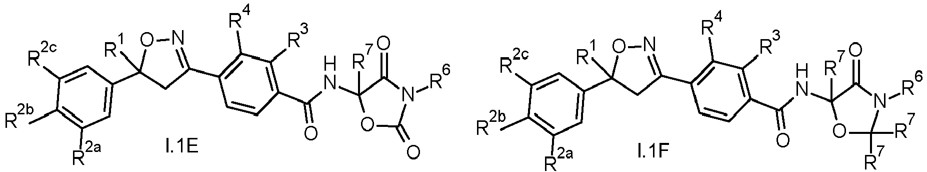

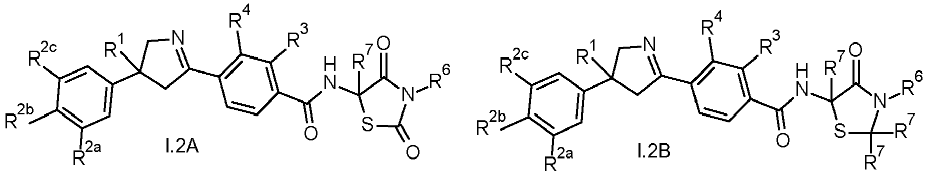

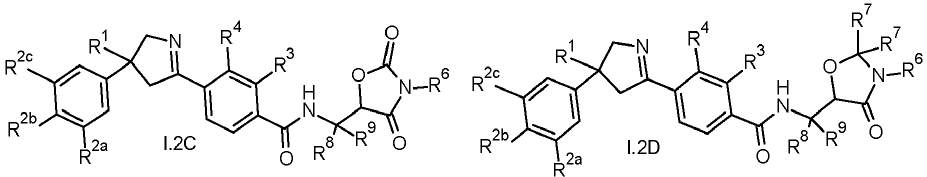

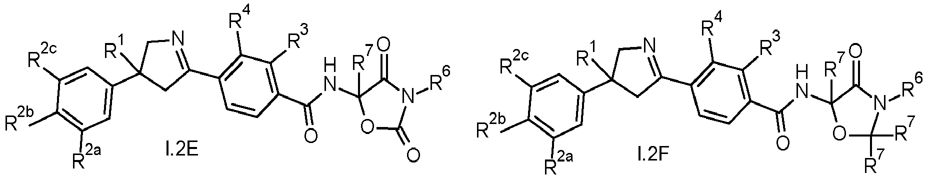

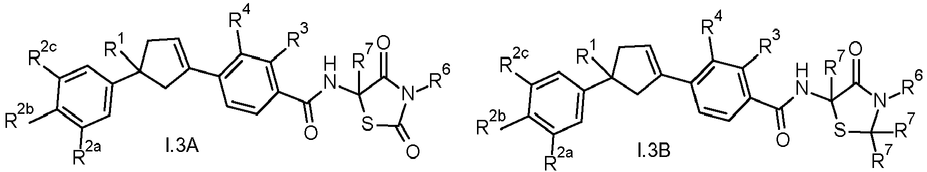

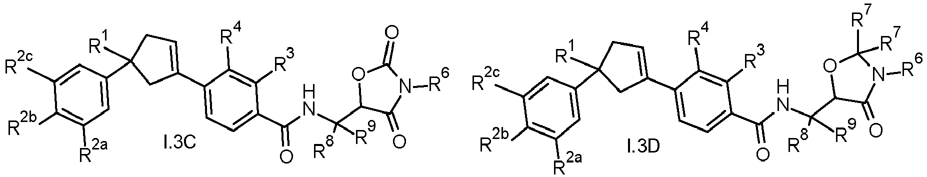

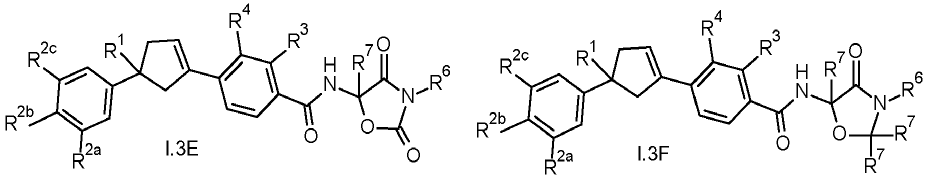

In particular with a view to their use, preference is given to the compounds of formula I compiled in the tables below, which compounds correspond to formulae I.1A, I.1B, I.1C, I.1D, 1.1 E, and I.1F, resp., wherein in each case R⁷, R⁸, and R⁹ are H. Each of the groups mentioned for a substituent in the tables is furthermore per se, independently of the combination in which it is mentioned, a particularly preferred aspect of the substituent in question.
Table 1 : Compounds of formula I.1A in which R⁶ is CH₃, and the other variables for a compound correspond in each case to one row of Table B
Table 2 : Compounds of formula I.1B in which R⁶ is CH₃, and the other variables for a compound correspond in each case to one row of Table B
Table 3 : Compounds of formula I.1C in which R⁶ is CH₃, and the other variables for a compound correspond in each case to one row of Table B
Table 4 : Compounds of formula I.1D in which R⁶ is CH₃, and the other variables for a compound correspond in each case to one row of Table B
Table 5 : Compounds of formula I.1E in which R⁶ is CH₃, and the other variables for a compound correspond in each case to one row of Table B
Table 6 : Compounds of formula I.1F in which R⁶ is CH₃, and the other variables for a compound correspond in each case to one row of Table B
Table 7 : Compounds of formula I.1A in which R⁶ is C₂H₅, and the other variables for a compound correspond in each case to one row of Table B
Table 8 : Compounds of formula I.1B in which R⁶ is C₂H₅, and the other variables for a compound correspond in each case to one row of Table B
Table 9 : Compounds of formula I.1C in which R⁶ is C₂H₅, and the other variables for a compound correspond in each case to one row of Table B
Table 10 : Compounds of formula I.1D in which R⁶ is C₂H₅, and the other variables for a compound correspond in each case to one row of Table B
Table 11 : Compounds of formula I.1E in which R⁶ is C₂H₅, and the other variables for a compound correspond in each case to one row of Table B
Table 12 : Compounds of formula I.1F in which R⁶ is C₂H₅, and the other variables for a compound correspond in each case to one row of Table B
Table 13 : Compounds of formula I.1A in which R⁶ is CH₂(CH₃)₂, and the other variables for a compound correspond in each case to one row of Table B
Table 14 : Compounds of formula I.1B in which R⁶ is CH₂(CH₃)₂, and the other variables for a compound correspond in each case to one row of Table B
Table 15 : Compounds of formula I.1C in which R⁶ is CH₂(CH₃)₂, and the other variables for a compound correspond in each case to one row of Table B
Table 16 : Compounds of formula I.1D in which R⁶ is CH₂(CH₃)₂, and the other variables for a compound correspond in each case to one row of Table B
Table 17 : Compounds of formula I.1E in which R⁶ is CH₂(CH₃)₂, and the other variables for a compound correspond in each case to one row of Table B
Table 18 : Compounds of formula I.1F in which R⁶ is CH₂(CH₃)₂, and the other variables for a compound correspond in each case to one row of Table B
Table 19 : Compounds of formula I.1A in which R⁶ is CH₂CH=CH₂, and the other variables for a compound correspond in each case to one row of Table B
Table 20 : Compounds of formula I.1B in which R⁶ is CH₂CH=CH₂, and the other variables for a compound correspond in each case to one row of Table B
Table 21 : Compounds of formula I.1C in which R⁶ is CH₂CH=CH₂, and the other variables for a compound correspond in each case to one row of Table B
Table 22 : Compounds of formula I.1D in which R⁶ is CH₂CH=CH₂, and the other variables for a compound correspond in each case to one row of Table B
Table 23 : Compounds of formula I.1E in which R⁶ is CH₂CH=CH₂, and the other variables for a compound correspond in each case to one row of Table B
Table 24 : Compounds of formula I.1F in which R⁶ is CH₂CH=CH₂, and the other variables for a compound correspond in each case to one row of Table B
Table 25 : Compounds of formula I.1A in which R⁶ is CH₂CF₃, and the other variables for a compound correspond in each case to one row of Table B
Table 26 : Compounds of formula I.1B in which R⁶ is CH₂CF₃, and the other variables for a compound correspond in each case to one row of Table B
Table 27 : Compounds of formula I.1C in which R⁶ is CH₂CF₃, and the other variables for a compound correspond in each case to one row of Table B
Table 28 : Compounds of formula I.1D in which R⁶ is CH₂CF₃, and the other variables for a compound correspond in each case to one row of Table B
Table 29 : Compounds of formula I.1E in which R⁶ is CH₂CF₃, and the other variables for a compound correspond in each case to one row of Table B
Table 30 : Compounds of formula I.1F in which R⁶ is CH₂CF₃, and the other variables for a compound correspond in each case to one row of Table B
Table 31 : Compounds of formula I.1A in which R⁶ is CH₂CH₂CF₃, and the other variables for a compound correspond in each case to one row of Table B
Table 32 : Compounds of formula I.1B in which R⁶ is CH₂CH₂CF₃, and the other variables for a compound correspond in each case to one row of Table B
Table 33 : Compounds of formula I.1C in which R⁶ is CH₂CH₂CF₃, and the other variables for a compound correspond in each case to one row of Table B
Table 34 : Compounds of formula I.1D in which R⁶ is CH₂CH₂CF₃, and the other variables for a compound correspond in each case to one row of Table B
Table 35 : Compounds of formula I.1E in which R⁶ is CH₂CH₂CF₃, and the other variables for a compound correspond in each case to one row of Table B
Table 36 : Compounds of formula I.1F in which R⁶ is CH₂CH₂CF₃, and the other variables for a compound correspond in each case to one row of Table B
Table 37 : Compounds of formula I.1A in which R⁶ is CH₂C₆H₅, and the other variables for a compound correspond in each case to one row of Table B
Table 38 : Compounds of formula I.1B in which R⁶ is CH₂C₆H₅, and the other variables for a compound correspond in each case to one row of Table B
Table 39 : Compounds of formula I.1C in which R⁶ is CH₂C₆H₅, and the other variables for a compound correspond in each case to one row of Table B
Table 40 : Compounds of formula I.1D in which R⁶ is CH₂C₆H₅, and the other variables for a compound correspond in each case to one row of Table B
Table 41 : Compounds of formula I.1E in which R⁶ is CH₂C₆H₅, and the other variables for a compound correspond in each case to one row of Table B
Table 42 : Compounds of formula I.1F in which R⁶ is CH₂C₆H₅, and the other variables for a compound correspond in each case to one row of Table B
Table 43 : Compounds of formula I.1A in which R⁶ is CH₂C(=O)OCH₃, and the other variables for a compound correspond in each case to one row of Table B
Table 44 : Compounds of formula I.1B in which R⁶ is CH₂C(=O)OCH₃, and the other variables for a compound correspond in each case to one row of Table B
Table 45 : Compounds of formula I.1C in which R⁶ is CH₂C(=O)OCH₃, and the other variables for a compound correspond in each case to one row of Table B
Table 46 : Compounds of formula I.1D in which R⁶ is CH₂C(=O)OCH₃, and the other variables for a compound correspond in each case to one row of Table B
Table 47 : Compounds of formula I.1E in which R⁶ is CH₂C(=O)OCH₃, and the other variables for a compound correspond in each case to one row of Table B
Table 48 : Compounds of formula I.1F in which R⁶ is CH₂C(=O)OCH₃, and the other variables for a compound correspond in each case to one row of Table B
Table 49 : Compounds of formula I.1A in which R⁶ is CH₂C(=O)OC₂H₅, and the other variables for a compound correspond in each case to one row of Table B
Table 50 : Compounds of formula I.1B in which R⁶ is CH₂C(=O)OC₂H₅, and the other variables for a compound correspond in each case to one row of Table B
Table 51 : Compounds of formula I.1C in which R⁶ is CH₂C(=O)OC₂H₅, and the other variables for a compound correspond in each case to one row of Table B
Table 52 : Compounds of formula I.1D in which R⁶ is CH₂C(=O)OC₂H₅, and the other variables for a compound correspond in each case to one row of Table B
Table 53 : Compounds of formula I.1E in which R⁶ is CH₂C(=O)OC₂H₅, and the other variables for a compound correspond in each case to one row of Table B
Table 54 : Compounds of formula I.1F in which R⁶ is CH₂C(=O)OC₂H₅, and the other variables for a compound correspond in each case to one row of Table B

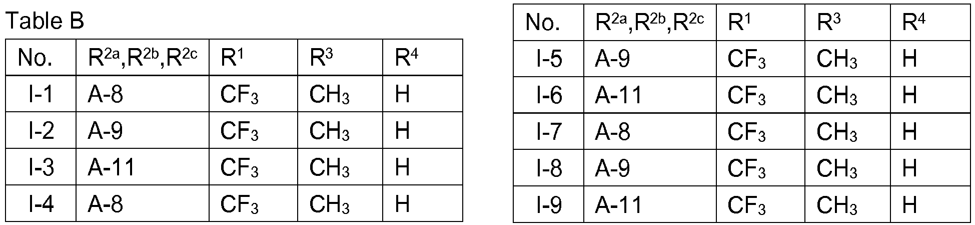

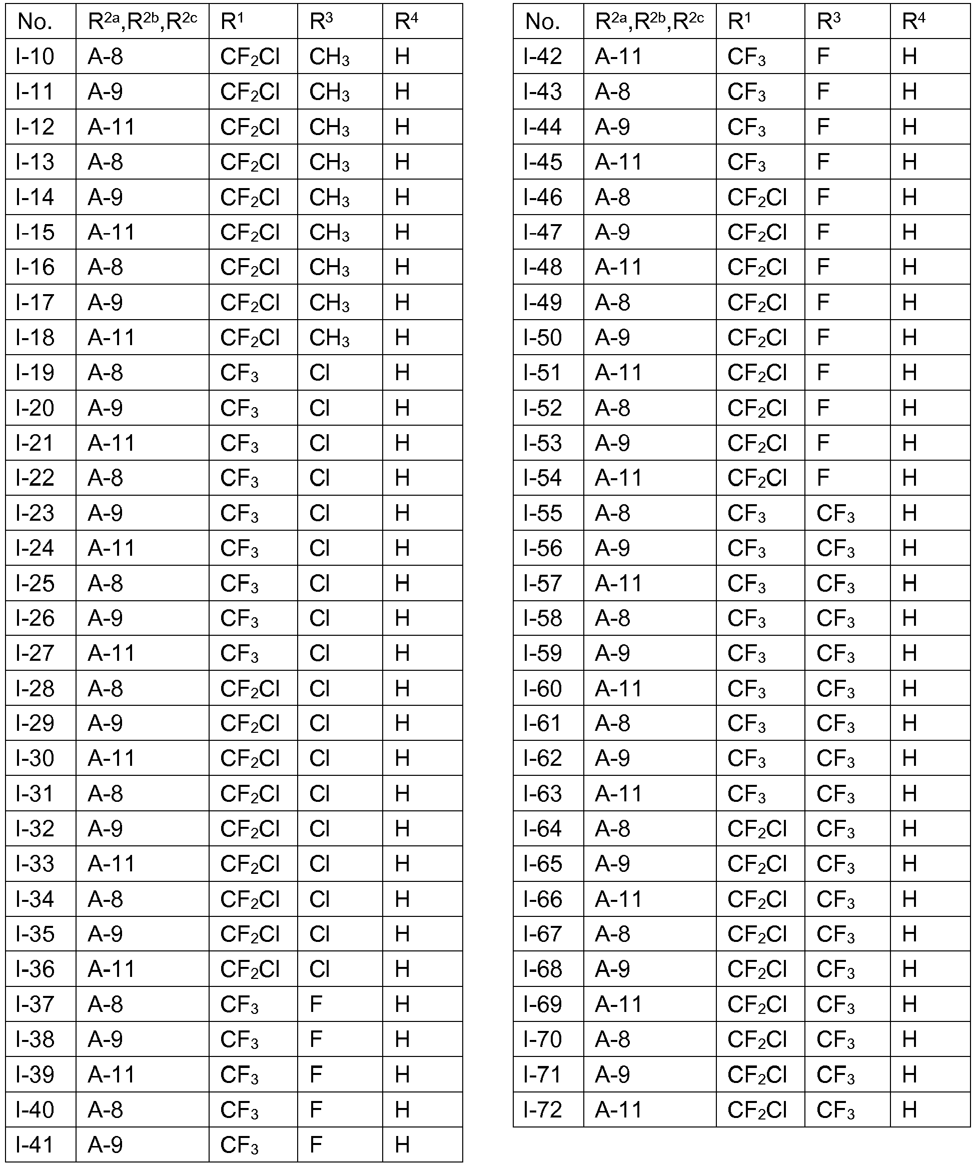

As used herein, the term "compound(s) of the invention" or "compound(s) according to the invention" refers to the compound(s) of formula (I) as defined above, which are also referred to as "compound(s) of formula I" or "compound(s) I" or "formula I compound(s)", and includes their salts, tautomers, stereoisomers, and N-oxides.

The invention also relates to a mixture of at least one compound of the invention with at least one mixing partner as defined herein. Preferred are binary mixtures of one compound of the invention as component I with one mixing partner as defined herein as component II. Preferred weight ratios for such binary mixtures are from 5000:1 to 1:5000, preferably from 1000:1 to 1:1000, more preferably from 100:1 to 1:100, particularly from 10:1 to 1:10. In such binary mixtures, components I and II may be used in equal amounts, or an excess of component I, or an excess of component II may be used.

Mixing partners can be selected from pesticides, in particular insecticides, nematicides, and acaricides, fungicides, herbicides, plant growth regulators, fertilizers. Preferred mixing partners are insecticides, nematicides and fungicides.

The following list M of pesticides, grouped and numbered according the Mode of Action Classification of the Insecticide Resistance Action Committee (IRAC), together with which the compounds of the invention can be used and with which potential synergistic effects might be produced, is intended to illustrate the possible combinations:
M.1 Acetylcholine esterase (AChE) inhibitors: carbamates, e.g. aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb and triazamate; organophosphates, e.g. acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos- methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;
M.2. GABA-gated chloride channel antagonists: cyclodiene organochlorine compounds, e.g. endosulfan, chlordane; phenylpyrazoles, e.g. ethiprole, fipronil, flufiprole, pyrafluprole, pyriprole;
M.3 Sodium channel modulators from the class of pyrethroids, e.g. acrinathrin, allethrin, d-cis-trans allethrin, d-trans allethrin, bifenthrin, kappa-bifenthrin, bioallethrin, bioallethrin S-cyclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin,metofluthrin, momfluorothrin, epsilon-momfluorothrin, permethrin, phenothrin, prallethrin, profluthrin, pyrethrin (pyrethrum), resmethrin, silafluofen, tefluthrin, kappa-tefluthrin, tetramethylfluthrin, tetramethrin, tralomethrin, and transfluthrin; sodium channel modulators, DDT, methoxychlor;
M.4 Nicotinic acetylcholine receptor agonists (nAChR): neonicotinoids, e.g. acetamiprid, clothianidin, cycloxaprid, dinotefuran, imidacloprid, nitenpyram, thiacloprid and thiamethoxam; 4,5-dihydro-N-nitro-1-(2-oxiranylmethyl)-1H-imidazol-2-amine, (2E-)-1-[(6-chloropyridin-3-yl)methyl]-N'-nitro-2-pentylidenehydrazinecarboximidamide; 1-[(6-chloropyridin-3-yl)methyl]-7-methyl-8-nitro-5-propoxy-1,2,3,5,6,7-hexahydroimidazo[1,2-a]pyridine; nicotine; sulfoxaflor; flupyradifurone; triflumezopyrim, (3R)-3-(2-chlorothiazol-5-yl)-8-methyl-5-oxo-6-phenyl-2,3-dihydrothiazolo[3,2-a]pyrimidin-8-ium-7-olate, (3S)-3-(6-chloro-3-pyridyl)-8-methyl-5-oxo-6-phenyl-2,3-dihydrothiazolo[3,2-a]pyrimidin-8-ium-7-olate, (3S)-8-methyl-5-oxo-6-phenyl-3-pyrimidin-5-yl-2,3-dihydrothiazolo[3,2-a]pyrimidin-8-ium-7-olate, (3R)-3-(2-chlorothiazol-5-yl)-8-methyl-5-oxo-6-[3-(trifluoromethyl)phenyl]-2,3-dihydrothiazolo[3,2-a]pyrimidin-8-ium-7-olate; (3R)-3-(2-chlorothiazol-5-yl)-6-(3,5-dichlorophenyl)-8-methyl-5-oxo-2,3-dihydrothiazolo[3,2-a]pyrimidin-8-ium-7-olate, (3R)-3-(2-chlorothiazol-5-yl)-8-ethyl-5-oxo-6-phenyl-2,3-dihydrothiazolo[3,2-a]pyrimidin-8-ium-7-olate;
M.5 Nicotinic acetylcholine receptor allosteric activators: spinosyns, e.g. spinosad, spinetoram;
M.6 Chloride channel activators from avermectins and milbemycins, e.g. abamectin, emamectin benzoate, ivermectin, lepimectin, milbemectin;
M.7 Juvenile hormone mimics, such as juvenile hormone analogues hydroprene, kinoprene, and methoprene; fenoxycarb, pyriproxyfen;
M.8 miscellaneous non-specific (multi-site) inhibitors, e.g. alkyl halides as methyl bromide and other alkyl halides, chloropicrin, sulfuryl fluoride, borax, tartar emetic;
M.9 Chordotonal organ TRPV channel modulators, e.g. pymetrozine; pyrifluquinazon;
M.10 Mite growth inhibitors, e.g. clofentezine, hexythiazox, and diflovidazin, etoxazole;
M.11 Microbial disruptors of insect midgut membranes, e.g. *bacillus thuringiensis* or *bacillus sphaericus* and the insecticdal proteins they produce such as *bacillus thuringiensis subsp. israelensis, bacillus sphaericus, bacillus thuringiensis subsp. aizawai, bacillus thuringiensis subsp. kurstakiand bacillus thuringiensis subsp. tenebrionis,* or the Bt crop proteins: Cry1Ab, Cry1Ac, Cry1Fa, Cry2Ab, mCry3A, Cry3Ab, Cry3Bb, Cry34/35Ab1;
M.12 Inhibitors of mitochondrial ATP synthase, e.g. diafenthiuron, or organotin miticides such as azocyclotin, cyhexatin, fenbutatin oxide, propargite, tetradifon;
M.13 Uncouplers of oxidative phosphorylation via disruption of the proton gradient, e.g. chlorfenapyr, DNOC, sulfluramid;
M.14 Nicotinic acetylcholine receptor (nAChR) channel blockers, e.g. nereistoxin analogues bensultap, cartap hydrochloride, thiocyclam, thiosultap-sodium;
M.15 Inhibitors of the chitin biosynthesis type 0, such as benzoylureas e.g. bistrifluron, chlorfluazuron, diflubenzuron, flucycloxuron, flufenoxuron, hexaflumuron, lufenuron, novaluron, noviflumuron, teflubenzuron, triflumuron;
M.16 Inhibitors of the chitin biosynthesis type 1, e.g. buprofezin;
M.17 Moulting disruptors, Dipteran, e.g. cyromazine;
M.18 Ecdyson receptor agonists such as diacylhydrazines, e.g. methoxyfenozide, tebufenozide, halofenozide, fufenozide, chromafenozide;
M.19 Octopamin receptor agonists, e.g. amitraz;
M.20 Mitochondrial complex III electron transport inhibitors, e.g. hydramethylnon, acequinocyl, fluacrypyrim; bifenazate;
M.21 Mitochondrial complex I electron transport inhibitors, e.g. METI acaricides and insecticides such as fenazaquin, fenpyroximate, pyrimidifen, pyridaben, tebufenpyrad, tolfenpyrad, or rotenone;
M.22 Voltage-dependent sodium channel blockers, e.g. indoxacarb, metaflumizone, or 2-[2-(4-cyanophenyl)-1-[3-(trifluoromethyl)phenyl]ethylidene]-N-[4-(difluoromethoxy)phenyl]-hydrazinecarboxamide, N-(3-chloro-2-methylphenyl)-2-[(4-chlorophenyl)[4-[methyl(methylsulfonyl)amino]phenyl]methylene]-hydrazinecarboxamide;
M.23 Inhibitors of the of acetyl CoA carboxylase, such as spirodiclofen, spiromesifen, spirotetramat; spiropidion;
M.24 Mitochondrial complex IV electron transport inhibitors, e.g. phosphine such as aluminium phosphide, calcium phosphide, phosphine or zinc phosphide, cyanide;
M.25 Mitochondrial complex II electron transport inhibitors, such as beta-ketonitrile derivatives, e.g. cyenopyrafen, cyflumetofen;
M.28 Ryanodine receptor-modulators from the class of diamides, e.g. flubendiamide, chlorantraniliprole, cyantraniliprole, tetraniliprole, (R)-3-chlor-N1-{2-methyl-4-[1,2,2,2-tetrafluoro-1-(trifluoromethyl)ethyl]phenyl}-N2-(1-methyl-2-methylsulfonylethyl)phthalamid, (S)-3-chloro-N1-{2-methyl-4-[1,2,2,2-tetrafluoro-1-(trifluoromethyl)ethyl]phenyl}-N2-(1-methyl-2-methylsulfonylethyl)phthalamid, cyclaniliprole, methyl-2-[3,5-dibromo-2-({[3-bromo-1-(3-chlorpyridin-2-yl)-1H-pyrazol-5-yl]carbonyl}amino)benzoyl]-1,2-dimethylhydrazinecarboxylate; N-[2-(5-Amino-1,3,4-thiadiazol-2-yl)-4-chloro-6-methylphenyl]-3-bromo-1-(3-chloro-2-pyridinyl)-1 H-pyrazole-5-carboxamide; 3-Chloro-1-(3-chloro-2-pyridinyl)-N-[2,4-dichloro-6-[[(1-cyano-1-methylethyl)amino]carbonyl]phenyl]-1H-pyrazole-5-carboxamide; tetrachlorantraniliprole; N-[4-Chloro-2-[[(1,1-dimethylethyl)amino]carbonyl]-6-methylphenyl]-1-(3-chloro-2-pyridinyl)-3-(fluoromethoxy)-1H-pyrazole-5-carboxamide;
M.28.6: cyhalodiamide;
M.29: Chordotonal organ Modulators - undefined target site, e.g. flonicamid;
M.UN. insecticidal active compounds of unknown mode of action, e.g. afidopyropen, afoxolaner, azadirachtin, amidoflumet, benzoximate, broflanilide, bromopropylate, chinomethionat, cryolite, dicloromezotiaz, dicofol, dimpropyridaz, flufenerim, flometoquin, fluensulfone, fluhexafon, fluopyram, fluralaner, metaldehyde, metoxadiazone, piperonyl butoxide, pyflubumide, pyridalyl, tioxazafen, 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 1-[2-fluoro-4-methyl-5-[(2,2,2-trifluoroethyl)sulfinyl]phenyl]-3-(trifluoromethyl)-1H-1,2,4-triazole-5-amine, or actives on basis of *bacillus firmus* (Votivo, I-1582); flupyrimin; fluazaindolizine; 4-[5-(3,5-dichlorophenyl)-5-(trifluoromethyl)-4H-isoxazol-3-yl]-2-methyl-N-(1-oxothietan-3-yl)benzamide; fluxametamide; 5-[3-[2,6-dichloro-4-(3,3-dichloroallyloxy)phenoxy]propoxy]-1H-pyrazole; 4-cyano-N-[2-cyano-5-[[2,6-dibromo-4-[1,2,2,3,3,3-hexafluoro-1-(trifluoromethyl)propyl]phenyl]carbamoyl]phenyl]-2-methyl-benzamide; 4-cyano-3-[(4-cyano-2-methyl-benzoyl)amino]-N-[2,6-dichloro-4-[1,2,2,3,3,3-hexafluoro-1-(trifluoromethyl)propyl]phenyl]-2-fluoro-benzamide; N-[5-[[2-chloro-6-cyano-4-[1,2,2,3,3,3-hexafluoro-1-(trifluoromethyl)propyl]phenyl]carbamoyl]-2-cyano-phenyl]-4-cyano-2-methyl-benzamide; N-[5-[[2-bromo-6-chloro-4-[2,2,2-trifluoro-1-hydroxy-1-(trifluoromethyl)ethyl]phenyl]carbamoyl]-2-cyano-phenyl]-4-cyano-2-methyl-benzamide; N-[5-[[2-bromo-6-chloro-4-[1,2,2,3,3,3-hexafluoro-1-(trifluoromethyl)propyl]phenyl]carbamoyl]-2-cyano-phenyl]-4-cyano-2-methyl-benzamide; 4-cyano-N-[2-cyano-5-[[2,6-dichloro-4-[1,2,2,3,3,3-hexafluoro-1-(trifluoromethyl)propyl]phenyl]carbamoyl]phenyl]-2-methyl-benzamide; 4-cyano-N-[2-cyano-5-[[2,6-dichloro-4-[1,2,2,2-tetrafluoro-1-(trifluoromethyl)ethyl]phenyl]carbamoyl]phenyl]-2-methyl-benzamide; N-[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide; 2-(1,3-Dioxan-2-yl)-6-[2-(3-pyridinyl)-5-thiazolyl]-pyridine; 2-[6-[2-(5-Fluoro-3-pyridinyl)-5-thiazolyl]-2-pyridinyl]-pyrimidine; 2-[6-[2-(3-Pyridinyl)-5-thiazolyl]-2-pyridinyl]-pyrimidine; N-Methylsulfonyl-6-[2-(3-pyridyl)thiazol-5-yl]pyridine-2-carboxamide; N-Methylsulfonyl-6-[2-(3-pyridyl)thiazol-5-yl]pyridine-2-carboxamide; 1-[(6-Chloro-3-pyridinyl)methyl]-1,2,3,5,6,7-hexahydro-5-methoxy-7-methyl-8-nitro-imidazo[1,2-a]pyridine; 1-[(6-Chloropyridin-3-yl)methyl]-7-methyl-8-nitro-1,2,3,5,6,7-hexahydroimidazo[1,2-a]pyridin-5-ol; N-(1-methylethyl)-2-(3-pyridinyl)-2H-indazole-4-carboxamide; N-cyclopropyl-2-(3-pyridinyl)-2H-indazole-4-carboxamide; N-cyclohexyl-2-(3-pyridinyl)-2H-indazole-4-carboxamide; 2-(3-pyridinyl)-N-(2,2,2-trifluoroethyl)-2H-indazole-4-carboxamide; 2-(3-pyridinyl)-N-[(tetrahydro-2-furanyl)methyl]-2H-indazole-5-carboxamide; methyl 2-[[2-(3-pyridinyl)-2H-indazol-5-yl]carbonyl]hydrazinecarboxylate; N-[(2,2-difluorocyclopropyl)methyl]-2-(3-pyridinyl)-2H-indazole-5-carboxamide; N-(2,2-difluoropropyl)-2-(3-pyridinyl)-2H-indazole-5-carboxamide; 2-(3-pyridinyl)-N-(2-pyrimidinylmethyl)-2H-indazole-5-carboxamide; N-[(5-methyl-2-pyrazinyl)methyl]-2-(3-pyridinyl)-2H-indazole-5-carboxamide, tyclopyrazoflor; sarolaner, lotilaner; N-[4-chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; 2-(3-ethylsulfonyl-2-pyridyl)-3-methyl-6-(trifluoromethyl)imidazo[4,5-b]pyridine, 2-[3-ethylsulfonyl-5-(trifluoromethyl)-2-pyridyl]-3-methyl-6-(trifluoromethyl)imidazo[4,5-b]pyridine; Isocycloseram; N-[4-chloro-3-(cyclopropylcarbamoyl)phenyl]-2-methyl-5-(1,1,2,2,2-pentafluoroethyl)-4-(trifluoromethyl)pyrazole-3-carboxamide; N-[4-chloro-3-[(1-cyanocyclopropyl)carbamoyl]phenyl]-2-methyl-5-(1,1,2,2,2-pentafluoroethyl)-4-(trifluoromethyl)pyrazole-3-carboxamide; acynonapyr; benzpyrimoxan; tigolaner; oxazosulfyl; [(2S,3R,4R,5S,6S)-3,5-dimethoxy-6-methyl-4-propoxy-tetrahydropyran-2-yl]N-[4-[1-[4-(trifluoromethoxy)phenyl]-1,2,4-triazol-3-yl]phenyl]carbamate; [(2S,3R,4R,5S,6S)-3,4,5-trimethoxy-6-methyl-tetrahydropyran-2-yl] N-[4-[1-[4-(trifluoromethoxy)phenyl]-1,2,4-triazol-3-yl]phenyl]carbamate; [(2S,3R,4R,5S,6S)-3,5-dimethoxy-6-methyl-4-propoxy-tetrahydropyran-2-yl] N-[4-[1-[4-(1,1,2,2,2-pentafluoroethoxy)phenyl]-1,2,4-triazol-3-yl]phenyl]carbamate; [(2S,3R,4R,5S,6S)-3,4,5-trimethoxy-6-methyl-tetrahydropyran-2-yl] N-[4-[1-[4-(1,1,2,2,2-pentafluoroethoxy)phenyl]-1,2,4-triazol-3-yl]phenyl]carbamate; (2Z)-3-(2-isopropylphenyl)-2-[(E)-[4-[1-[4-(trifluoromethoxy)phenyl]-1,2,4-triazol-3-yl]phenyl]methylenehydrazono]thiazolidin-4-one; (2Z)-3-(2-isopropylphenyl)-2-[(E)-[4-[1-[4-(1,1,2,2,2-pentafluoroethoxy)phenyl]-1,2,4-triazol-3-yl]phenyl]methylenehydrazono]thiazolidin-4-one; 2-(6-chloro-3-ethylsulfonyl-imidazo[1,2-a]pyridin-2-yl)-3-methyl-6-(trifluoromethyl)imidazo[4,5-b]pyridine, 2-(6-bromo-3-ethylsulfonyl-imidazo[1,2-a]pyridin-2-yl)-3-methyl-6-(trifluoromethyl)imidazo[4,5-b]pyridine, 2-(3-ethylsulfonyl-6-iodo-imidazo[1,2-a]pyridin-2-yl)-3-methyl-6-(trifluoromethyl)imidazo[4,5-b]pyridine, 2-[3-ethylsulfonyl-6-(trifluoromethyl)imidazo[1,2-a]pyridin-2-yl]-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(3-ethylsulfonyl-7-iodo-imidazo[1,2-a]pyridin-2-yl)-3-methyl-6-(trifluoromethyl)imidazo[4,5-b]pyridine, 3-ethylsulfonyl-6-iodo-2-[3-methyl-6-(trifluoromethyl)imidazo[4,5-b]pyridin-2-yl]imidazo[1,2-a]pyridine-8-carbonitrile, 2-[3-ethylsulfonyl-8-fluoro-6-(trifluoromethyl)imidazo[1,2-a]pyridin-2-yl]-3-methyl-6-(trifluoromethyl)imidazo[4,5-b]pyridine, 2-[3-ethylsulfonyl-7-(trifluoromethyl)imidazo[1,2-a]pyridin-2-yl]-3-methyl-6-(trifluoromethylsulfinyl)imidazo[4,5-b]pyridine, 2-[3-ethylsulfonyl-7-(trifluoromethyl)imidazo[1,2-a]pyridin-2-yl]-3-methyl-6-(trifluoromethyl)imidazo[4,5-c]pyridine, or 2-(6-bromo-3-ethylsulfonyl-imidazo[1,2-a]pyridin-2-yl)-6-(trifluoromethyl)pyrazolo[4,3-c]pyridine.

The commercially available compounds of the group M listed above may be found in The Pesticide Manual, 17th Edition, C. MacBean, British Crop Protection Council (2015) among other publications. The online Pesticide Manual is updated regularly and is accessible through http://bcpcdata.com/pesticide-manual.html. Another online data base for pesticides providing the ISO common names is http://www.alanwood.net/pesticides. The active compounds described by IUPAC nomenclature are known from WO2010/069266, WO2011/069456, CN103814937; CN105367557, CN105481839, WO 2013/003977, WO 2007/101369, WO2018177970, CN10171577, CN102126994, WO 2014/191271, WO2007/101540, WO2005/077934, WO2007/043677, WO2007/006670, WO2013/024009, WO 2013/024010, WO2011/085575, WO2008/134969, US2011/046186, WO2012/034403, WO2012/034472, WO2006/089633, WO2008/067911, WO2006/043635, WO2009/124707, WO2012/029672, WO2013/055584, WO2013/050317, WO2014/126208, WO2010/060379, WO2010/018714, WO2010/127926, WO2010/006713, WO2012/000896, WO2007/101369, WO2015/038503, US2014/0213448, WO2014/036056, WO2014/090918, EP2910126, WO2015/059039 WO2015/190316, WO2013/050302, WO2012/126766, WO2011/105506, WO2016/104516, WO2016/174049, WO2017/104592, WO2009/102736, WO2013116053, WO2013/050302, WO2018/052136.

The following list of fungicides, in conjunction with which the compounds of the invention can be used, is intended to illustrate the possible combinations but does not limit them:
A) Respiration inhibitors
   - Inhibitors of complex III at Qₒ site: azoxystrobin, coumethoxystrobin, coumoxystrobin, dimoxystrobin, enestroburin, fenaminstrobin, fenoxystrobin/flufenoxystrobin, fluoxastrobin, kresoxim-methyl, mandestrobin, metominostrobin, orysastrobin, picoxystrobin, pyraclostrobin, pyrametostrobin, pyraoxystrobin, trifloxystrobin, 2-(2-(3-(2,6-dichlorophenyl)-1-methyl-allylidene-aminooxymethyl)-phenyl)-2-methoxyimino-*N*-methyl-acetamide, pyribencarb, triclopyricarb/chlorodincarb, famoxadone, fenamidone, methyl-*N*-[2-[(1,4-dimethyl-5-phenyl-pyrazol-3-yl)oxylmethyl]phenyl]-*N*-methoxy-carbamate, metyltetrapole, (*Z*,2*E*)-5-[1-(2,4-dichlorophenyl)pyrazol-3-yl]-oxy-2-methoxyimino-*N*,3-dimethyl-pent-3-enamide, (*Z*,2*E*)-5-1[1-(4-chlorophenyl)pyrazol-3-yl]oxy-2-methoxyimino-*N*,3-dimethyl-pent-3-enamide, pyriminostrobin, bifujunzhi, 2-(ortho-((2,5-dimethylphenyl-oxymethylen)phenyl)-3-methoxy-acrylic acid methylester;
   - inhibitors of complex III at Qᵢ site: cyazofamid, amisulbrom, [(6*S*,7*R*,8*R*)-8-benzyl-3-[(3-hydroxy-4-methoxy-pyridine-2-carbonyl)amino]-6-methyl-4,9-dioxo-1,5-dioxonan-7-yl] 2-methylpropanoate, fenpicoxamid, florylpicoxamid;
   - inhibitors of complex II: benodanil, benzovindiflupyr, bixafen, boscalid, carboxin, fenfuram, fluopyram, flutolanil, fluxapyroxad, furametpyr, isofetamid, isopyrazam, mepronil, oxycarboxin, penflufen, penthiopyrad, pydiflumetofen, pyraziflumid, sedaxane, tecloftalam, thifluzamide, inpyrfluxam, pyrapropoyne, fluindapyr, N-[2-[2-chloro-4-(trifluoromethyl)phenoxy]phenyl]-3-(difluoromethyl)-5-fluoro-1-methyl-pyrazole-4-carboxamide, methyl (*E*)-2-[2-[(5-cyano-2-methylphenoxy)methyl]phenyl]-3-methoxy-prop-2-enoate, isoflucypram, 2-(difluoromethyl)-*N-*(1,1,3-trimethyl-indan-4-yl)pyridine-3-carboxamide, 2-(difluoromethyl)-*N-*[(3*R*)-1,1,3-trimethylindan-4-yl]pyridine-3-carboxamide, 2-(difluoromethyl)-*N*-(3-ethyl-1,1-dimethyl-indan-4-yl)pyridine-3-carboxamide, 2-(difluoromethyl)-*N-*[(3*R*)-3-ethyl-1,1-dimethyl-indan-4-yl]pyridine-3-carboxamide, 2-(difluoromethyl)-*N*-(1,1-dimethyl-3-propyl-indan-4-yl)pyridine-3-carboxamide, 2-(difluoromethyl)-*N*-[(3*R*)-1,1-dimethyl-3-propyl-indan-4-yl]pyridine-3-carboxamide, 2-(difluoromethyl)-*N*-(3-isobutyl-1,1-dimethyl-indan-4-yl)pyridine-3-carboxamide, 2-(difluoromethyl)-*N-*[(3*R*)-3-isobutyl-1,1-dimethyl-indan-4-yl]pyridine-3-carboxamide;
   - other respiration inhibitors: diflumetorim; nitrophenyl derivates: binapacryl, dinobuton, dinocap, fluazinam, meptyldinocap, ferimzone; organometal compounds: fentin salts, e. g. fentinacetate, fentin chloride, or fentin hydroxide; ametoctradin; silthiofam;
B) Sterol biosynthesis inhibitors (SBI fungicides)
   - C14 demethylase inhibitors: azaconazole, bitertanol, bromuconazole, cyproconazole, difenoconazole, diniconazole, diniconazole-M, epoxiconazole, fenbuconazole, fluquinconazole, flusilazole, flutriafol, hexaconazole, imibenconazole, ipconazole, metconazole, myclobutanil, oxpoconazole, paclobutrazole, penconazole, propiconazole, prothioconazole, simeconazole, tebuconazole, tetraconazole, triadimefon, triadimenol, triticonazole, uniconazole, 2-(2,4-difluorophenyl)-1,1-difluoro-3-(tetrazol-1-yl)-1-[5-[4-(2,2,2-trifluoroethoxy)phenyl]-2-pyridyl]propan-2-ol, 2-(2,4-difluorophenyl)-1,1-difluoro-3-(tetrazol-1-yl)-1-[5-[4-(trifluoromethoxy)phenyl]-2-pyridyl]propan-2-ol, ipfentrifluconazole, mefentrifluconazole, 2-(chloromethyl)-2-methyl-5-(p-tolylmethyl)-1-(1,2,4-triazol-1-ylmethyl)cyclopentanol; imidazoles: imazalil, pefurazoate, prochloraz, triflumizol; pyrimidines, pyridines, piperazines: fenarimol, pyrifenox, triforine, [3-(4-chloro-2-fluorophenyl)-5-(2,4-difluorophenyl)isoxazol-4-yl]-(3-pyridyl)methanol;
   - Delta14-reductase inhibitors: aldimorph, dodemorph, dodemorph-acetate, fenpropimorph, tridemorph, fenpropidin, piperalin, spiroxamine;
   - Inhibitors of 3-keto reductase: fenhexamid;
   - Other Sterol biosynthesis inhibitors: chlorphenomizole;
C) Nucleic acid synthesis inhibitors
   - phenylamides or acyl amino acid fungicides: benalaxyl, benalaxyl-M, kiralaxyl, metalaxyl, metalaxyl-M, ofurace, oxadixyl;
   - other nucleic acid synthesis inhibitors: hymexazole, octhilinone, oxolinic acid, bupirimate, 5-fluorocytosine, 5-fluoro-2-(p-tolylmethoxy)pyrimidin-4-amine, 5-fluoro-2-(4-fluorophenylmethoxy)pyrimidin-4-amine, 5-fluoro-2-(4-chlorophenylmethoxy)pyrimidin-4 amine;
D) Inhibitors of cell division and cytoskeleton
   - tubulin inhibitors: benomyl, carbendazim, fuberidazole, thiabendazole, thiophanate-methyl, pyridachlometyl, *N*-ethyl-2-[(3-ethynyl-8-methyl-6-quinolyl)oxy]butanamide, *N*-ethyl-2-[(3-ethynyl-8-methyl-6-quinolyl)oxy]-2-methylsulfanyl-acetamide, 2-[(3-ethynyl-8-methyl-6-quinolyl)oxy]-*N*-(2-fluoroethyl)butanamide, 2-[(3-ethynyl-8-methyl-6-quinolyl)oxy]-*N*-(2-fluoroethyl)-2-methoxy-acetamide, 2-[(3-ethynyl-8-methyl-6-quinolyl)oxy]-*N*-propyl-butanamide, 2-[(3-ethynyl-8-methyl-6-quinolyl)oxy]-2-methoxy-*N*-propyl-acetamide, 2-[(3-ethynyl-8-methyl-6-quinolyl)oxy]-2-methylsulfanyl-*N*-propyl-acetamide, 2-[(3-ethynyl-8-methyl-6-quinolyl)oxy]-*N*-(2-fluoroethyl)-2-methylsulfanyl-acetamide, 4-(2-bromo-4-fluoro-phenyl)-*N*-(2-chloro-6-fluoro-phenyl)-2,5-dimethyl-pyrazol-3-amine;
   - other cell division inhibitors: diethofencarb, ethaboxam, pencycuron, fluopicolide, zoxamide, metrafenone, pyriofenone;
E) Inhibitors of amino acid and protein synthesis
   - methionine synthesis inhibitors: cyprodinil, mepanipyrim, pyrimethanil;
   - protein synthesis inhibitors: blasticidin-S, kasugamycin, kasugamycin hydrochloride-hydrate, mildiomycin, streptomycin, oxytetracyclin;
F) Signal transduction inhibitors
   - MAP / histidine kinase inhibitors: fluoroimid, iprodione, procymidone, vinclozolin, fludioxonil;
   - G protein inhibitors: quinoxyfen;
G) Lipid and membrane synthesis inhibitors
   - Phospholipid biosynthesis inhibitors: edifenphos, iprobenfos, pyrazophos, isoprothiolane;
   - lipid peroxidation: dicloran, quintozene, tecnazene, tolclofos-methyl, biphenyl, chloroneb, etridiazole;
   - phospholipid biosynthesis and cell wall deposition: dimethomorph, flumorph, mandipropamid, pyrimorph, benthiavalicarb, iprovalicarb, valifenalate;
   - compounds affecting cell membrane permeability and fatty acides: propamocarb;
   - inhibitors of oxysterol binding protein: oxathiapiprolin, fluoxapiprolin, 4-[1-[2-[3-(difluoromethyl)-5-methyl-pyrazol-1-yl]acetyl]-4-piperidyl]-*N*-tetralin-1-yl-pyridine-2-carboxamide, 4-[1-[2-[3,5-bis(difluoromethyl)pyrazol-1-yl]acetyl]-4-piperidyl]-*N*-tetralin-1-yl-pyridine-2-carboxamide, 4-[1-[2-[3-(difluoromethyl)-5-(trifluoromethyl)pyrazol-1-yl]acetyl]-4-piperidyl]-*N*-tetralin-1-yl-pyridine-2-carboxamide, 4-[1-[2-[5-cyclopropyl-3-(difluoromethyl)pyrazol-1-yl]acetyl]-4-piperidyl]-*N-*tetralin-1-yl-pyridine-2-carboxamide, 4-[1-[2-[5-methyl-3-(trifluoromethyl)pyrazol-1-yl]acetyl]-4-piperidyl]-*N*-tetralin-1-yl-pyridine-2-carboxamide, 4-[1-[2-[5-(difluoromethyl)-3-(trifluoromethyl)pyrazol-1-yl]acetyl]-4-piperidyl]-*N*-tetralin-1-yl-pyridine-2-carboxamide, 4-[1-[2-[3,5-bis(trifluoromethyl)pyrazol-1-yl]acetyl]-4-piperidyl]-*N*-tetralin-1-yl-pyridine-2-carboxamide, (4-[1-[2-[5-cyclopropyl-3-(trifluoromethyl)pyrazol-1-yl]acetyl]-4-piperidyl]-*N*-tetralin-1-yl-pyridine-2-carboxamide;
H) Inhibitors with Multi Site Action:
   - inorganic active substances: Bordeaux mixture, copper, copper acetate, copper hydroxide, copper oxychloride, basic copper sulfate, sulfur;
   - thio- and dithiocarbamates: ferbam, mancozeb, maneb, metam, metiram, propineb, thiram, zineb, ziram;
   - organochlorine compounds: anilazine, chlorothalonil, captafol, captan, folpet, dichlofluanid, dichlorophen, hexachlorobenzene, pentachlorphenole and its salts, phthalide, tolylfluanid;
   - guanidines and others: guanidine, dodine, dodine free base, guazatine, guazatine-acetate, iminoctadine, iminoctadine-triacetate, iminoctadine-tris(albesilate), dithianon, 2,6-dimethyl-1*H*,5*H-*[1,4]dithiino[2,3-c:5,6-c']dipyrrole-1 ,3,5,7(2*H*,6*H*-tetraone;
I) Cell wall synthesis inhibitors
   - inhibitors of glucan synthesis: validamycin, polyoxin B;
   - melanin synthesis inhibitors: pyroquilon, tricyclazole, carpropamid, dicyclomet, fenoxanil;
J) Plant defence inducers
   - acibenzolar-S-methyl, probenazole, isotianil, tiadinil, prohexadione-calcium; phosphonates: fosetyl, fosetyl-aluminum, phosphorous acid and its salts, calcium phosphonate, potassium phosphonate, potassium or sodium bicarbonate, 4-cyclopropyl-*N*(2,4-dimethoxyphenyl)thiadiazole-5-carboxamide;
K) Unknown mode of action
   - bronopol, chinomethionat, cyflufenamid, cymoxanil, dazomet, debacarb, diclocymet, diclomezine, difenzoquat, difenzoquat-methylsulfate, diphenylamin, fenitropan, fenpyrazamine, flumetover, flusulfamide, flutianil, harpin, methasulfocarb, nitrapyrin, nitrothal-isopropyl, tolprocarb, oxin-copper, proquinazid, tebufloquin, tecloftalam, triazoxide, *N'*-(4-(4-chloro-3-trifluoromethyl-phenoxy)-2,5-dimethyl-phenyl)-*N*-ethyl-*N*-methyl formamidine, *N'-*(4-(4-fluoro-3-trifluoromethyl-phenoxy)-2,5-dimethyl-phenyl)-*N*-ethyl-*N*-methyl formamidine, *N'-*[4-[[3-[(4-chlorophenyl)methyl]-1,2,4-thiadiazol-5-yl]oxy]-2,5-dimethyl-phenyl]-*N*-ethyl-*N*-methyl-formamidine, *N'*-(5-bromo-6-indan-2-yloxy-2-methyl-3-pyridyl)-*N*-ethyl-*N*-methyl-formamidine, *N*'-[5-bromo-6-[1-(3,5-difluorophenyl)ethoxy]-2-methyl-3-pyridyl]-*N*-ethyl-*N*-methyl-formamidine, *N'*-[5-bromo-6-(4-isopropylcyclohexoxy)-2-methyl-3-pyridyl]-*N*-ethyl-*N*-methyl-formamidine, *N'*-[5-bromo-2-methyl-6-(1-phenylethoxy)-3-pyridyl]-*N*-ethyl-*N*-methyl-formamidine, *N'-*(2-methyl-5-trifluoromethyl-4-(3-trimethylsilanyl-propoxy)-phenyl)-*N*-ethyl-*N*-methyl formamidine, *N'*-(5-difluoromethyl-2-methyl-4-(3-trimethyisilanyl-propoxy)-phenyl)-*N*-ethyl-*N*-methy formamidine, 2-(4-chloro-phenyl)-*N*-[4-(3,4-dimethoxy-phenyl)-isoxazol-5-yl]-2-prop-2-ynyloxy-acetamide, 3-[5-(4-chloro-phenyl)-2,3-dimethyl-isoxazolidin-3-yl]-pyridine (pyrisoxazole), 3-[5-(4-methylphenyl)-2,3-dimethyl-isoxazolidin-3 yl]-pyridine, 5-chloro-1-(4,6-dimethoxy-pyrimidin-2-yl)-2-methyl-1*H*-benzoimidazole, ethyl (*Z*)-3-amino-2-cyano-3-phenyl-prop-2-enoate, picarbutrazox, pentyl *N-*[6-[[(2)-[(1-methyltetrazol-5-yl)-phenyl-methylene]amino]oxymethyl]-2-pyridyl]carbamate, but-3-ynyl *N*-[6-[[(*Z*)-[(1-methyltetrazol-5-yl)-phenyl-methylene]amino]oxymethyl]-2-pyridyl]carbamate, ipflufenoquin, quinofumelin, 2-(6-benzyl-2-pyridyl)quinazoline, 2-[6-(3-fluoro-4-methoxy-phenyl)-5-methyl-2-pyridyl]quinazoline, dichlobentiazox, *N'-*(2,5-dimethyl-4-phenoxy-phenyl)-*N*-thyl-*N*-methyl-formamidine, pyrifenamine.

The fungicides described by common names, their preparation and their activity e.g. against harmful fungi is known (cf.: http://www.alanwood.net/pesticides/); these substances are commercially available.

The active substances referred to as component 2, their preparation and their activity e. g. against harmful fungi is known (cf.: http://www.alanwood.net/pesticides/); these substances are commercially available. The compounds described by IUPAC nomenclature are also known (cf. Can. J. Plant Sci. 48(6), 587-94, 1968; EP 141 317; EP 152 031; EP 226 917; EP 243 970; EP 256 503; EP 428 941; EP 532 022; EP 1 028 125; EP 1 035 122; EP 1 201 648; EP 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441). Some compounds are identified by their CAS Registry Number which is separated by hyphens into three parts, the first consisting from two up to seven digits, the second consisting of two digits, and the third consisting of a single digit.

### Biopesticides

Suitable mixing partners for the compounds of the invention also include biopesticides. Biopesticides have been defined as a form of pesticides based on micro-organisms (bacteria, fungi, viruses, nematodes, etc.) or natural products (compounds, such as metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular.
(2) Biochemical pesticides are naturally occurring substances or or structurally-similar and functionally identical to a naturally-occurring substance and extracts from biological sources that control pests or provide other crop protection uses as defined below, but have non-toxic mode of actions (such as growth or developmental regulation, attractents, repellents or defence activators (e.g. induced resistance) and are relatively non-toxic to mammals.

Biopesticides for use against crop diseases have already established themselves on a variety of crops. E.g., biopesticides already play an important role in controlling downy mildew diseases. Their benefits include: a 0-Day Pre-Harvest Interval, the ability to use under moderate to severe disease pressure, and the ability to use in mixture or in a rotational program with other registered pesticides.

A major growth area for biopesticides is in the area of seed treatments and soil amendments. Biopesticidal seed treatments are e.g. used to control soil borne fungal pathogens that cause seed rots, damping-off, root rot and seedling blights. They can also be used to control internal seed borne fungal pathogens as well as fungal pathogens that are on the surface of the seed. Many biopesticidal products also show capacities to stimulate plant host defenses and other physiological processes that can make treated crops more resistant to a variety of biotic and abiotic stresses or can regulate plant growth. Many biopesticidal products also show capacities to stimulate plant health, plant growth and/or yield enhancing activity.

The following list of biopesticides, in conjunction with which the compounds of the invention can be used, is intended to illustrate the possible combinations but does not limit them:

### L) Biopesticides

L1) Microbial pesticides with fungicidal, bactericidal, viricidal and/or plant defense activator activity: *Ampelomyces quisqualis, Aspergillus flavus, Aureobasidium pullulans, Bacillus altitudinis, B. amyloliquefaciens, B. amyloliquefaciens* ssp. *plantarum* (also referred to as *B. velezensis*), *B. megaterium, B. mojavensis, B. mycoides, B. pumilus, B. simplex, B. solisalsi, B. subtilis, B. subtilis* var. *amyloliquefaciens, B. velezensis, Candida oleophila, C. saitoana, Clavibacter michiganensis* (bacteriophages), *Coniothyrium minitans, Cryphonectria parasitica, Cryptococcus albidus, Dilophosphora alopecuri, Fusarium oxysporum, Clonostachys rosea* f. *catenulate* (also named *Gliocladium catenulatum*), *Gliocladium roseum, Lysobacter antibioticus, L. enzymogenes, Metschnikowia fructicola, Microdochium dimerum, Microsphaeropsis ochracea, Muscodor albus, Paenibacillus alvei, Paenibacillus epiphyticus, P. polymyxa, Pantoea vagans, Penicillium bilaiae, Phlebiopsis gigantea, Pseudomonas* sp., *Pseudomonas chloraphis, Pseudozyma flocculosa, Pichia anomala, Pythium oligandrum, Sphaerodes mycoparasitica, Streptomyces griseoviridis, S. lydicus, S. violaceusniger, Talaromyces flavus, Trichoderma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia,* zucchini yellow mosaic virus (avirulent strain);
L2) Biochemical pesticides with fungicidal, bactericidal, viricidal and/or plant defense activator activity: harpin protein, *Reynoutria sachalinensis* extract;
L3) Microbial pesticides with insecticidal, acaricidal, molluscidal and/or nematicidal activity: *Agrobacterium radiobacter, Bacillus cereus, B. firmus, B. thuringiensis, B. thuringiensis* ssp. *aizawai, B. t.* ssp. *israelensis, B. t.* ssp. *galleriae, B. t.* ssp. *kurstaki, B. t.* ssp. *tenebrionis, Beauveria bassiana, B. brongniartii, Burkholderia* spp., *Chromobacterium subtsugae, Cydia pomonella* granulovirus (CpGV), *Cryptophlebia leucotreta* granulovirus (CrleGV), *Flavobacterium* spp., *Helicoverpa armigera* nucleopolyhedrovirus (HearNPV), *Helicoverpa zea* nucleopolyhedrovirus (HzNPV), *Helicoverpa zea* single capsid nucleopolyhedrovirus (HzSNPV), *Heterorhabditis bacteriophora, Isaria fumosorosea, Lecanicillium longisporum, L. muscarium, Metarhizium anisopliae, M. anisopliae* var. *anisopliae, M. anisopliae* var. *acridum, Nomuraea rileyi, Paecilomyces fumosoroseus, P. lilacinus, Paenibacillus popilliae, Pasteuria* spp., *P. nishizawae, P. penetrans, P. ramosa, P. thornea, P. usgae, Pseudomonas fluorescens, Spodoptera littoralis* nucleopolyhedrovirus (SpliNPV), *Steinernema carpocapsae, S. feltiae, S. kraussei, Streptomyces galbus, S. microtiavus*;
L4) Biochemical pesticides with insecticidal, acaricidal, molluscidal, pheromone and/or nematicidal activity: L-carvone, citral, (*E,Z*)-7,9-dodecadien-1-yl acetate, ethyl formate, (*E,Z*)-2,4-ethyl decadienoate (pear ester), (*Z,Z,E*)-7,11,13-hexadecatrienal, heptyl butyrate, isopropyl myristate, lavanulyl senecioate, cis-jasmone, 2-methyl 1-butanol, methyl eugenol, methyl jasmonate, (*E,Z*)-2,13-octadecadien-1-ol, (*E,Z*)-2,13-octadecadien-1-ol acetate, (*E,Z*)-3,13-octadecadien-1-ol, (*R*)-1-octen-3-ol, pentatermanone, (*E,Z,Z*)-3,8,11-tetradecatrienyl acetate, (*Z,E*)-9,12-tetra-decadien-1-yl acetate, (*Z*)-7-tetradecen-2-one, (*Z*)-9-tetradecen-1-yl acetate, (*Z*)-11-tetradecenal, (*Z*)-11-tetradecen-1-ol, extract of *Chenopodium ambrosiodes,* Neem oil, Quillay extract;
L5) Microbial pesticides with plant stress reducing, plant growth regulator, plant growth promoting and/or yield enhancing activity: *Azospirillum amazonense, A. brasilense, A. lipoferum, A. irakense, A. halopraeferens, Bradyrhizobium* spp., *B. elkanii, B. japonicum, B. liaoningense, B. lupini, Delftia acidovorans, Glomus intraradices, Mesorhizobium* spp., *Rhizobium leguminosarum* bv. *phaseoli, R. I*. bv. *trifolii, R. I*. bv. *viciae, R. tropici, Sinorhizobium meliloti.*

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematicidal activity.

Many of these biopesticides have been deposited under deposition numbers mentioned herein (the prefices such as ATCC or DSM refer to the acronym of the respective culture collection, for details see e. g. here: http://www. wfcc.info/ccinfo/collection/by_acronym/), are referred to in literature, registered and/or are commercially available: mixtures of *Aureobasidium pullulans* DSM 14940 and DSM 14941 isolated in 1989 in Konstanz, Germany (e. g. blastospores in BlossomProtect® from bio-ferm GmbH, Austria), *Azospirillum brasilense* Sp245 originally isolated in wheat reagion of South Brazil (Passo Fundo) at least prior to 1980 (BR 11005; e. g. GELFIX® Gramineas from BASF Agricultural Specialties Ltd., Brazil), *A. brasilense* strains Ab-V5 and Ab-V6 (e. g. in AzoMax from Novozymes BioAg Produtos papra Agricultura Ltda., Quattro Barras, Brazil or Simbiose-Maíz® from Simbiose-Agro, Brazil; Plant Soil 331, 413-425, 2010), *Bacillus amyloliquefaciens* strain AP-188 (NRRL B-50615 and B-50331; US 8,445,255); *B. amyloliquefaciens* ssp. *plantarum* strains formerly also sometimes referred to as *B. subtilis,* recently together with *B. methylotrophicus,* and *B. velezensis* classified as *B. velezensis* (Int. J. Syst. Evol. Microbiol. 66, 1212-1217, 2016): *B. a.* ssp. *plantarum* or *B. velezensis* D747 isolated from air in Kikugawa-shi, Japan (US 20130236522 A1; FERM BP-8234; e. g. Double Nickel™ 55 WDG from Certis LLC, USA), *B. a.* ssp. *plantarum* or *B. velezensis* FZB24 isolated from soil in Brandenburg, Germany (also called SB3615; DSM 96-2; J. Plant Dis. Prot. 105, 181-197, 1998; e. g. Taegro® from Novozyme Biologicals, Inc., USA), *B. a.* ssp. *plantarum* or *B. velezensis* FZB42 isolated from soil in Brandenburg, Germany (DSM 23117; J. Plant Dis. Prot. 105, 181-197, 1998; e. g. RhizoVital® 42 from AbiTEP GmbH, Germany), *B. a.* ssp. *plantarum* or *B. velezensis* MBI600 isolated from faba bean in Sutton Bonington, Nottinghamshire, U.K. at least before 1988 (also called 1430; NRRL B-50595; US 2012/0149571 A1; e. g. Integral® from BASF Corp., USA), *B. a.* ssp. *plantarum* or *B. velezensis* QST-713 isolated from peach orchard in 1995 in California, U.S.A. (NRRL B-21661; e. g. Serenade® MAX from Bayer Crop Science LP, USA), *B. a.* ssp. *plantarum* or *B. velezensis* TJ1000 isolated in 1992 in South Dakoda, U.S.A. (also called 1BE; ATCC BAA-390; CA 2471555 A1; e. g. QuickRoots™ from TJ Technologies, Watertown, SD, USA); *B. firmus* CNCM I-1582, a variant of parental strain EIP-N1 (CNCM I-1556) isolated from soil of central plain area of Israel (WO 2009/126473, US 6,406,690; e. g. Votivo® from Bayer CropScience LP, USA), *B. pumilus* GHA 180 isolated from apple tree rhizosphere in Mexico (IDAC 260707-01; e. g. PRO-MIX® BX from Premier Horticulture, Quebec, Canada), *B. pumilus* INR-7 otherwise referred to as BU-F22 and BU-F33 isolated at least before 1993 from cucumber infested by *Erwinia tracheiphila* (NRRL B-50185, NRRL B-50153; US 8,445,255), *B. pumilus* KFP9F isolated from the rhizosphere of grasses in South Africa at least before 2008 (NRRL B-50754; WO 2014/029697; e. g. BAC-UP or FUSION-P from BASF Agricultural Specialities (Pty) Ltd., South Africa), *B. pumilus* QST 2808 was isolated from soil collected in Pohnpei, Federated States of Micronesia, in 1998 (NRRL B-30087; e. g. Sonata® or Ballad® Plus from Bayer Crop Science LP, USA), *B. simplex* ABU 288 (NRRL B-50304; US 8,445,255), *B. subtilis* FB17 also called UD 1022 or UD10-22 isolated from red beet roots in North America (ATCC PTA-11857; System. Appl. Microbiol. 27, 372-379, 2004; US 2010/0260735; WO 2011/109395); *B. thuringiensis ssp. aizawai* ABTS-1857 isolated from soil taken from a lawn in Ephraim, Wisconsin, U.S.A., in 1987 (also called ABG-6346; ATCC SD-1372; e. g. XenTari® from BioFa AG, Münsingen, Germany), *B. t.* ssp. *kurstaki* ABTS-351 identical to HD-1 isolated in 1967 from diseased Pink Bollworm black larvae in Brownsville, Texas, U.S.A. (ATCC SD-1275; e. g. Dipel® DF from Valent BioSciences, IL, USA), *B. t.* ssp. *kurstaki* SB4 isolated from *E. saccharina* larval cadavers (NRRL B-50753; e. g. Beta Pro® from BASF Agricultural Specialities (Pty) Ltd., South Africa), *B. t.* ssp. *tenebrionis* NB-176-1, a mutant of strain NB-125, a wild type strain isolated in 1982 from a dead pupa of the beetle Tenebrio molitor (DSM 5480; EP 585 215 B1; e. g. Novodor® from Valent BioSciences, Switzerland), *Beauveria bassiana* GHA (ATCC 74250; e. g. BotaniGard® 22WGP from Laverlam Int. Corp., USA), *B. bassiana* JW-1 (ATCC 74040; e. g. Naturalis® from CBC (Europe) S.r.l., Italy), *B. bassiana* PPRI 5339 isolated from the larva of the tortoise beetle *Conchyloctenia punctata* (NRRL 50757; e. g. BroadBand® from BASF Agricultural Specialities (Pty) Ltd., South Africa), *Bradyrhizobium elkaniistrains* SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e. g. GELFIX 5 from BASF Agricultural Specialties Ltd., Brazil), *B. japonicum* 532c isolated from Wisconsin field in U.S.A. (Nitragin 61A152; Can. J. Plant. Sci. 70, 661-666, 1990; e. g. in Rhizoflo®, Histick®, Hicoat® Super from BASF Agricultural Specialties Ltd., Canada), *B. japonicum* E-109 variant of strain USDA 138 (INTA E109, SEMIA 5085; Eur. J. Soil Biol. 45, 28-35, 2009; Biol. Fertil. Soils 47, 81-89, 2011); *B. japonicum* strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e. g. GELFIX 5 or ADHERE 60 from BASF Agricultural Specialties Ltd., Brazil), *B*. *japonicum* SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e. g. GELFIX 5 or ADHERE 60 from BASF Agricultural Specialties Ltd., Brazil); *Burkholderia sp.* A396 isolated from soil in Nikko, Japan, in 2008 (NRRL B-50319; WO 2013/032693; Marrone Bio Innovations, Inc., USA), *Coniothyrium minitans* CON/M/91-08 isolated from oilseed rape (WO 1996/021358; DSM 9660; e. g. Contans® WG, Intercept® WG from Bayer CropScience AG, Germany), harpin (alpha-beta) protein (Science 257, 85-88, 1992; e. g. Messenger™ or HARP-N-Tek from Plant Health Care plc, U.K.), *Helicoverpa armigera* nucleopolyhedrovirus (HearNPV) (J. Invertebrate Pathol. 107, 112-126, 2011; e. g. Helicovex® from Adermatt Biocontrol, Switzerland; Diplomata® from Koppert, Brazil; Vivus® Max from AgBiTech Pty Ltd., Queensland, Australia), *Helicoverpa zea* single capsid nucleopolyhedrovirus (HzSNPV) (e. g. Gemstar® from Certis LLC, USA), *Helicoverpa zea* nucleopolyhedrovirus ABA-NPV-U (e. g. Heligen® from AgBiTech Pty Ltd., Queensland, Australia), *Heterorhabditis bacteriophora* (e. g. Nemasys® G from BASF Agricultural Specialities Limited, UK), *Isaria fumosorosea* Apopka-97 isolated from mealy bug on gynura in Apopka, Florida, U.S.A. (ATCC 20874; Biocontrol Science Technol. 22(7), 747-761, 2012; e. g. PFR-97™ or PreFeRal® from Certis LLC, USA), *Metarhizium anisopliae* var. *anisopliae* F52 also called 275 or V275 isolated from codling moth in Austria (DSM 3884, ATCC 90448; e. g. Met52® Novozymes Biologicals BioAg Group, Canada), *Metschnikowia fructicola* 277 isolated from grapes in the central part of Israel (US 6,994,849; NRRL Y-30752; e. g. formerly Shemer® from Agrogreen, Israel), *Paecilomyces ilacinus* 251 isolated from infected nematode eggs in the Philippines (AGAL 89/030550; WO1991/02051; Crop Protection 27, 352-361, 2008; e. g. BioAct®from Bayer CropScience AG, Germany and MeloCon® from Certis, USA), *Paenibacillus alvei* NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), *Paenibacillus* strains isolated from soil samples from a variety of European locations including Germany: *P. epiphyticus* Lu17015 (WO 2016/020371; DSM 26971), *P. polymyxa* ssp. *plantarum* Lu16774 (WO 2016/020371; DSM 26969), *P. p.* ssp. *plantarum* strain Lu17007 (WO 2016/020371; DSM 26970); *Pasteuria nishizawae* Pn1 isolated from a soybean field in the mid-2000s in Illinois, U.S.A. (ATCC SD-5833; Federal Register 76(22), 5808, February 2, 2011; e.g. Clariva™ PN from Syngenta Crop Protection, LLC, USA), *Penicillium bilaiae* (also called *P. bilaii*) strains ATCC 18309 (= ATCC 74319), ATCC 20851 and/or ATCC 22348 (= ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e. g. Jump Start®, Provide® from Novozymes Biologicals BioAg Group, Canada), *Reynoutria sachalinensis* extract (EP 0307510 B1; e. g. Regalia® SC from Marrone Biolnnovations, Davis, CA, USA or Milsana® from BioFa AG, Germany), *Steinernema carpocapsae* (e. g. Millenium® from BASF Agricultural Specialities Limited, UK), *S. feltiae* (e. g. Nemashield® from BioWorks, Inc., USA; Nemasys® from BASF Agricultural Specialities Limited, UK), *Streptomyces microfiavus* NRRL B-50550 (WO 2014/124369; Bayer CropScience, Germany), *Trichoderma asperelloides* JM41R isolated in South Africa (NRRL 50759; also referred to as *T. fertile;* e. g. Trichoplus® from BASF Agricultural Specialities (Pty) Ltd., South Africa), *T. harzianum* T-22 also called KRL-AG2 (ATCC 20847; BioControl 57, 687-696, 2012; e. g. Plantshield® from BioWorks Inc., USA or SabrEx™ from Advanced Biological Marketing Inc., Van Wert, OH, USA).

According to the invention, the solid material (dry matter) of the biopesticides (except for oils such as Neem oil) are considered as active components (e.g. to be obtained after drying or evaporation of the extraction or suspension medium in case of liquid formulations of the microbial pesticides).

In accordance with the invention, the weight ratios and percentages used herein for a biological extract such as Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calclulate the total weight of the respective active component with the following equation that 1 x 10¹⁰ CFU equals one gram of total weight of the respective active component. Colony forming unit is measure of viable microbial cells, in particular fungal and bacterial cells. In addition, here "CFU" may also be understood as the number of (juvenile) individual nematodes in case of (entomopathogenic) nematode biopesticides, such as *Steinernema feltiae.*

When mixtures comprising microbial pesticides are employed in crop protection, the application rates preferably range from about 1 x 10⁶ to 5 x 10¹⁵ (or more) CFU/ha, preferably from about 1 x 10⁸ to about 1 x 10¹³ CFU/ha, and even more preferably from about 1 x 10⁹ to about 1 x 10¹² CFU/ha. In the case of (entomopathogenic) nematodes as microbial pesticides (e. g. Steinernema feltiae), the application rates preferably range inform about 1 x 10⁵ to 1 x 10¹² (or more), more preferably from 1 x 10⁸ to 1 x 10¹¹, even more preferably from 5 x 10⁸ to 1 x 10¹⁰ individuals (e. g. in the form of eggs, juvenile or any other live stages, preferably in an infetive juvenile stage) per ha.

When mixtures comprising microbial pesticides are employed in seed treatment, the application rates with respect to plant propagation material preferably range from about 1 x 10⁶ to 1 x 10¹² (or more) CFU/seed. Preferably, the concentration is about 1 x 10⁶ to about 1 x 10⁹ CFU/seed. In the case of the microbial pesticides II, the application rates with respect to plant propagation material also preferably range from about 1 x 10⁷ to 1 x 10¹⁴ (or more) CFU per 100 kg of seed, preferably from 1 x 10⁹ to about 1 x 10¹² CFU per 100 kg of seed.

The invention also relates to agrochemical compositions comprising an auxiliary and at least one compound of the invention or a mixture thereof.

An agrochemical composition comprises a pesticidally effective amount of a compound of the invention or a mixture thereof. The term "pesticidally effective amount" is defined below.

The compounds of the invention or the mixtures thereof can be converted into customary types of agro-chemical compositions, e. g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Examples for suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharide powders, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sul-fates, phosphates, carboxylates, and mixtures thereof. Examples of sulfonates are alkylaryl-sulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethox-ylated alcohols, or of fatty acid esters. Examples of phosphates are phosphate esters. Examples of carboxylates are alkyl carboxylates, and carboxylated alcohol or alkylphenol eth-oxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpoly-glucosides. Examples of polymeric surfactants are homo- or copolymers of vinylpyrrolidone, vinylalcohols, or vinylacetate.

Suitable cationic surfactants are quaternary surfactants, e.g. quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compounds of the invention on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea, and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I according to the invention and 5-15 wt% wetting agent (e.g. alcohol alkoxylates) are dissolved in water and/or in a water-soluble solvent (e.g. alcohols) up to 100 wt%. The active substance dissolves upon dilution with water.
ii) Dispersible concentrates (DC)
   5-25 wt% of a compound I according to the invention and 1-10 wt% dispersant (e. g. polyvinylpyrrolidone) are dissolved in up to 100 wt% organic solvent (e.g. cyclohexanone). Dilution with water gives a dispersion.
iii) Emulsifiable concentrates (EC)
   15-70 wt% of a compound I according to the invention and 5-10 wt% emulsifiers (e.g. calcium dodecylbenzenesulfonate and castor oil ethoxylate) are dissolved in up to 100 wt% water-insoluble organic solvent (e.g. aromatic hydrocarbon). Dilution with water gives an emulsion.
iv) Emulsions (EW, EO, ES)
   5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into up to 100 wt% water by means of an emulsifying machine and made into a homogeneous emulsion. Dilution with water gives an emulsion.
v) Suspensions (SC, OD, FS)
   In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and up to 100 wt% water to give a fine active substance suspension. Dilution with water gives a stable suspension of the active substance. For FS type composition up to 40 wt% binder (e.g. polyvinylalcohol) is added.
vi) Water-dispersible granules and water-soluble granules (WG, SG)
   50-80 wt% of a compound I according to the invention are ground finely with addition of up to 100 wt% dispersants and wetting agents (e.g. sodium lignosulfonate and alcohol ethoxylate) and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound I according to the invention are ground in a rotor-stator mill with addition of 1-5 wt% dispersants (e.g. sodium lignosulfonate), 1-3 wt% wetting agents (e.g. alcohol ethoxylate) and up to 100 wt% solid carrier, e.g. silica gel. Dilution with water gives a stable dispersion or solution of the active substance.
viii) Gel (GW, GF)
   In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and up to 100 wt% water to give a fine suspension of the active substance. Dilution with water gives a stable suspension of the active substance.
ix) Microemulsion (ME)
   5-20 wt% of a compound I according to the invention are added to 5-30 wt% organic solvent blend (e.g. fatty acid dimethylamide and cyclohexanone), 10-25 wt% surfactant blend (e.g. alkohol ethoxylate and arylphenol ethoxylate), and water up to 100 %. This mixture is stirred for 1 h to produce spontaneously a thermodynamically stable microemulsion.
x) Microcapsules (CS)
   An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radi-cal initiator results in the formation of poly(meth)acrylate microcapsules. Alternatively, an oil phase comprising 5-50 wt% of a compound I according to the invention, 0-40 wt% water insolu-ble organic solvent (e.g. aromatic hydrocarbon), and an isocyanate monomer (e.g. diphenylme-thene-4,4'-di-isocyanatae) are dispersed into an aqueous solution of a protective colloid (e.g. polyvinyl alcohol). The addition of a polyamine (e.g. hexamethylenediamine) results in the for-mation of a polyurea microcapsule. The monomers amount to 1-10 wt%. The wt% relate to the total CS composition.
xi) Dustable powders (DP, DS)
   1-10 wt% of a compound I according to the invention are ground finely and mixed intimately with up to 100 wt% solid carrier, e.g. finely divided kaolin.
xii) Granules (GR, FG)
   0.5-30 wt% of a compound I according to the invention is ground finely and associated with up to 100 wt% solid carrier (e.g. silicate). Granulation is achieved by extrusion, spray-drying or the fluidized bed.
xiii) Ultra-low volume liquids (UL)
   1-50 wt% of a compound I according to the invention are dissolved in up to 100 wt% organic solvent, e.g. aromatic hydrocarbon.

The compositions types i) to xi) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and most preferably between 0.5 and 75%, by weight of active sub-stance. The active substances are employed in a purity of from 90% to 100%, preferably from 95% to 100% (according to NMR spectrum).

Various types of oils, wetters, adjuvants, fertilizer, or micronutrients, and other pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank and further auxiliaries may be added, if appropriate.

In a further embodiment, either individual components of the composition according to the invention or partially premixed components, e. g. components comprising compounds of the invention and/or mixing partners as defined above, may be mixed by the user in a spray tank and further auxiliaries and additives may be added, if appropriate.

In a further embodiment, either individual components of the composition according to the invention or partially premixed components, e. g. components comprising compounds of the invention and/or mixing partners as defined above, can be applied jointly (e.g. after tank mix) or consecutively.

The compounds of the invention are suitable for use in protecting crops, plants, plant propagation materials, such as seeds, or soil or water, in which the plants are growing, from attack or infestation by animal pests. Therefore, the invention also relates to a plant protection method, which comprises contacting crops, plants, plant propagation materials, such as seeds, or soil or water, in which the plants are growing, to be protected from attack or infestation by animal pests, with a pesticidally effective amount of a compound of the invention.

The compounds of the invention are also suitable for use in combating or controlling animal pests. Therefore, the invention also relates to a method of combating or controlling animal pests, which comprises contacting the animal pests, their habitat, breeding ground, or food supply, or the crops, plants, plant propagation materials, such as seeds, or soil, or the area, material or environment in which the animal pests are growing or may grow, with a pesticidally effective amount of a compound of the invention.

The compounds of the invention are effective through both contact and ingestion. Furthermore, the compounds of the invention can be applied to any and all developmental stages, such as egg, larva, pupa, and adult.

The compounds of the invention can be applied as such or in form of compositions comprising them as defined above. Furthermore, the compounds of the invention can be applied together with a mixing partner as defined above or in form of compositions comprising said mixtures as defined above. The components of said mixture can be applied simultaneously, jointly or separately, or in succession, that is immediately one after another and thereby creating the mixture "in situ" on the desired location, e.g. the plant, the sequence, in the case of separate application, generally not having any effect on the result of the control measures.

The application can be carried out both before and after the infestation of the crops, plants, plant propagation materials, such as seeds, soil, or the area, material or environment by the pests.

Suitable application methods include inter alia soil treatment, seed treatment, in furrow application, and foliar application. Soil treatment methods include drenching the soil, drip irrigation (drip application onto the soil), dipping roots, tubers or bulbs, or soil injection. Seed treatment techniques include seed dressing, seed coating, seed dusting, seed soaking, and seed pelleting. In furrow applications typically include the steps of making a furrow in cultivated land, seeding the furrow with seeds, applying the pesticidally active compound to the furrow, and closing the furrow. Foliar application refers to the application of the pesticidally active compound to plant foliage, e.g. through spray equipment. For foliar applications, it can be advantageous to modify the behavior of the pests by use of pheromones in combination with the compounds of the invention. Suitable pheromones for specific crops and pests are known to a skilled person and publicly available from databases of pheromones and semiochemicals, such as http://www.pherobase.com.

As used herein, the term "contacting" includes both direct contact (applying the compounds/compositions directly on the animal pest or plant - typically to the foliage, stem or roots of the plant) and indirect contact (applying the compounds/compositions to the locus, i.e. habitat, breeding ground, plant, seed, soil, area, material or environment in which a pest is growing or may grow, of the animal pest or plant).

The term "animal pest" includes arthropods, gastropods, and nematodes. Preferred animal pests according to the invention are arthropods, preferably insects and arachnids, in particular insects. Insects, which are of particular relevance for crops, are typically referred to as crop insect pests.

The term "crop" refers to both, growing and harvested crops.

The term "plant" includes cereals, e.g. durum and other wheat, rye, barley, triticale, oats, rice, or maize (fodder maize and sugar maize / sweet and field corn); beet, e.g. sugar beet or fodder beet; fruits, such as pomes, stone fruits or soft fruits, e.g. apples, pears, plums, peaches, nectarines, almonds, cherries, papayas, strawberries, raspberries, blackberries or gooseberries; leguminous plants, such as beans, lentils, peas, alfalfa or soybeans; oil plants, such as rapeseed (oilseed rape), turnip rape, mustard, olives, sunflowers, coconut, cocoa beans, castor oil plants, oil palms, ground nuts or soybeans; cucurbits, such as squashes, pumpkins, cucumber or melons; fiber plants, such as cotton, flax, hemp or jute; citrus fruit, such as oranges, lemons, grape-fruits or mandarins; vegetables, such as eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, such as avocados, cinnamon or camphor; energy and raw material plants, such as corn, soybean, rapeseed, sugar cane or oil palm; tobacco; nuts, e.g. walnuts; pistachios; coffee; tea; bananas; vines (table grapes and grape juice grape vines); hop; sweet leaf (also called Stevia); natural rubber plants or ornamental and forestry plants, such as flowers (e.g. carnation, petunias, geranium/pelargoniums, pansies and impatiens), shrubs, broad-leaved trees (e.g. poplar) or evergreens, e.g. conifers; eucalyptus; turf; lawn; grass such as grass for animal feed or ornamental uses. Preferred plants include potatoes sugar beets, tobacco, wheat, rye, barley, oats, rice, corn, cotton, soybeans, rapeseed, legumes, sunflowers, coffee or sugar cane; fruits; vines; ornamentals; or vegetables, such as cucumbers, tomatoes, beans or squashes.

The term "cultivated plants" is to be understood as including plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, in order to create mutations at a specific locus of a plant genome. Targeted mutagenesis techniques frequently use oligonucleotides or proteins like CRISPR/Cas, zinc-finger nucleases, TALENs or meganucleases to achieve the targeting effect.

Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant to add a trait or improve a trait. These integrated genes are also referred to as transgenes in the art, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to acetolactate synthase (ALS) inhibitor herbicides by conventional methods of mutagenesis and breeding comprise plant varieties commercially available under the name Clearfield®. However, most of the herbicide tolerance traits have been created via the use of transgenes.

Herbicide tolerance has been created to glyphosate, glufosinate, 2,4-D, dicamba, oxynil herbicides, like bromoxynil and ioxynil, sulfonylurea herbicides, ALS inhibitor herbicides and 4-hydroxyphenylpyruvate dioxygenase (HPPD) inhibitors, like isoxaflutole and mesotrione.

Transgenes wich have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gm-hra, S4-HrA, for tolerance to ALS inhibitor herbicides: csr1-2, for tolerance to HPPD inhibitor herbicides: hppdPF, W336 and avhppd-03.

Transgenic corn events comprising herbicide tolerance genes are e.g., but not excluding others, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are e.g., but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127.

Transgenic cotton events comprising herbicide tolerance genes are e.g., but not excluding others, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are e.g., but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal proteins to plants. Transgenes which have most frequently been used are toxin genes of *Bacillus spec.* and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinll. A further approach uses transgenes in order to produce double stranded RNA in plants to target and downregulate insect genes. An example for such a transgene is dvsnf7.

Transgenic corn events comprising genes for insecticidal proteins or double stranded RNA are e.g., but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are e.g., but not excluding others, MON87701, MON87751 and DAS-81419.

Transgenic cotton events comprising genes for insecticidal proteins are e.g., but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by increasing ear biomass using the transgene athb17, being present in corn event MON87403, or by enhancing photosynthesis using the transgene bbx32, being present in the soybean event MON87712.

Cultivated plants comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-ØØ41Ø-5.

Traits are frequently combined by combining genes in a transformation event or by combining different events during the breeding process. Preferred combination of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are well known in the art. E.g., detailed information as to the mutagenized or integrated genes and the respective events are available from websites of the organizations "International Service for the Acquisition of Agri-biotech Applications (ISAAA)" (http://www.isaaa.org/gmap-provaldatabase) and the "Center for Environmental Risk Assessment (CERA)" (http://cera-gmc.org/GMCropDatabase), Further information on specific events and methods to detect them can be found for canola events MS1, MS8, RF3, GT73, MON88302, KK179 in WO01/031042, WO01/041558, WO01/041558, WO02/036831, WO11/153186, WO13/003558, for cotton events MON1445, MON15985, MON531(MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527, for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571, for potato events E12, F10, J3, J55, V11, X17, Y9 in WO14/178910, WO14/178913, WO14/178941, WO14/179276, WO16/183445, WO17/062831, WO17/062825, for rice events LLRICE06, LLRICE601, LLRICE62 in WO00/026345, WO00/026356, WO00/026345 for soybean events H7-1, MON89788, A2704-12, A5547-127, DP305423, DP356043, MON87701, MON87769, CV127, MON87705, DAS68416-4, MON87708, MON87712, SYHT0H2, DAS81419, DAS81419 x DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compositions according to the invention on cultivated plants may result in effects which are specific to a cultivated plant comprising a certain gene or event. These effects might involve changes in growth behavior or changed resistance to biotic or abiotic stress factors. Such effects may in particular comprise enhanced yield, enhanced resistance or tolerance to insects, nematodes, fungal, bacterial, mycoplasma, viral or viroid pathogens as well as early vigour, early or delayed ripening, cold or heat tolerance as well as changed amino acid or fatty acid spectrum or content.

It has surprisingly been found that the pesticidal activity of the compounds of the invention may be enhanced by the insecticidal trait of a modified plant. Furthermore, it has been found that the compounds of the invention are suitable for preventing insects to become resistant to the insecticidal trait or for combating pests, which already have become resistant to the insecticidal trait of a modified plant. Moreover, the compounds of the invention are suitable for combating pests, against which the insecticidal trait is not effective, so that a complementary insecticidal activity can advantageously be used.

The term "plant propagation material" refers to all the generative parts of the plant such as seeds and vegetative plant material such as cuttings and tubers (e.g. potatoes), which can be used for the multiplication of the plant. This includes seeds, roots, fruits, tubers, bulbs, rhizomes, shoots, sprouts and other parts of plants. Seedlings and young plants, which are to be transplanted after germination or after emergence from soil, may also be included. These plant propagation materials may be treated prophylactically with a plant protection compound either at or before planting or transplanting.

The term "seed" embraces seeds and plant propagules of all kinds including but not limited to true seeds, seed pieces, suckers, corms, bulbs, fruit, tubers, grains, cuttings, cut shoots and the like, and means in a preferred embodiment true seeds.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application.

In the case of soil treatment, in furrow application or of application to the pests dwelling place or nest, the quantity of active ingredient ranges from 0.0001 to 500 g per 100 m², preferably from 0.001 to 20 g per 100 m².

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare, more desirably from 10 g to 50 g per hectare, e.g., 10 to 20 g per hectare, 20 to 30 g per hectare, 30 to 40 g per hectare, or 40 to 50 g per hectare.

The compounds of the invention are particularly suitable for use in the treatment of seeds in order to protect the seeds from insect pests, in particular from soil-living insect pests, and the resulting seedling's roots and shoots against soil pests and foliar insects. The invention therefore also relates to a method for the protection of seeds from insects, in particular from soil insects, and of the seedling's roots and shoots from insects, in particular from soil and foliar insects, said method comprising treating the seeds before sowing and/or after pregermination with a compound of the invention. The protection of the seedling's roots and shoots is preferred. More preferred is the protection of seedling's shoots from piercing and sucking insects, chewing insects and nematodes.

The term "seed treatment" comprises all suitable seed treatment techniques known in the art, such as seed dressing, seed coating, seed dusting, seed soaking, seed pelleting, and in-furrow application methods. Preferably, the seed treatment application of the active compound is carried out by spraying or by dusting the seeds before sowing of the plants and before emergence of the plants.

The invention also comprises seeds coated with or containing the active compound. The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

Suitable seed is e.g. seed of cereals, root crops, oil crops, vegetables, spices, ornamentals, e.g. seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants such as potatoes, sugar cane, tobacco, grapes, petunias, geranium/pelargoniums, pansies and impatiens.

In addition, the active compound may also be used for the treatment of seeds from plants, which have been modified by mutagenisis or genetic engineering, and which e.g. tolerate the action of herbicides or fungicides or insecticides. Such modified plants have been described in detail above.

Conventional seed treatment formulations include e.g. flowable concentrates FS, solutions LS, suspoemulsions (SE), powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter. Preferably, the formulations are applied such that germination is not included.

The active substance concentrations in ready-to-use formulations, which may be obtained after two-to-tenfold dilution, are preferably from 0.01 to 60% by weight, more preferably from 0.1 to 40 % by weight.

In a preferred embodiment a FS formulation is used for seed treatment. Typically, a FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, preferably water.

Especially preferred FS formulations of the compounds of the invention for seed treatment usually comprise from 0.1 to 80% by weight (1 to 800 g/l) of the active ingredient, from 0.1 to 20 % by weight (1 to 200 g/l) of at least one surfactant, e.g. 0.05 to 5 % by weight of a wetter and from 0.5 to 15 % by weight of a dispersing agent, up to 20 % by weight, e.g. from 5 to 20 % of an anti-freeze agent, from 0 to 15 % by weight, e.g. 1 to 15 % by weight of a pigment and/or a dye, from 0 to 40 % by weight, e.g. 1 to 40 % by weight of a binder (sticker /adhesion agent), optionally up to 5 % by weight, e.g. from 0.1 to 5 % by weight of a thickener, optionally from 0.1 to 2 % of an anti-foam agent, and optionally a preservative such as a biocide, antioxidant or the like, e.g. in an amount from 0.01 to 1 % by weight and a filler/vehicle up to 100 % by weight.

In the treatment of seed, the application rates of the compounds of the invention are generally from 0.1 g to 10 kg per 100 kg of seed, preferably from 1 g to 5 kg per 100 kg of seed, more preferably from 1 g to 1000 g per 100 kg of seed and in particular from 1 g to 200 g per 100 kg of seed, e.g. from 1 g to 100 g or from 5 g to 100 g per 100 kg of seed.

The invention therefore also relates to seed comprising a compound of the invention, or an agriculturally useful salt thereof, as defined herein. The amount of the compound of the invention or the agriculturally useful salt thereof will in general vary from 0.1 g to 10 kg per 100 kg of seed, preferably from 1 g to 5 kg per 100 kg of seed, in particular from 1 g to 1000 g per 100 kg of seed. For specific crops such as lettuce the rate can be higher.

The compounds of the invention may also be used for improving the health of a plant. Therefore, the invention also relates to a method for improving plant health by treating a plant, plant propagation material and/or the locus where the plant is growing or is to grow with an effective and non-phytotoxic amount of a compound of the invention.

As used herein "an effective and non-phytotoxic amount" means that the compound is used in a quantity which allows to obtain the desired effect but which does not give rise to any phytotoxic symptom on the treated plant or on the plant grown from the treated propagule or treated soil.

The terms "plant" and "plant propagation material" are defined above.

"Plant health" is defined as a condition of the plant and/or its products which is determined by several aspects alone or in combination with each other such as yield (e.g. increased biomass and/or increased content of valuable ingredients), quality (e.g. improved content or composition of certain ingredients or shelf life), plant vigour (e.g. improved plant growth and/or greener leaves ("greening effect"), tolerance to abiotic (e.g. drought) and/or biotic stress (e.g. disease) and production efficiency (e.g., harvesting efficiency, processability).

The above identified indicators for the health condition of a plant may be interdependent and may result from each other. Each indicator is defined in the art and can be determined by methods known to a skilled person.

The compounds of the invention are also suitable for use against non-crop insect pests. For use against said non-crop pests, compounds of the invention can be used as bait composition, gel, general insect spray, aerosol, as ultra-low volume application and bed net (impregnated or surface applied). Furthermore, drenching and rodding methods can be used.

As used herein, the term "non-crop insect pest" refers to pests, which are particularly relevant for non-crop targets, such as ants, termites, wasps, flies, ticks, mosquitoes, bed bugs, crickets, or cockroaches.

The bait can be a liquid, a solid or a semisolid preparation (e.g. a gel). The bait employed in the composition is a product, which is sufficiently attractive to incite insects such as ants, termites, wasps, flies, mosquitoes, crickets etc. or cockroaches to eat it. The attractiveness can be manipulated by using feeding stimulants or sex pheromones. Food stimulants are chosen, e.g., but not exclusively, from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are described in the literature (e.g. http://www.phero-base.com), and are known to those skilled in the art.

For use in bait compositions, the typical content of active ingredient is from 0.001 weight % to 15 weight %, desirably from 0.001 weight % to 5% weight % of active compound.

Formulations of the compounds of the invention as aerosols (e.g in spray cans), oil sprays or pump sprays are highly suitable for professional or non-professional users for controlling pests such as flies, fleas, ticks, bed bugs, mosquitoes or cockroaches. Aerosol recipes are preferably composed of the active compound, solvents, furthermore auxiliaries such as emulsifiers, perfume oils, if appropriate stabilizers, and, if required, propellants.

The oil spray formulations differ from the aerosol recipes in that no propellants are used.

For use in spray compositions, the content of active ingredient is from 0.001 to 80 weights %, preferably from 0.01 to 50 weight % and most preferably from 0.01 to 15 weight %.

The compounds of the invention and its respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniasis) with compounds of the invention and its respective compositions also comprise treating surfaces of huts and houses, air spraying and impregnation of curtains, tents, clothing items, bed nets, tsetse-fly trap or the like. Insecticidal compositions for application to fibers, fabric, knitgoods, nonwovens, netting material or foils and tarpaulins preferably comprise a mixture including the insecticide, optionally a repellent and at least one binder.

The compounds of the invention and its compositions can be used for protecting wooden materials such as trees, board fences, sleepers, frames, artistic artifacts, etc. and buildings, but also construction materials, furniture, leathers, fibers, vinyl articles, electric wires and cables etc. from ants, termites and/or wood or textile destroying beetles, and for controlling ants and termites from doing harm to crops or human beings (e.g. when the pests invade into houses and public facilities or nest in yards, orchards or parks).

Customary application rates in the protection of materials are, e.g., from 0.001 g to 2000 g or from 0.01 g to 1000 g of active compound per m² treated material, desirably from 0.1 g to 50 g per m².

Insecticidal compositions for use in the impregnation of materials typically contain from 0.001 to 95 weight %, preferably from 0.1 to 45 weight %, and more preferably from 1 to 25 weight % of at least one repellent and/or insecticide.

The compounds of the the invention are especially suitable for efficiently combating animal pests such as arthropods, gastropods and nematodes including but not limited to:
insects from the order of **Lepidoptera,** e.g. *Achroia grisella, Acleris* spp. such as *A. fimbriana, A. gloverana, A. variana; Acrolepiopsis assectella, Acronicta major, Adoxophyes* spp. such as *A. cyrtosema, A. orana; Aedia leucomelas, Agrotis* spp. such as *A. exclamationis, A. fucosa, A. ipsilon, A. orthogoma, A. segetum, A. subterranea; Alabama argillacea, Aleurodicus dispersus, Alsophila pometaria, Ampelophaga rubiginosa, Amyelois transitella, Anacampsis sarcitella, Anagasta kuehniella, Anarsia lineatella, Anisota senatoria, Antheraea pernyi, Anticarsia (=Thermesia)* spp. such as *A. gemmatalis; Apamea* spp., *Aproaerema modicella, Archips* spp. such as *A. argyrospila, A. fuscocupreanus, A. rosana, A. xyloseanus; Argyresthia conjugella, Argyroploce* spp., *Argyrotaenia* spp. such as *A. velutinana; Athetis mindara, Austroasca viridigrisea, Autographa gamma, Autographa nigrisigna, Barathra brassicae, Bedellia* spp., *Bonagota salubricola, Borbo cinnara, Bucculatrix thurberiella, Bupalus piniarius, Busseola* spp., *Cacoecia* spp. such as *C. murinana, C. podana; Cactoblastis cactorum, Cadra cautella, Calingo braziliensis, Caloptilis theivora, Capua reticulana, Carposina* spp. such as *C. niponensis, C. sasakii; Cephus* spp., *Chaetocnema aridula, Cheimatobia brumata, Chilo* spp. such as *C. Indicus, C. suppressalis, C. partellus; Choreutis pariana, Choristoneura* spp. such as *C. conflictana, C. fumiferana, C. longicellana, C. murinana, C. occidentalis, C. rosaceana; Chrysodeixis (=Pseudoplusia)* spp. *such as C. eriosoma, C. includens; Cirphis unipuncta, Clysia ambiguella, Cnaphalocerus* spp., *Cnaphalocrocis medinalis, Cnephasia* spp., *Cochylis hospes, Coleophora* spp., *Colias eurytheme, Conopomorpha* spp., *Conotrachelus* spp., *Copitarsia* spp., *Corcyra cephalonica, Crambus caliginosellus, Crambus teterrellus, Crocidosema (=Epinotia) aporema, Cydalima (=Diaphania) perspectalis, Cydia (=Carpocapsa)* spp. such as *C. pomonella, C. tatiferreana; Dalaca noctuides, Datana integerrima, Dasychira pinicola, Dendrolimus* spp. such as *D. pini, D. spectabilis, D. sibiricus; Desmia funeralis, Diaphania* spp. such as *D. nitidalis, D. hyalinata; Diatraea grandiosella, Diatraea saccharalis, Diphthera festiva, Earias* spp. such as *E. insulana, E. vittella; Ecdytolopha aurantianu, Egira (=Xylomyges) curialis, Elasmopalpus lignosellus, Eldana saccharina, Endopiza viteana, Ennomos subsignaria, Eoreuma Ioftini, Ephestia* spp. such as *E. cautella, E. elutella, E. kuehniella; Epinotia aporema, Epiphyas postvittana, Erannis tiliaria, Erionota thrax, Etiella* spp., *Eulia* spp., *Eupoecilia ambiguella, Euproctis chrysorrhoea, Euxoa* spp., *Evetria bouliana, Faronta albilinea, Feltia* spp. such as *F. subterranean; Galleria mellonella, Gracillaria* spp., *Grapholita* spp. such as *G. funebrana, G. molesta, G. inopinata; Halysidota* spp., *Harrisina americana, Hedylepta* spp., *Helicoverpa* spp. such as *H. armigera (=Heliothis armigera), H. zea (=Heliothis zea); Heliothis* spp. such as *H. assulta, H. subflexa, H. virescens; Hellula* spp. such as *H. undalis, H. rogatalis; Helocoverpa gelotopoeon, Hemileuca oliviae, Herpetogramma licarsisalis, Hibernia defoliaria, Hofmannophila pseudospretella, Homoeosoma electellum, Homona magnanima, Hypena scabra, Hyphantria cunea, Hyponomeuta padella, Hyponomeuta malinellus, Kakivoria flavofasciata, Keiferia lycopersicella, Lambdina fiscellaria fiscellaria, Lambdina fiscellaria lugubrosa, Lamprosema indicata, Laspeyresia molesta, Leguminivora glycinivorella, Lerodea eufala, Leucinodes orbonalis, Leucoma salicis, Leucoptera* spp. such as *L. coffeella, L. scitella; Leuminivora lycinivorella, Lithocolletis blancardella, Lithophane antennata, Llattia octo (=Amyna axis), Lobesia botrana, Lophocampa* spp., *Loxagrotis albicosta, Loxostege* spp. such as *L. sticticalis, L. cereralis; Lymantria* spp. such as *L. dispar, L. monacha; Lyonetia clerkella, Lyonetia prunifoliella, Malacosoma* spp. such as *M. americanum, M. californicum, M. constrictum, M. neustria; Mamestra* spp. such as *M. brassicae, M. configurata; Mamstra brassicae, Manduca* spp. such as *M. quinquemaculata, M. sexta; Marasmia spp, Marmara* spp., *Maruca testulalis, Megalopyge lanata, Melanchra picta, Melanitis leda, Mods* spp. such as *M. lapites, M. repanda; Mods latipes, Monochroa fragariae, Mythimna separata, Nemapogon cloacella, Neoleucinodes elegantalis, Nepytia* spp., *Nymphula* spp., *Oiketicus* spp., *Omiodes indicata, Omphisa anastomosalis, Operophtera brumata, Orgyia pseudotsugata, Oria* spp., *Orthaga thyrisalis, Ostrinia* spp. such as *O. nubilalis; Oulema oryzae, Paleacrita vernata, Panolis flammea, Parnara* spp., *Papaipema nebris, Papilio cresphontes, Paramyelois transitella, Paranthrene regalis, Paysandisia archon, Pectinophora* spp. such as *P. gossypiella; Peridroma saucia, Perileucoptera* spp., such as *P. coffeella; Phalera bucephala, Phryganidia californica, Phthorimaea* spp. such as *P. operculella; Phyllocnistis citrella, Phyllonoryder* spp*.* such as *P. blancardella, P. crataegella, P. issikii, P. ringoniella; Pieris* spp. such as *P. brassicae, P. rapae, P. napi; Pilocrocis tripunctata, Plathypena scabra, Platynota* spp. such as *P. flavedana, P. idaeusalis, P. stuttana; Platyptilia carduidactyla, Plebejus argus, Plodia interpunctella, Plusia spp, Plutella maculipennis, Plutella xylostella, Pontia protodica, Prays* spp., *Prodenia* spp., *Proxenus lepigone, Pseudaletia* spp. such as *P. sequax, P. unipuncta; Pyrausta nubilalis, Rachiplusia nu, Richia albicosta, Rhizobius ventralis, Rhyacionia frustrana, Sabulodes aegrotata, Schizura concinna, Schoenobius* spp., *Schreckensteinia festaliella, Scirpophaga* spp. such as *S. incertulas, S. innotata; Scotia segetum, Sesamia* spp. such as *S. inferens, Seudyra subflava, Sitotroga cerealella, Sparganothis pilleriana, Spilonota lechriaspis, S. ocellana, Spodoptera (=Lamphygma)* spp. such as *S. cosmoides, S. eridania, S. exigua, S. frugiperda, S. latisfascia, S. littoralis, S. litura, S. omithogalli; Stigmella* spp., *Stomopteryx subsecivella, Strymon bazochii, Sylepta derogata, Synanthedon* spp. such as S. exitiosa, *Tecia solanivora, Telehin licus, Thaumatopoea pityocampa, Thaumatotibia (=Cryptophlebia) leucotreta, Thaumetopoea pityocampa, Thecla* spp., *Theresimima ampelophaga, Thyrinteina* spp, *Tildenia inconspicuella, Tinea* spp. such as *T. cloacella, T. pellionella; Tineola bisselliella, Tortrix* spp*.* such as *T. viridana; Trichophaga tapetzella, Trichoplusia* spp. such as *T. ni; Tuta (=Scrobipalpula) absoluta, Udea* spp. such as *U. rubigalis, U. rubigalis; Virachola* spp., *Yponomeuta padella, and Zeiraphera canadensis;* insects from the order of **Coleoptera,** e.g. *Acalymma vittatum, Acanthoscehdes obtectus, Adoretus* spp., *Agelastica alni, Agrilus* spp. such as *A. anxius, A. planipennis, A. sinuatus; Agriotes* spp. such as *A. fuscicollis, A. lineatus, A. obscurus; Alphitobius diaperinus, Amphimallus solstitialis, Anisandrus dispar, Anisoplia austriaca, Anobium punctatum, Anomala corpulenta, Anomala rufocuprea, Anoplophora* spp. such as *A. giabripennis; Anthonomus* spp. such as *A. eugenii, A. grandis, A. pomorum; Anthrenus* spp., *Aphthona euphoridae, Apion* spp., *Apogonia* spp., *Athous haemorrhoidalis, Atomaria* spp. such as *A. linearis; Attagenus* spp., *Aulacophora femoralis, Blastophagus piniperda, Blitophaga undata, Bruchidius obtectus, Bruchus* spp. such as *B. lentis, B. pisorum, B. rufimanus; Byctiscus betulae, Callidiellum rufipenne, Callopistria floridensis, Callosobruchus chinensis, Cameraria ohridella, Cassida nebulosa, Cerotoma trifurcata, Cetonia aurata, Ceuthorhynchus* spp. such as *C. assimilis, C. napi; Chaetocnema tibialis, Cleonus mendicus, Conoderus* spp. such as *C*. *vespertinus; Conotrachelus nenuphar, Cosmopolites* spp., *Costelytra zealandica, Crioceris asparagi, Cryptolestes ferrugineus, Cryptorhynchus lapathi, Ctenicera* spp. such as *C. destructor; Curculio* spp., *Cylindrocopturus* spp., *Cyclocephala* spp., *Dactylispa balyi, Dectes texanus, Dermestes* spp., *Diabrotica* spp. such as *D. undecimpunctata, D. speciosa, D. longicornis, D. semipunctata, D. virgifera; Diaprepes abbreviates, Dichocrocis* spp., *Dicladispa armigera, Diloboderus abderus, Diocalandra frumenti (Diocalandra stigmaticollis), Enaphalodes rufulus, Epilachna* spp. such as *E. varivestis, E. vigintioctomaculata; Epitrix* spp*.* such as *E. hirtipennis, E. similaris; Eutheola humilis, Eutinobothrus brasiliensis, Faustinus cubae, Gibbium psylloides, Gnathocerus cornutus, Hellula undalis, Heteronychus arator, Hylamorpha elegans, Hylobius abietis, Hylotrupes bajulus, Hypera* spp. such as *H. brunneipennis, H. postica; Hypomeces squamosus, Hypothenemus* spp., *lps typographus, Lachnosterna consanguinea, Lasioderma serricorne, Latheticus oryzae, Lathridius* spp., *Lema* spp*.* such as *L. bilineata, L. melanopus; Leptinotarsa* spp. such as *L. decemtineata; Leptispa pygmaea, Limonius californicus, Lissorhoptrus oryzophilus, Lixus* spp., *Luperodes* spp., *Lyctus* spp. such as *L. bruneus; Liogenys fuscus, Macrodactylus* spp. such as *M. subspinosus; Maladera matrida, Megaplatypus mutates, Megascelis* spp., *Melanotus communis, Meligethes* spp. such as *M. aeneus; Melolontha* spp. such as *M. hippocastani, M. melolontha; Metamasius hemipterus, Microtheca* spp., *Migdolus* spp. such as *M. fryanus, Monochamus* spp. such as *M. alternatus; Naupactus xanthographus, Niptus hololeucus, Oberia brevis, Oemona hirta, Oryctes rhinoceros, Oryzaephilus surinamensis, Oryzaphagus oryzae, Otiorrhynchus sulcatus, Otiorrhynchus ovatus, Otiorrhynchus sulcatus, Oulema melanopus, Oulema oryzae, Oxycetonia jucunda, Phaedon* spp*.* such as *P. brassicae, P. cochieariae; Phoracantha recurva, Phyllobius pyri, Phyllopertha horticola, Phyllophaga* spp. such as *P. helleri; Phyllotreta* spp. such as *P. chrysocephala, P. nemorum, P. striolata, P. vittula; Phyllopertha horticola, Popillia japonica, Premnotrypes* spp., *Psacothea hilaris, Psylliodes chrysocephala, Prostephanus truncates, Psylliodes* spp., *Ptinus* spp., *Pulga saltona, Rhizopertha dominica, Rhynchophorus* spp. such as *R. billineatus, R. ferrugineus, R. palmarum, R. phoenicis, R. vuineratus; Saperda candida, Scolytus schevyrewi, Scyphophorus acupunctatus, Sitona lineatus, Sitophilus* spp. such as *S. granaria, S. oryzae, S. zeamais; Sphenophorus* spp. such as *S. levis; Stegobium paniceum, Sternechus* spp. such as *S. subsignatus; Strophomorphus ctenotus, Symphyletes* spp., *Tanymecus* spp., *Tenebrio molitor, Tenebrioides mauretanicus, Tribolium* spp. such as *T. castaneum; Trogoderma* spp., *Tychius* spp., *Xylotrechus* spp. such as *X. pyrrhoderus; and, Zabrus* spp. *such as Z. tenebrioides;*
insects from the order of Diptera e.g. *Aedes* spp. such as *A. aegypti, A. albopictus, A. vexans; Anastrepha ludens, Anopheles* spp. such as *A. albimanus, A. crucians, A. freeborni, A. gambiae, A. leucosphyrus, A. maculipennis, A. minimus, A. quadrimaculatus, A. sinensis; Bactrocera in vadens, Bibio hortulanus, Calliphora erythrocephala, Calliphora vicina, Ceratitis capitata, Chrysomyia* spp. such as *C. bezziana, C. hominivorax, C. macellaria; Chrysops atlanticus, Chrysops discalis, Chrysops silacea, Cochliomyia* spp. such as *C. hominivorax; Contarinia* spp. such as *C. sorghicoia; Cordylobia anthropophaga, Culex* spp*.* such as *C. nigripalpus, C. pipiens, C. quinquefasciatus, C. tarsalis, C. tritaeniorhynchus; Culicoides furens, Culiseta inornata, Culiseta melanura, Cuterebra* spp., *Dacus cucurbitae, Dacus oleae, Dasineura brassicae, Dasineura oxycoccana, Delia* spp. such as *D. antique, D. coarctata, D. platura, D. radicum; Dermatobia hominis, Drosophila* spp. such as *D. suzukii, Fannia* spp. such as *F. canicularis; Gastraphilus* spp*.* such as *G. intestinalis; Geomyza tipunctata, Glossina* spp. such as *G. fuscipes, G. morsitans, G. palpalis, G. tachinoides; Haematobia irritans, Haplodiplosis equestris, Hippelates* spp., Hylemyia spp. such as *H. platura; Hypoderma* spp. such as *H. lineata; Hyppobosca* spp., *Hydrellia philippina, Leptoconops torrens, Liriomyza* spp. such as *L. sativae, L. trifolii; Lucilia* spp. such as *L. caprina, L. cuprina, L. sericata; Lycoria pectoralis, Mansonia titillanus, Mayetiola* spp. such as *M. destructor; Musca* spp. such as *M. autumnalis, M. domestica; Muscina stabulans, Oestrus* spp. such as *O. ovis; Opomyza florum, Oscinella* spp. such as *O. frit; Orseolia oryzae, Pegomya hysocyami, Phlebotomus argentipes, Phorbia* spp. such as *P. antiqua, P. brassicae, P. coarctata; Phytomyza gymnostoma, Prosimulium mixtum, Psila rosae, Psorophora columbiae, Psorophora discolor, Rhagoletis* spp. such as *R. cerasi, R. cingulate, R. indifferens, R. mendax, R. pomonella; Rivellia quadrifasciata, Sarcophaga* spp. such as *S. haemorrhoidalis; Simulium vittatum, Sitodiplosis mosellana, Stomoxys* spp. such as *S. calcitrans; Tabanus* spp. such as *T. atratus, T. bovinus, T. lineola, T. similis; Tannia spp., Thecodiplosis japonensis, Tipula oleracea, Tipula paludosa,* and *Wohlfahrtia* spp;
insects from the order of **Thysanoptera** e.g., *Baliothrips biformis, Dichromothrips corbetti, Dichromothrips* ssp., *Echinothrips americanus, Enneothrips flavens, Frankliniella* spp. such as *F. fusca, F. occidentalis, F. tritici; Heliothrips* spp., *Hercinothrips femoralis, Kakothrips* spp., *Microcephalothrips abdominalis, Neohydatothrips samayunkur, Pezothrips kellyanus, Rhipiphorothrips cruentatus, Scirtothrips* spp. such as *S. citri, S. dorsalis, S. perseae; Stenchaetothrips spp, Taeniothrips cardamoni, Taeniothrips inconsequens, Thrips* spp. such as *T. imagines, T. hawaiiensis, T. oryzae, T. palmi, T. parvispinus, T. tabaci;*
insects from the order of **Hemiptera** e.g., *Acizzia jamatonica, Acrosternum* spp. such as *A. hilare;* Acyrthosipon spp. such as *A. onobrychis, A. pisum; Adelges laricis, Adelges tsugae, Adelphocoris* spp., such as *A. rapidus, A. superbus; Aeneolamia* spp., *Agonoscena* spp., *Aulacorthum solani, Aleurocanthus woglumi, Aleurodes* spp., *Aleurodicus disperses, Aleurolobus barodensis, Aleurothrixus* spp., *Amrasca* spp., *Anasa tristis, Antestiopsis* spp., *Anuraphis cardui, Aonidiella* spp., *Aphanostigma piri, Aphidula nasturtii, Aphis* spp. such as *A. craccivora, A. fabae, A. forbesi, A. gossypii, A. grossulariae, A. maidiradicis, A. pomi, A. sambuci, A. schneideri, A. spiraecola; Arboridia apicalis, Arilus critatus, Aspidiella* spp., *Aspidiotus* spp., *Atanus* spp., *Aulacaspis yasumatsui, Aulacorthum solani, Bactericera cockerelli (Paratrioza cockerelli), Bemisia* spp. such as *B. argentifolii, B. tabaci (Aleurodes tabaci); Blissus* spp. such as *B. leucopterus; Brachycaudus* spp. such as *B. cardui, B. helichrysi, B. persicae, B. prunicola; Brachycolus* spp., *Brachycorynella asparagi, Brevicoryne brassicae, Cacopsylla* spp. such as *C. fulguralis, C. pyricola (Psylla piri); Calligypona marginata, Calocoris* spp., *Campylomma livida, Capitophorus horni, Carneocephala fulgida, Cavelerius* spp., *Ceraplastes* spp., *Ceratovacuna lanigera, Ceroplastes ceriferus, Cerosipha gossypii, Chaetosiphon fragaefolii, Chionaspis tegalensis, Chlorita onukii, Chromaphisjuglandicola, Chrysomphalus ficus, Cicadulina mbila, Cimex* spp. such as *C. hemipterus, C. lectularius; Coccomytilus halli, Coccus* spp. such as *C. hesperidum, C. pseudomagnoliarum; Corythucha arcuata, Creontiades dilutus, Cryptomyzus ribis, Chrysomphalus aonidum, Cryptomyzus ribis, Ctenarytaina spatulata, Cyrtopeltis notatus, Dalbulus* spp., *Dasynus piperis, Dialeurodes* spp. such as *D. citrifolii; Dalbulus maidis, Diaphorina* spp. such as *D. citri; Diaspis* spp. such as *D. bromeliae; Dichelops furcatus, Diconocoris hewetti, Doralis* spp., *Dreyfusia nordmannianae, Dreyfusia piceae, Drosicha* spp., *Dysaphis* spp. such as *D. plantaginea, D. pyri, D. radicola; Dysaulacorthum pseudosolani, Dysdercus* spp. such as *D. cingulatus, D. intermedius; Dysmicoccus* spp., *Edessa* spp., *Geocoris* spp., *Empoasca* spp. such as *E. fabae, E. solana; Epidiaspis leperii, Eriosoma* spp. such as *E. lanigerum, E. pyricola; Erythroneura* spp., *Eurygasterspp.* such as *E. integriceps; Euscelis bilobatus, Euschistus* spp. such as *E. heros, E. impictiventris, E. servus; Fiorinia theae, Geococcus coffeae, Glycaspis brimblecombei, Halyomorpha* spp. such as *H. halys; Heliopeltis* spp., *Homalodisca vitripennis (=H. coagulata), Horcias nobilellus, Hyalopterus pruni, Hyperomyzus lactucae, Icerya* spp. such as *I. purchase; Idiocerus* spp., *Idioscopus* spp., *Laodelphax striatellus, Lecanium* spp., *Lecanoideus floccissimus, Lepidosaphes* spp. such as *L. ulmi; Leptocorisa* spp., *Leptoglossus phyllopus, Lipaphis erysimi, Lygus* spp. such as *L. hesperus, L. lineolaris, L. pratensis; Maconellicoccus hirsutus, Marchalina hellenica, Macropes excavatus, Macrosiphum* spp. such as *M. rosae, M. avenae, M. euphorbiae; Macrosteles quadrilineatus, Mahanarva fimbriolata, Megacopta cribraria, Megoura viciae, Melanaphis pyrarius, Melanaphis sacchari, Melanocallis (=Tinocallis) caryaefoliae, Metcafiella* spp., *Metopolophium dirhodum, Monellia costalis, Monelliopsis pecanis, Myzocallis coryli, Murgantia* spp., *Myzus* spp. such as *M. ascalonicus, M. cerasi, M. nicotianae, M. persicae, M. varians; Nasonovia ribis-nigri, Neotoxoptera formosana, Neomegalotomus spp, Nephotettix* spp. such as *N. malayanus, N. nigropictus, N. parvus, N. virescens; Nezara* spp. such as *N. viridula; Nilaparvata lugens, Nysius huttoni, Oebalus* spp. such as *O. pugnax; Oncometopia* spp., *Orthezia praelonga, Oxycaraenus hyalinipennis, Parabemisia myricae, Parlatoria* spp., *Parthenolecanium* spp. such as *P. corni, P. persicae; Pemphigus* spp. such as *P. bursarius, P. populivenae; Peregrinus maidis, Perkinsiella saccharicida, Phenacoccus* spp. such as *P. aceris, P. gossypii; Phloeomyzus passerinii, Phorodon humuli, Phylloxera* spp. such as *P. devastatrix, Piesma quadrata, Piezodorus* spp. such as *P. guildinii; Pinnaspis aspidistrae, Planococcus* spp. such as *P. citri, P. ficus; Prosapia bicincta, Protopulvinaria pyriformis, Psallus seriatus, Pseudacysta persea, Pseudaulacaspis pentagona, Pseudococcus* spp. such as *P. comstocki; Psylla* spp. such as *P. mali; Pteromalus* spp., *Pulvinaria amygdali, Pyrilla* spp., *Quadraspidiotus* spp., such as *Q. perniciosus; Quesada gigas, Rastrococcus* spp., *Reduvius senilis, Rhizoecus americanus, Rhodnius* spp., *Rhopalomyzus ascalonicus, Rhopalosiphum* spp. such as *R. pseudobrassicas, R. insertum, R. maidis, R. padi; Sagatodes* spp., *Sahlbergella singularis, Saissetia* spp., *Sappaphis mala, Sappaphis mali, Scaptocoris* spp., *Scaphoides titanus, Schizaphis graminum, Schizoneura lanuginosa, Scotinophora* spp., *Selenaspidus articulatus, Sitobion avenae, Sogata* spp., *Sogatella furcifera, Solubea insularis, Spissistilus festinus (=Stictocephala festina), Stephanitis nashi, Stephanitis pyrioides, Stephanitis takeyai, Tenalaphara malayensis, Tetraleurodes perseae, Therioaphis maculate, Thyanta* spp. such as *T. accerra, T. perditor; Tibraca* spp., *Tomaspis* spp., *Toxoptera* spp. such as *T. aurantii; Trialeurodes* spp. such as *T. abutilonea, T. ricini, T. vaporariorum; Triatoma* spp., *Trioza* spp., *Typhlocyba* spp., *Unaspis* spp. such as *U. citri, U. yanonensis; and Viteus vitifolii,*
Insects from the order **Hymenoptera** e.g. *Acanthomyops interjectus, Athalia rosae, Atta* spp. *such as A. capiguara, A. cephalotes, A. cephalotes, A. laevigata, A. robusta, A. sexdens, A. texana, Bombus* spp., *Brachymyrmex* spp., *Camponotus* spp. such as *C. floridanus, C. pennsyl-vanicus, C. modoc; Cardiocondyla nuda, Chalibion sp, Crematogaster* spp., *Dasymutilla occidentalis, Diprion* spp., *Dolichovespula maculata, Dorymyrmex* spp., *Dryocosmus kuriphilus, Formica* spp., *Hoplocampa* spp. such as *H. minuta, H. testudinea; Iridomyrmex humilis,* Lasius spp. such as *L. niger, Linepithema humile, Liometopum* spp., *Leptocybe invasa, Monomorium* spp. such as *M. pharaonis, Monomorium, Nylandria fulva, Pachycondyla chinensis, Paratrechina longicornis, Paravespula* spp., such as *P. germanica, P. pennsylvanica, P. vulgaris; Pheidole* spp. such as *P. megacephala; Pogonomyrmex* spp. such as *P. barbatus, P. californicus, Polistes rubiginosa, Prenolepis impairs, Pseudomyrmex gracilis, Schelipron* spp., *Sirex cyaneus, Solenopsis* spp. such as *S. geminata, S.invicta, S. molesta, S. richteri, S. xyloni, Sphecius speciosus, Sphex* spp., *Tapinoma* spp. such as *T. melanocephalum, T. sessile; Tetramorium* spp. such as *T. caespitum, T. bicarinatum, Vespa* spp. such as *V. crabro; Vespula* spp. such as *V. squamosal; Wasmannia auropunctata, Xylocopa* sp;
Insects from the order **Orthoptera** e.g. *Acheta domesticus, Calliptamus italicus, Chortoicetes terminifera, Ceuthophilus* spp., *Diastrammena asynamora, Dociostaurus maroccanus, Gryllotalpa* spp. such as *G. africana, G. gryllotalpa; Gryllus* spp., *Hieroglyphus daganensis, Kraussaria angulifera, Locusta* spp. such as *L. migratoria, L. pardalina; Melanoplus* spp. such as *M. bivittatus, M. femurrubrum, M. mexicanus, M. sanguinipes, M. spretus; Nomadacris septemfasciata, Oedaleus senegalensis, Scapteriscus* spp., *Schistocerca* spp. such as *S. americana, S. gregaria, Stemopelmatus* spp., *Tachycines asynamorus,* and *Zonozerus variegatus;*
Pests from the Class **Arachnida** e.g. **Acari**,e.g. of the families Argasidae, Ixodidae and Sarcoptidae, such as *Amblyomma* spp. (e.g. *A. americanum, A. variegatum, A. maculatum*), Argas spp. such as *A. persicu), Boophilus* spp. such as *B. annulatus, B. decoloratus, B. microplus, Dermacentorspp.* such as *D.silvarum, D. andersoni, D. variabilis, Hyalomma* spp. such as *H. truncatum, Ixodes* spp. such as *I. ricinus, I. rubicundus, I. scapularis, I. holocyclus, I. pacificus, Rhipicephalus sanguineus, Ornithodorus* spp. such as *O. moubata, O. hermsi, O. turicata, Ornithonyssus bacoti, Otobius megnini, Dermanyssus gallinae, Psoroptes* spp. such as *P. ovis, Rhipicephalus* spp. such as *R. sanguineus, R. appendiculatus, Rhipicephalus evertsi, Rhizoglyphus* spp., *Sarcoptes* spp. such as*S*. *Scabiei*; and Family **Eriophyidae** including *Aceria* spp. such as *A. sheldoni, A. anthocoptes, Acallitus* spp., *Aculops* spp. such as *A. lycopersici, A. pelekassi*; *Aculus* spp. such as *A. schlechtendali; Colomerus vitis, Epitrimerus pyri, Phyllocoptruta oleivora; Eriophytes ribis* and *Eriophyes* spp. such as *Eriophyes sheldoni*; Family Tarsonemidae including *Hemitarsonemus* spp., *Phytonemus pallidus and Polyphagotarsonemus latus, Stenotarsonemus* spp. *Steneotarsonemus spinki*; Family **Tenuipalpidae** including Brevipalpus spp. such as *B. phoenicis*; Family **Tetranychidae** including *Eotetranychus* spp., *Eutetranychus* spp., *Oligonychus* spp., *Petrobia latens, Tetranychus* spp. such as *T. cinnabarinus, T. evansi, T. kanzawai, T, pacificus, T. phaseulus, T. telarius* and *T. urticae*; *Bryobia praetiosa*; *Panonychus* spp. such as *P. ulmi, P. citri*; *Metatetranychus* spp. and *Oligonychus* spp. such as *O. pratensis, O. perseae, Vasates lycopersici*; *Raoiella indica, Familly* **Carpoglyphidae** including *Carpoglyphus* spp.*; Penthaleidae* spp. such as *Halotydeus destructor,* Family **Demodicidae** with species such as *Demodexspp.;* Family **Trombicidea** including *Trombicula* spp.; Family **Macronyssidae** including *Ornothonyssus* spp.; Family **Pyemotidae** including *Pyemotes tritici*; *Tyrophagus putrescentiae*; Family **Acaridae** including *Acarus siro*; Family **Araneida** including *Latrodectus mactans, Tegenaria agrestis, Chiracanthium sp, Lycosa sp Achaearanea tepidariorum* and *Loxosceles reclusa*;
Pests from the Phylum **Nematoda**, e.g., plant parasitic nematodes such as root-knot nematodes, *Meloidogyne* spp. such as *M. hapla, M. incognita, M. javanica;* cyst-forming nematodes, *Globodera* spp. such as *G. rostochiensis; Heterodera* spp. such as *H. avenae, H. glycines, H. schachtii, H. trifoiii;* Seed gall nematodes, *Anguina* spp.; Stem and foliar nematodes, *Aphelenchoides* spp. such as *A. besseyi;* Sting nematodes, *Belonolaimus* spp. such as *B. longicaudatus;* Pine nematodes, *Bursaphelenchus* spp. such as *B. lignicolus, B. xylophilus;* Ring nematodes, *Criconema* spp., *Criconemella* spp. such as *C. xenoplax* and *C. ornata;* and, *Criconemoides* spp. such as *Criconemoides informis; Mesocriconema* spp.*;* Stem and bulb nematodes, *Ditylenchus* spp. such as *D. destructor, D. dipsaci;* Awl nematodes, *Dolichodorus* spp.*;* Spiral nematodes, *Heliocotylenchus muiticinctus;* Sheath and sheathoid nematodes, *Hemicycliophora* spp. and *Hemicriconemoides* spp.*; Hirshmanniella* spp.*;* Lance nematodes, *Hoploaimus* spp.*;* False rootknot nematodes, *Nacobbus* spp.*;* Needle nematodes, *Longidorus* spp. such as *L. elongatus;* Lesion nematodes, *Pratylenchus* spp. such as *P. brachyurus, P. neglectus, P. penetrans, P. curvitatus, P. goodeyi;* Burrowing nematodes, *Radopholus* spp. such as *R. similis; Rhadopholus* spp.*; Rhodopholus* spp.; Reniform nematodes, *Rotylenchus* spp. such as *R. robustus, R. reniformis; Scutellonema* spp.*;* Stubby-root nematode, *Trichodorus* spp. such as *T. obtusus, T. primitivus; Paratrichodorus* spp. such as *P. minor;* Stunt nematodes, *Tylenchorhynchus spp.* such as *T. claytoni, T. dubius;* Citrus nematodes, *Tylenchulus* spp. such as *T. semi-penetrans;* Dagger nematodes, *Xiphinema* spp.*;* and other plant parasitic nematode species;
Insects from the order **Blattodea** e.g. *Macrotermes* spp. such as *M. natalensis; Cornitermes cumulans, Procornitermes* spp., *Globitermes sulfureus, Neocapritermes* spp. such as *N. opacus, N. parvus; Odontotermes spp., Nasutitermes* spp. such as *N. corniger, Coptotermes* spp. such as *C. formosanus, C. gestroi, C. acinaciformis; Reticulitermes* spp. such as *R. hesperus, R. tibialis, R. speratus, R. flavipes, R. grassei, R. lucifugus, R. virginicus; Heterotermes* spp. such as *H. aureus, H. longiceps, H. tenuis; Cryptotermes* spp. such as *C. brevis, C. cavifrons; Incisitermes* spp. such as *I. minor, I. snyderi*; *Marginitermes hubbardi, Kalotermes flavicollis, Neotermes* spp. such as *N. castaneus, Zootermopsis* spp. *such as Z. angusticollis, Z. nevadensis, Mastotermes* spp. such as *M. darwiniensis; Blatta* spp. such as *B. orientalis, B. lateralis; Blattella* spp. such as *B. asahinae, B. germanica; Rhyparobia maderae, Panchlora nivea, Periplaneta* spp. such as *P. americana, P. australasiae, P. brunnea, P. fuliginosa, P. japonica; Supella longipalpa, Parcoblatta pennsylvanica, Eurycotis floridana, Pycnoscelus surinamensis,*
Insects from the order **Siphonoptera** e.g. *Cediopsylla simples, Ceratophyllus* spp., *Ctenocephalides* spp. such as *C. felis, C. canis, Xenopsylla cheopis, Pulex irritans, Trichodectes canis, Tunga penetrans,* and *Nosopsyllus fasciatus,*
Insects from the order **Thysanura** e.g. *Lepisma saccharina, Ctenolepisma urbana,* and *Thermobia domestica,*
Pests from the class **Chilopoda** e.g. *Geophilus* spp., Scutigera spp. such as *Scutigera coleoptrata*;
Pests from the class **Diplopoda** e.g. *Blaniulus guttulatus, Julus* spp., *Narceus* spp.,
Pests from the class **Symphyla** e.g. *Scutigerella immaculata,*
Insects from the order **Dermaptera,** e.g. *Forficula auricularia,*
Insects from the order **Collembola,** e.g. *Onychiurus* spp., such as *Onychiurus armatus,*
Pests from the order **Isopoda** e.g., *Armadillidium vulgare, Oniscus asellus, Porcellio scaber,* Insects from the order **Phthiraptera,** e.g. *Damalinia* spp., Pediculus spp. such as *Pediculus humanus capitis, Pediculus humanus corporis, Pediculus humanus humanus; Pthirus pubis,* Haematopinus spp. such as *Haematopinus eurysternus, Haematopinus suis*; Linognathus spp. such as *Linognathus vituli; Bovicola bovis, Menopon gallinae, Menacanthus stramineus* and *Solenopotes capillatus, Trichodectes* spp.,
Examples of further pest species which may be controlled by compounds of fomula (I) include: from the Phylum **Mollusca,** class **Bivalvia,** e.g., *Dreissena* spp.; class **Gastropoda,** e.g., *Arion* spp., *Biomphalaria* spp., *Bulinus* spp., *Deroceras* spp., *Galba* spp., *Lymnaea* spp., *Oncomelania* spp., *Pomacea canaliclata, Succinea* spp.*;* from the class of the **helminths,** e.g., *Ancylostoma duodenale, Ancylostoma ceylanicum, Acylostoma braziliensis, Ancylostoma* spp., *Ascaris lubricoides, Ascaris spp., Brugia malayi, Brugia timori, Bunostomum* spp., *Chabertia* spp., *Clonorchis* spp., *Cooperia* spp., *Dicrocoelium* spp., *Dictyocaulus filaria, Diphyllobothrium latum, Dracunculus medinensis, Echinococcus granulosus, Echinococcus multilocularis, Enterobius vermicularis, Faciola* spp., Haemonchus spp. such as *Haemonchus contortus; Heterakis* spp., *Hymenolepis nana, Hyostrongulus* spp., *Loa Loa, Nematodirus* spp., *Oesophagostomum* spp., *Opisthorchis* spp., *Onchocerca volvulus, Ostertagia* spp., *Paragonimus* spp., *Schistosomen* spp., *Strongyloides fuelleborni, Strongyloides stercora lis, Stronyloides* spp., *Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.*

The compounds of the invention are suitable for use in treating or protecting animals against infestation or infection by parasites. Therefore, the invention also relates to the use of a compound of the invention for the manufacture of a medicament for the treatment or protection of animals against infestation or infection by parasites. Furthermore, the invention relates to a method of treating or protecting animals against infestation and infection by parasites, which comprises orally, topically or parenterally administering or applying to the animals a parasiticidally effective amount of a compound of the invention.

The invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound of the invention.

The compounds of the invention are further suitable for use in combating or controlling parasites in and on animals. Furthermore, the invention relates to a method of combating or controlling parasites in and on animals, which comprises contacting the parasites with a parasitically effective amount of a compound of the invention.

The invention also relates to the non-therapeutic use of compounds of the invention for controlling or combating parasites. Moreover, the invention relates to a non-therapeutic method of combating or controlling parasites, which comprises applying to a locus a parasiticidally effective amount of a compound of the invention.

The compounds of the invention can be effective through both contact (via soil, glass, wall, bed net, carpet, blankets or animal parts) and ingestion (e.g. baits). Furthermore, the compounds of the invention can be applied to any and all developmental stages.

The compounds of the invention can be applied as such or in form of compositions comprising the compounds of the invention.

The compounds of the invention can also be applied together with a mixing partner, which acts against pathogenic parasites, e.g. with synthetic coccidiosis compounds, polyetherantibiotics such as Amprolium, Robenidin, Toltrazuril, Monensin, Salinomycin, Maduramicin, Lasalocid, Narasin or Semduramicin, or with other mixing partners as defined above, or in form of compositions comprising said mixtures.

The compounds of the invention and compositions comprising them can be applied orally, parenterally or topically, e.g. dermally. The compounds of the invention can be systemically or non-systemically effective.

The application can be carried out prophylactically, therapeutically or non-therapeutically. Furthermore, the application can be carried out preventively to places at which occurrence of the parasites is expected.

As used herein, the term "contacting" includes both direct contact (applying the compounds/compositions directly on the parasite, including the application directly on the animal or excluding the application directly on the animal, e.g. at it's locus for the latter) and indirect contact (applying the compounds/compositions to the locus of the parasite). The contact of the parasite through application to its locus is an example of a non-therapeutic use of the compounds of the invention.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include, but are not limited to, lice, biting lice, ticks, nasal bots, keds, biting flies, muscoid flies, flies, myiasitic fly larvae, chiggers, gnats, mosquitoes and fleas.

The compounds of the invention are especially useful for combating parasites of the following orders and species, respectively:
**fleas (Siphonaptera),** e.g. *Ctenocephalides felis, Ctenocephalides canis, Xenopsylla cheopis, Pulex irritans, Tunga penetrans,* and *Nosopsyllus fasciatus;* **cockroaches (Blattaria - Blattodea),** e.g. *Blattella germanica, Blattella asahinae, Periplaneta americana, Periplaneta japonica, Periplaneta brunnea, Periplaneta fuligginosa, Periplaneta australasiae,* and *Blatta orientalis;* **flies, mosquitoes (Diptera),** e.g. *Aedes aegypti, Aedes albopictus, Aedes vexans, Anastrepha ludens, Anopheles maculipennis, Anopheles crucians, Anopheles albimanus, Anopheles gambiae, Anopheles freeborni, Anopheles leucosphyrus, Anopheles minimus, Anopheles quadrimaculatus, Calliphora vicina, Chrysomya bezziana, Chrysomya hominivorax, Chrysomya macellaria, Chrysops discalis, Chrysops silacea, Chrysops atlanticus, Cochliomyia hominivorax, Cordylobia anthropophaga, Culicoides furens, Culex pipiens, Culex nigripalpus, Culex quinquefasciatus, Culex tarsalis, Culiseta inornata, Culiseta melanura, Dermatobia hominis, Fannia canicularis, Gasterophilus intestinalis, Glossina morsitans, Glossina palpalis, Glossina fuscipes, Glossina tachinoides, Haematobia irritans, Haplodiplosis equestris, Hippelates spp., Hypoderma lineata, Leptoconops torrens, Lucilia caprina, Lucilia cuprina, Lucilia sericata, Lycoria pectoralis, Mansonia spp., Musca domestica, Muscina stabulans, Oestrus ovis, Phlebotomus argentipes, Psorophora columbiae, Psorophora discolor, Prosimulium mixtum, Sarcophaga haemorrhoidalis, Sarcophaga sp., Simulium vittatum, Stomoxys calcitrans, Tabanus bovinus, Tabanus atratus, Tabanus lineola,* and *Tabanus similis;* **lice (Phthiraptera),** e.g. *Pediculus humanus capitis, Pediculus humanus humanus, Pthirus pubis, Haematopinus eurysternus, Haematopinus suis, Linognathus vituli, Bovicola bovis, Menopon gallinae, Menacanthus stramineus* and *Solenopotes capillatus;* **ticks and parasitic mites (Parasitiformes): ticks (Ixodida),** e.g. *Ixodes scapularis, Ixodes holocyclus, Ixodes pacificus, Rhiphicephalus sanguineus, Dermacentor andersoni, Dermacentor variabilis, Amblyomma americanum, Ambryomma maculatum, Ornithodorus hermsi, Ornithodorus turicata* and **parasitic mites (Mesostigmata),** e.g. *Ornithonyssus bacoti* and *Dermanyssus gallinae*; **Actinedida (Prostigmata) und Acaridida (Astigmata),** e.g. *Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp.,* and *Laminosioptes spp;* **Bugs (Heteropterida):** *Cimex lectularius, Cimex hemipterus, Reduvius senilis, Triatoma spp., Rhodnius ssp., Panstrongylus ssp.,* and *Arilus critatus;* **Anoplurida,** e.g. *Haematopinus spp., Linognathus spp., Pediculus spp., Phtirus spp.,* and *Solenopotes spp.;* **Mallophagida (suborders Arnblycerina and Ischnocerina),** e.g. *Trimenopon spp., Menopon spp., Trinoton spp., Bovicola spp., Werneckiella spp., Lepikentron spp., Trichodectes spp.,* and *Felicola spp.;* **Roundworms Nematoda: Wipeworms and Trichinosis (Trichosyringida),** e.g. Trichinellidae *(Trichinella spp.), (*Trichuridae*) Trichuris spp., Capillaria spp.;* **Rhabditida,** e.g. *Rhabditis spp., Strongyloides spp., Helicephalobus spp.;* **Strongylida,** e.g. *Strongylus spp., Ancylostoma spp., Necator americanus, Bunostomum spp. (Hookworm), Trichostrongylus spp., Haemonchus contortus, Ostertagia spp., Cooperia spp., Nematodirus spp., Dictyocaulus spp., Cyathostoma spp., Oesophagostomum spp., Stephanurus dentatus, Ollulanus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp., Aleurostrongylus abstrusus,* and *Dioctophyma renale;* **Intestinal roundworms (Ascaridida),** e.g. *Ascaris lumbricoides, Ascaris suum, Ascaridia galli, Parascaris equorum, Enterobius vermicularis (Threadworm), Toxocara canis, Toxascaris leonine, Skrjabinema spp.,* and *Oxyuris equi;* **Camallanida,** e.g. *Dracunculus medinensis* (guinea worm); **Spirurida,** e.g. *Thelazia spp., Wuchereria spp., Brugia spp., Onchocerca spp., Dirofilari spp.a, Dipetalonema spp., Setaria spp., Elaeophora spp., Spirocerca lupi,* and *Habronema spp.;* **Thorny headed worms (Acanthocephala),** e.g. *Acanthocephalus spp., Macracanthorhynchus hirudinaceus* and *Oncicola spp.;* **Planarians (Plathelminthes): Flukes (Trematoda),** e.g. *Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp.,* and *Nanocyetes spp.;* **Cercomeromorpha,** in particular **Cestoda (Tape-worms),** e.g. *Diphyllobothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp.,* and *Hymenolepis spp..*

As used herein, the term "animal" includes warm-blooded animals (including humans) and fish. Preferred are mammals, such as cattle, sheep, swine, camels, deer, horses, pigs, poultry, rabbits, goats, dogs and cats, water buffalo, donkeys, fallow deer and reindeer, and also in furbearing animals such as mink, chinchilla and raccoon, birds such as hens, geese, turkeys and ducks and fish such as fresh- and salt-water fish such as trout, carp and eels. Particularly preferred are domestic animals, such as dogs or cats.

In general, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions such as desired parasiticidal effect and duration, target species, mode of application.

Generally, it is favorable to apply the compounds of the invention in total amounts of 0.5 mg/kg to 100 mg/kg per day, preferably 1 mg/kg to 50 mg/kg per day.

For oral administration to warm-blooded animals, the formula I compounds may be formulated as animal feeds, animal feed premixes, animal feed concentrates, pills, solutions, pastes, suspensions, drenches, gels, tablets, boluses and capsules. In addition, the formula I compounds may be administered to the animals in their drinking water. For oral administration, the dosage form chosen should provide the animal with 0.01 mg/kg to 100 mg/kg of animal body weight per day of the formula I compound, preferably with 0.5 mg/kg to 100 mg/kg of animal body weight per day.

Alternatively, the formula I compounds may be administered to animals parenterally, e.g., by intraruminal, intramuscular, intravenous or subcutaneous injection. The formula I compounds may be dispersed or dissolved in a physiologically acceptable carrier for subcutaneous injection. Alternatively, the formula I compounds may be formulated into an implant for subcutaneous administration. In addition the formula I compound may be transdermally administered to animals. For parenteral administration, the dosage form chosen should provide the animal with 0.01 mg/kg to 100 mg/kg of animal body weight per day of the formula I compound.

The formula I compounds may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the formula I compound. In addition, the formula I compounds may be formulated as ear tags for animals, particularly quadrupeds such as cattle and sheep.

Suitable preparations are:
- Solutions such as oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations such as powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further auxiliaries such as acids, bases, buffer salts, preservatives, and solubilizers. Suitable auxiliaries for injection solutions are known in the art. The solutions are filtered and filled sterile.

Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on. Solutions for use on the skin are prepared according to the state of the art and according to what is described above for injection solutions, sterile procedures not being necessary.

Gels are applied to or spread on the skin or introduced into body cavities. Gels are prepared by treating solutions which have been prepared as described in the case of the injection solutions with sufficient thickener that a clear material having an ointment-like consistency results. Suitable thickeners are known in the art.

Pour-on formulations are poured or sprayed onto limited areas of the skin, the active compound penetrating the skin and acting systemically. Pour-on formulations are prepared by dissolving, suspending or emulsifying the active compound in suitable skin-compatible solvents or solvent mixtures. If appropriate, other auxiliaries such as colorants, bioabsorption-promoting substances, antioxidants, light stabilizers, adhesives are added. Suitable such auxiliaries are known in the art.

Emulsions can be administered orally, dermally or as injections. Emulsions are either of the water-in-oil type or of the oil-in-water type. They are prepared by dissolving the active compound either in the hydrophobic or in the hydrophilic phase and homogenizing this with the solvent of the other phase with the aid of suitable emulsifiers and, if appropriate, other auxiliaries such as colorants, absorption-promoting substances, preservatives, antioxidants, light stabilizers, viscosity-enhancing substances. Suitable hydrophobic phases (oils), suitable hydrophilic phases, suitable emulsifiers, and suitable further auxiliaries for emulsions are known in the art.

Suspensions can be administered orally or topically/dermally. They are prepared by suspending the active compound in a suspending agent, if appropriate with addition of other auxiliaries such as wetting agents, colorants, bioabsorption-promoting substances, preservatives, antioxidants, light stabilizers. Suitable suspending agents, and suitable other auxiliaries for suspensions including wetting agents are known in the art.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form. Suitable auxiliaries for this purpose are known in the art.

The compositions which can be used in the invention can comprise generally from about 0.001 to 95% of the compound of the invention.

Ready-to-use preparations contain the compounds acting against parasites, preferably ectoparasites, in concentrations of 10 ppm to 80 per cent by weight, preferably from 0.1 to 65 per cent by weight, more preferably from 1 to 50 per cent by weight, most preferably from 5 to 40 per cent by weight.

Preparations which are diluted before use contain the compounds acting against ectoparasites in concentrations of 0.5 to 90 per cent by weight, preferably of 1 to 50 per cent by weight.

Furthermore, the preparations comprise the compounds of formula I against endoparasites in concentrations of 10 ppm to 2 per cent by weight, preferably of 0.05 to 0.9 per cent by weight, very particularly preferably of 0.005 to 0.25 per cent by weight.

Topical application may be conducted with compound-containing shaped articles such as collars, medallions, ear tags, bands for fixing at body parts, and adhesive strips and foils.

Generally it is favorable to apply solid formulations which release compounds of the invention in total amounts of 10 mg/kg to 300 mg/kg, preferably 20 mg/kg to 200 mg/kg, most preferably 25 mg/kg to 160 mg/kg body weight of the treated animal in the course of three weeks.

### Examples

### A. Preparation examples

With appropriate modification of the starting materials, the procedures given in the synthesis description were used to obtain further compounds I. The compounds obtained in this manner are listed in the table that follows, together with physical data.

The products shown below were characterized by melting point determination, by NMR spectroscopy or by the masses ([m/z]) or retention time (RT; [min.]) determined by HPLC-MS or HPLC spectrometry.
HPLC-MS = high performance liquid chromatography-coupled mass spectrometry;
HPLC method A: HPLC Phenomenex Kinetex 1,7µm XB-C18 100A, 50 x 2,1mm", Mobile Phase: A: water + 0,1% TFA; B:CAN; Temperature: 60°C; Gradient:5% B to 100% B in 1,50min; 100% B 0,25min; Flow: 0,8ml/min to 1,0ml/min in 1,51 min; MS method: ESI positive; Mass range (m/z): 100-700.

HPLC method B: HPLC method: Merck Chromolith SpeedROD RP-18e, 50 x 4,6mm; mobile phase: A: water + 0,1% TFA; B: ACN+0,1%TFA; gradient: 5% B to 100% B in 4,00min; 100% B 2,00min; flow: 1,8ml/min to 2,1ml/min in 7,00min at 40 °C. MS method: ESI positive, mass range (m/z): 100-700.

The syntheses of 2-chloro-4-[5-(3,5-dichloro-4-fluoro-phenyl)-5-(trifluoromethyl)-4H-isoxazol-3-yl]benzoic acid, 2-chloro-4-[5-(3,5-dichlorophenyl)-5-(trifluoromethyl)-4H-isoxazol-3-yl]benzoic acid, and 4-[5-(3,5-dichlorophenyl)-5-(trifluoromethyl)-4H-isoxazol-3-yl]-2-methyl-benzoic acid were performed in analogy to WO 2017/050922 and WO 2013/026695.

### Example 1: Synthesis of 2-chloro-4-[5-(3,5-dichloro-4-fluoro-phenyl)-5-(trifluoromethyl)-4H-isoxazol-3-yl]-N-[(3-methyl-4-oxo-oxazolidin-5-yl)methyl]benzamide [I-3-5].

Step 1: 2-Hydroxy-3-nitro-propanoic acid: To a solution of 2,2-dihydroxyacetic acid (150 g, 2.00 mol) in H₂O (500 mL) were slowly added NaOH (1N in H₂O, 2.0 L), followed by MeNO₂ (501 g, 8.20 mol). The resulting reaction mixture was stirred at 25 °C for 19 h and then heated to 70°C for 1 h. Then the reaction mixture was quenched into 6N H₂SO₄ to pH = 3, and the aqueous phase was extracted with EtOAc (3 × 500 mL), dried over Na₂SO₄, filtered and concentrated under reduced pressure to give 2-hydroxy-3-nitro-propanoic acid (80 g, crude) as a yellow oil.
¹H NMR (400 MHz, CDCl₃, RT) *δ* 4.82 - 4.87 (m, 1H) 4.68 - 4.74 (m, 1H) 4.59 (dd, J = 7.1, 4.0 Hz, 1H)

Step 2: 2-Hydroxy-*N*-methyl-3-nitro-propanamide: To a solution 2-hydroxy-3-nitro-propanoic acid (20.0 g, 148 mmol) in EtOAc (500 mL) was added MeNH₂ (1.0 M in THF, 220 mL) dropwise over 15 min. The reaction mixture was stirred at 25 °C for 15 min and then cooled to a temperature between -5 °C and 0 °C. A solution of 1-hydroxybenzotriazol (HOBt)/THF/H₂O (29.9 g, 0.220 mol /100 mL/ 20 mL) and 1-ethyl-3-(3-dimethylaminopropyl)carbodiimid (EDCI)/H₂O (42.0 g, 0.22 mol/ 80 mL) was prepared separately and added dropwise to the reaction mixture over 30 min at 0 °C. The resulting reaction mixture was stirred at 0 °C for 4 h, after which time TLC (EtOAc, Rf = 0.3, I₂) showed the reaction was completed. The reaction was quenched into NaHCO₃ solution (sat. aq., 200 mL), extracted with EtOAc (2 × 200 mL), dried over Na₂SO₄, filtered, and concentrated under reduced pressure. Purification by column chromatography (PE: EtOAc = 3:1) afforded 2-hydroxy-*N*-methyl-3-nitro-propanamide (14.0 g, 66% yield) as yellow solid.
¹H NMR (400 MHz, CDCl₃, RT) δ 8.0 (br d, J = 3.3 Hz, 1H), 6.5 (d, J = 6.0 Hz, 1H), 4.84 (dd, J = 12.7, 3.0 Hz, 1H), 4.49-4.64 (m, 2H), 2.62 (d, J = 4.8 Hz, 3H).

Step 3: 3-Methyl-5-(nitromethyl)oxazolidin-4-one: To a solution of 2-hydroxy-*N*-methyl-3-nitro-propanamide (10 g, 67 mmol) in toluene (200 mL) at 25 °C under an atmosphere of N₂ was added paraformaldehyde (9.0 g, 300 mmol), followed by p-TsOH (6.4 g, 34 mmol). The resulting mixture was heated to 120 °C and stirred at that temperature for 16 h. TLC analysis (EtOAc, Rf = 0.5, I₂) indicated completion and the reaction mixture was concentrated under reduced pressure. Purification by column chromatography (PE: EtOAc = 3:1, R_{f} = 0.4, I₂) afforded 3-methyl-5-(nitromethyl)oxazolidin-4-one (7.5 g, 75% yield) as brown oil.
¹H NMR (400 MHz, CDCl₃, RT) δ ppm 5.01 - 5.10 (m, 2H) 4.68 - 4.86 (m, 3H) 2.94 (s, 3H).

Step 4: 5-(Aminomethyl)-3-methyl-oxazolidin-4-one: A mixture of 3-Methyl-5-(nitromethyl)-oxazolidin-4-one (7.5 g, 47 mmol) and Raney Ni (3 g) in MeOH (200 mL) was stirred under of H₂ (50 psi) at 25 °C for 3 h. TLC (EtOAc, R_{f} = 0.1, I₂) showed the reaction was completed. The reaction mixture was filtered and filter cake washed with MeOH (200 mL) and concentrated to give the crude product which further purified by column (DCM/MeOH = 10:1, R_{f} = 0.1, I₂) to give the 5-(aminomethyl)-3-methyl-oxazolidin-4-one (5.0 g, 81% yield) as yellow oil.
¹H NMR (400 MHz, CDCl₃, RT) δ ppm 5.07 - 5.00 (m, 2H), 4.27 (br s, 1 H), 3.07 (d, J = 4.2 Hz, 2H), 2.90 (s, 3H), 1.50 (br s, 3H).

Step 5: 2-chloro-4-[5-(3,5-dichloro-4-fluoro-phenyl)-5-(trifluoromethyl)-4H-isoxazol-3-yl]-N-[(3-methyl-4-oxo-oxazolidin-5-yl)methyl]benzamide [I-3-5]: To a suspension of 2-chloro-4-[5-(3,5-dichloro-4-fluoro-phenyl)-5-(trifluoromethyl)-4H-isoxazol-3-yl]benzoic acid (1.00 g, 2.19 mmol) in CH₂Cl₂ (15 mL) at ambient temperature was added DMF (2 drops), followed by oxalyl chloride (834 mg, 6.57 mmol), and the resulting reaction mixture was stirred at that temperature over night. After that time, the reaction mixture was concentrated under reduced pressure, re-dissolved in CHCl₃ (15 mL) and concentrated again under reduced pressure (2x) to afford the crude acid chloride as an orange oil (1.12 g), which was used as described below without further purification.

In a separate flask, 5-(aminomethyl)-3-methyl-oxazolidin-4-one (164 mg, 1.26 mmol) was dissolved in THF (10 mL), followed by the addition of DMAP (13 mg, 0.11 mmol) and iPr₂NEt (1.1 mL, 6.3 mmol). The mixture was cooled to 0 °C and a solution of the above acid chloride (500 mg, 1.05 mmol) in THF (5.0 mL) was added dropwise. The resulting raction mixture was allowed warm to ambient temperature and then stirred for 2 days. After that time, the reaction mixture was concentrated under reduced pressure, re-dissolved in EtOAc and the organic phase washed with HCI solution (1.0 M in H₂O), H₂O, and NaHCO₃ solution (sat. aq.), dried over Na₂SO₄, filtered, and concentrated under reduced pressure. Purification by column chromatography (EtOAc/cyclohexane 0:100 to 50:50, gradient) afforded 2-chloro-4-[5-(3,5-dichloro-4-fluoro-phenyl)-5-(trifluoromethyl)-4H-isoxazol-3-yl]-N-[(3-methyl-4-oxo-oxazolidin-5-yl)methyl]benzamide (390 mg, 65%).
¹H NMR (400 MHz, CDCl₃, RT) δ ppm 7.75 (d, J = 8.1 Hz, 1H), 7.71 (d, J = 1.7 Hz, 1H), 7.64-7.54 (m, 3H), 6.75 (t, J = 6.2 Hz, 1H), 5.04 (d, J = 1.8 Hz, 2H), 4.43 (t, J = 5.7 Hz, 1H), 4.07 (d, J = 17.3 Hz, 1H), 3.94 (ddd, J = 13.8, 6.6, 5.2 Hz, 1H), 3.81 (dt, J = 14.0, 5.6 Hz, 1H), 3.68 (d, J = 17.3 Hz, 1H), 2.90 (s, 3H).

### Example 2: Synthesis of 2-chloro-4-[5-(3,5-dichlorophenyl)-5-(trifluoromethyl)-4H-isoxazol-3-yl]-N-[(3-methyl-2,4-dioxo-oxazolidin-5-yl)methyl]benzamide [I-4-1].

Step 1: Benzyl *N*-[2-hydroxy-3-(methylamino)-3-oxo-propyl]carbamate: To a solution of 3-(benzyloxycarbonylamino)-2-hydroxy-propanoic acid (15 g, 62 mmol) in CH₂Cl₂ (500 mL) at 25 °C was added NEt₃ (20 g, 200 mmol), followed by EDCI (15.5 g, 81.7 mmol), HOBt (11 g, 82 mmol), and DMAP (1.5 g, 13 mmol). The resulting mixture was stirred for 15 min, before MeNH₂ in THF (63 mL, 2 eq) was added dropwise to the reaction mixture and stirring was continued for another 15 h. After that time, TLC analysis (EtOAc, R_{f} = 0.3, I₂) indicated completion, and the reaction mixture was quenched into NaHCO₃ solution (sat. aqueous, 200 mL). The aqueous phase was extracted with CH₂Cl₂ (2 × 200 mL), and the combined organic phases were wash with 2N HCI (200 mL), dried over Na₂SO₄, filtered and concentrated under reduced pressure to give benzyl N-[2-hydroxy-3-(methylamino)-3-oxo-propyl]carbamate (12 g, 75% yield) as white solid.
¹H NMR (400 MHz, CDCl₃, RT) δ ppm 7.29 - 7.40 (m, 6H) 7.01 (br s, 1H) 5.68 (br s, 1H) 5.11 (s, 2H) 5.06 (br d, J = 8.7 Hz, 1H) 4.20 (dd, J = 5.1, 2.8 Hz, 1H) 3.60 - 3.66 (m, 1H) 3.49 - 3.57 (m, 1H) 2.81 (d, J = 5.0 Hz, 3H).

Step 2: Benzyl *N*-[(3-methyl-2,4-dioxo-oxazolidin-5-yl)methyl]carbamate: To a solution of benzyl *N*-[2-hydroxy-3-(methylamino)-3-oxo-propyl]carbamate (24 g, 95 mmol) in CH₂Cl₂ (100 mL)/DMF (300 mL) at 25°C under N₂ atmosphere was added N,N'-disuccinimidyl carbonate (97 g, 380 mmol) over 4 h, then the reaction mixture was heated to 70°C and stirred at that temperature for 72 h. TLC analysis (EtOAc, R_{f} = 0.5, I₂) showed that the reaction was complete, and the reaction was quenched into H₂O (500 mL). The aqueous phase was extracted with EtOAc (2 × 200 mL) and the combined organic phases were wash with H₂O (300 mL x 2), dried over Na₂SO₄, filtered, and concentrated under reduced pressure. Purification by column chromatography (PE: EtOAc = 5:1, R_{f} = 0.1, I₂) afforded benzyl *N*-[(3-methyl-2,4-dioxo-oxazolidin-5-yl)methyl]carbamate (25 g, 94% yield) as white solid.
¹H NMR (400 MHz, CDCl₃, RT) δ ppm 7.31 - 7.38 (m, 4H) 5.25 (br s, 1H) 5.11 (s, 2H) 4.88 (br s, 1H) 3.75 - 3.82 (m, 1H), 3.65 - 3.73 (m, 1H) 3.04 (s, 3H).

Step 3: 5-(Aminomethyl)-3-methyl-oxazolidine-2,4-dione hydrochloride: To a solution of benzyl *N*-[(3-methyl-2,4-dioxo-oxazolidin-5-yl)methyl]carbamate (20 g, 72 mmol) in THF (500 mL) at 25°C was added Pd/C (5 g), the vessel was purged with H₂ (50 psi), and the resulting reaction mixture stirred at that temperature for 5 h. TLC analysis (EtOAc, Rf = 0.1, I₂) showed that the reaction was complete. The reaction mixture was filtered, the filter cake washed with THF (50 mL), and the filtrate concentrated under reduced pressure. To the residue was added HCl/EtOAc (50 mL) at 25°C for 2 h. The reaction mixture was filtered, and filter cake washed with THF (50 mL) concentrated to give 5-(Aminomethyl)-3-methyl-oxazolidine-2,4-dione hydrochloride (6.0 g, 46% yield) as white solid.
¹H NMR (400 MHz, CDCl₃, RT) δ ppm 5.34 (dd, J = 8.5, 3.45 Hz, 1H) 3.63 (dd, J = 14.2, 3.4 Hz, 1H) 3.44-3.51 (m, 1H).

Step 4: From the above 5-(aminomethyl)-3-methyl-oxazolidine-2,4-dione hydrochloride the desired 2-chloro-4-[5-(3,5-dichlorophenyl)-5-(trifluoromethyl)-4H-isoxazol-3-yl]-N-[(3-methyl-2,4-dioxooxazolidin-5-yl)methyl]benzamide [I-4-1] was obtained analogously as described in Example 1 (Step 5).

### Example 3: Synthesis of 4-[5-(3,5-dichlorophenyl)-5-(trifluoromethyl)-4H-isoxazol-3-yl]-2-methyl-N-(3-methyl-2,4-dioxo-thiazolidin-5-yl)benzamide [I-1-4].

Step 1: 3-Methylthiazolidine-2,4-dione: To a solution of thiazolidine-2,4-dione (50 g, 0.43 mol) in DMF (300 mL), at 25°C under N₂ atmosphere, were slowly added K₂CO₃ (70 g, 0.51 mol), and CH₃I (73 g, 0.51 mol), and the resulting reaction mixture was heated at 80 °C for 16 h. After that time, TLC analysis (PE: EtOAc = 3:1, Rf = 0.3) showed that the reaction was complete. The reaction mixture was quenched into H₂O (1.0 L), extracted with EtOAc (3 × 200 mL), and the combined organic extracts were washed with H₂O (2 × 500 mL), NaCl solution (sat. aqueous, 1 × 200 mL), dried over Na₂SO₄, filtered and concentrated under reduced pressure. Purification by column chromatography (PE/EtOAc = 5:1, R_{f} = 0.3) afforded 3-methylthiazolidine-2,4-dione (40 g, 72% yield) as yellow oil.
¹H NMR (400 MHz, CDCl₃, RT) δ ppm 3.96 (s, 2H) 3.18 (s, 3H).

Step 2: 5-Bromo-3-methyl-thiazolidine-2,4-dione: To a solution of 3-methylthiazolidine-2,4-di-one (40 g, 0.31 mol) in AcOH (700 mL) at 0°C was added Br₂ (60 g, 0.37 mol) dropwise, before the cooling bath was removed and the reaction mixture heated at 60 °C for 16 h. TLC analysis (PE/EtOAc = 3:1, R_{f} = 0.6) showed that the reaction was complete. The reaction was quenched into water (1.0 L), the aqueous phase extracted with EtOAc (3 × 300 mL), and the combined organic extracts were washed with NaHCO₃ solution (sat. aq., 1 × 700 mL), NaCl solution (sat. aqueous, 1 × 200 mL) dried over Na₂SO₄, filtered and concentrated under reduced pressure. Purification by colum chromatography (PE:EtOAc = 10:1, Rf = 0.6) afforded 5-bromo-3-methylthiazolidine-2,4-dione (60 g, 63% yield) as light yellow oil.
¹H NMR (400 MHz, CDCl₃, RT) δ ppm 5.86 (d, J = 0.8 Hz, 1H) 3.21-3.22 (m, 3H).

Step 3: 5-(Benzhydrylideneamino)-3-methyl-thiazolidine-2,4-dione: To a solution of 5-bromo-3-methyl-thiazolidine-2,4-dione (25 g, 0.12 mol) in MeCN (1.0 L), at 25 °C and under an atmosphere of N₂, was added diphenylmethanimine (65 g, 0.36 mol), and the resulting reaction mixture was then heated at 75 °C for 24 h. TLC analysis (PE/EtOAc = 5/1, R_{f} = 0.6) showed that the reaction was complete. The reaction mixture was filtered, the filter cake washed with MeCN (100 mL), and the filtrate concentrated under reduced pressure. Purification by column chromatography (PE/EtOAc = 10:1, Rf = 0.6) afforded the desired 5-(benzhydrylideneamino)-3-methylthiazolidine-2,4-dione (7.0 g, crude) as yellow solid, which was used in next step.

Step 4: 5-Amino-3-methyl-thiazolidine-2,4-dione hydrochloride: To a solution of 5-(benzhydrylideneamino)-3-methyl-thiazolidine-2,4-dione (7.0 g, 23 mmol) in THF (10 mL) at 25 °C was added H₂O (0.80 g, 45 mmol), followed by HCI (4.0 M in EtOAc, 20 mL), and the resulting reaction mixture was stirred at that temperature for 4 h. Some precipitate was formed. After that time, TLC analysis (PE/EtOAc = 3/1, R_{f} = 0.2) showed that the reaction was complete. The reaction mixture was filtered, the filter cake washed with THF (10 mL), the filter cake concentrated to afford the desired 5-amino-3-methyl-thiazolidine-2,4-dione hydrochloride (3.0 g, 14% over two steps) as a white solid.
¹H NMR (400 MHz, CDCl₃, RT) δ ppm 5.80 (s, 1H) 3.01 (s, 3H).

Step 5: From the above 5-Amino-3-methyl-thiazolidine-2,4-dione hydrochloride the desired 4-[5-(3,5-dichlorophenyl)-5-(trifluoromethyl)-4H-isoxazol-3-yl]-2-methyl-N-(3-methyl-2,4-dioxo-thiazolidin-5-yl)benzamide [I-1-4] was obtained analogously as described in Example 1.

**Table C.1 - Compounds of formula I.A (Z = N; R⁷ = H)**

| | | | | | | | | | | | |
|---|---|---|---|---|---|---|---|---|---|---|---|
| 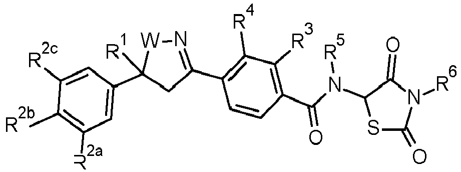 | | | | | | | | I.A (Z=N;R⁷ =H) | | | |

| No. | R^{2a} | R^{2b} | R^{2c} | R³ | R⁴ | R⁵ | W | R⁶ | HPLC Rt [min] | M+H [m/z] | HPLC Method |
|---|---|---|---|---|---|---|---|---|---|---|---|
| I-1-1 | Cl | H | Cl | Cl | H | H | O | CH₃ | 1.377 | 565.9 | A |
| I-1-2 | Cl | H | Cl | Cl | H | H | O | C₂H₅ | 1.406 | 581.7 | A |
| I-1-3 | Cl | H | Cl | Cl | H | H | O | CH(CH₃)₂ | 1.445 | 595.9 | A |
| I-1-4 | Cl | H | Cl | CH₃ | H | H | O | CH₃ | 1.359 | 547.6 | A |
| I-1-5 | Cl | H | Cl | CH₃ | H | H | O | C₂H₅ | 1.399 | 559.9 | A |
| I-1-6 | Cl | H | Cl | CH₃ | H | H | O | i-Pr | 1.440 | 573.9 | A |
| I-1-7 | Cl | F | Cl | Cl | H | H | O | C₂H₅ | 1.399 | 599.9 | A |
| I-1-8 | CF₃ | H | Cl | -CH₂-CH₂-CH₂- | | H | O | CH₃ | 1.426 | 606.0 | A |
| I-1-9 | Cl | F | Cl | -CH₂-CH₂-CH₂- | | H | O | CH₃ | 1.430 | 591.6 | A |
| I-1-10 | Cl | F | Cl | -O-CH₂-CH₂- | | H | O | CH(CH₃)₂ | 1.472 | 619.8 | A |
| I-1-11 | Cl | F | Cl | CH₃ | H | H | O | CH(CH₃)₂ | 1.432 | 591.8 | A |
| I-2-1 | Cl | F | Cl | -CH₂-CH₂-CH₂- | | H | CH₂ | C₂H₅ | 1.414 | 601.8 | A |

**Table C.2 - Compounds of formula I.D (Z = N)**

| | | | | | | | | | | | | |
|---|---|---|---|---|---|---|---|---|---|---|---|---|
| 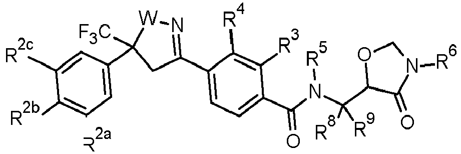 | | | | | | | | I.D (Z =N) | | | | |

| No. | R^{2a} | R^{2b} | R^{2c} | R³ | R⁴ | R⁵ | W | R⁶ | R⁸, R⁹ | HPLC Rt [min] | M+H [m/z] | HPLC Method |
|---|---|---|---|---|---|---|---|---|---|---|---|---|
| I-3-1 | Cl | F | Cl | -CH₂-CH₂-CH₂- | | H | O | CH₃ | H,H | 3.60 | 574.1 | B |
| I-3-2 | Cl | F | Cl | -CH₂-CH₂-CH₂- | | H | O | CH₂-c-Pr | H,H | 3.78 | 614.1 | B |
| I-3-3 | CF₃ | H | Cl | -CH₂-CH₂-CH₂- | | H | O | CH₃ | H,H | 3.68 | 590.1 | B |
| I-3-4 | CF₃ | H | Cl | -CH₂-CH₂-CH₂- | | H | O | CH₂-c-Pr | H,H | 3.83 | 630.1 | B |
| I-3-5 | Cl | F | Cl | Cl | H | H | O | CH₃ | H,H | 3.48 | 570.0 | B |
| I-3-6 | Cl | F | Cl | Cl | H | H | O | CH₂-c-Pr | H,H | 3.68 | 608.0 | B |
| I-3-7 | Cl | H | Cl | Cl | H | H | O | CH₃ | H,H | 1.264 | 551.8 | A |
| I-3-8 | Cl | H | Cl | Cl | H | H | O | C₂H₅ | H,H | 1.300 | 563.8 | A |
| I-3-9 | Cl | H | Cl | Cl | H | H | O | CH₂CF₃ | H,H | 1.356 | 617.8 | A |
| I-3-10 | Cl | H | Cl | Cl | H | H | O | CH₂-c-Pr | H,H | 1.352 | 591.8 | A |
| I-3-11 | Cl | H | Cl | CH₃ | H | H | O | CH₃ | H,H | 1.251 | 529.9 | A |
| I-3-12 | Cl | H | Cl | CH₃ | H | H | O | C₂H₅ | H,H | 1.287 | 543.9 | A |
| I-3-13 | Cl | H | Cl | CH₃ | H | H | O | CH₂CF₃ | H,H | 1.345 | 597.8 | A |
| I-3-14 | Cl | H | Cl | CH₃ | H | H | O | CH₂-c-Pr | H,H | 1.347 | 570.1 | A |

**Table C.3 - Compounds of formula I.C (Z = N)**

| | | | | | | | | | | | | |
|---|---|---|---|---|---|---|---|---|---|---|---|---|
| 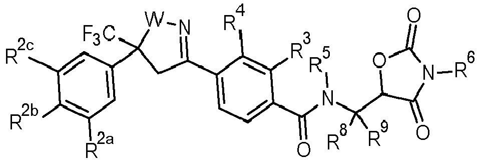 | | | | | | | | I.C (Z = N) | | | | |

| No. | R^{2a} | R^{2b} | R^{2c} | R³ | R⁴ | R⁵ | W | R⁶ | R⁸, R⁹ | HPLC Rt [min] | M+H [m/z] | HPLC Method |
|---|---|---|---|---|---|---|---|---|---|---|---|---|
| I-4-1 | Cl | H | Cl | Cl | H | H | O | CH₃ | H,H | 1.317 | 566.0 | A |
| I-4-2 | Cl | H | Cl | Cl | H | H | O | CH₂CF₃ | H,H | 1.387 | 633.8 | A |
| I-4-3 | Cl | H | Cl | Cl | H | H | O | CH₂-c-Pr | H,H | 1.398 | 605.9 | A |
| I-4-4 | Cl | H | Cl | CH₃ | H | H | O | CH₃ | H,H | 1.311 | 544.0 | A |
| I-4-5 | Cl | H | Cl | CH₃ | H | H | O | CH₂CF₃ | H,H | 1.370 | 611.8 | A |
| I-4-6 | Cl | H | Cl | CH₃ | H | H | O | CH₂-c-Pr | H,H | 1.381 | 583.9 | A |

### II. Evaluation of pesticidal activity:

The activity of the compounds of formula I of the invention can be demonstrated and evaluated by the following biological test.

### B1. Yellow fever mosquito (Aedes aegypti)

For evaluating control of yellow fever mosquito (Aedes aegypti) the test unit consisted of 96-well-microtiter plates containing 200µl of tap water per well and 5-15 freshly hatched A. aegypti larvae.

The active compounds were formulated using a solution containing 75% (v/v) water and 25% (v/v) DMSO. Different concentrations of formulated compounds or mixtures were sprayed onto the insect diet at 2.5µl, using a custom built micro atomizer, at two replications.

After application, microtiter plates were incubated at 28 + 1°C, 80 + 5 % RH for 2 days. Larval mortality was then visually assessed.

In this test, compounds I-1-1, I-1-2, I-1-3, I-1-4, I-1-5, I-1-6, I-1-7, I-1-8, I-1-9, I-1-10, I-1-11, I-2-1, I-3-1, I-3-2, I-3-3, I-3-4, I-3-5, I-3-6, I-3-8, I-3-9, I-3-10, I-3-11, I-3-13, I-3-14, I-4-2, I-4-3, I-4-4, I-4-5, and I-4-6, resp., at 2500 ppm showed over 75% mortality in comparison with untreated controls.

### B2. Orchid thrips (dichromothrips corbetti)

Dichromothrips corbetti adults used for bioassay are obtained from a colony maintained continu-ously under laboratory conditions. For testing purposes, the test compound is diluted in a 1:1 mix-ture of acetone:water (vol:vol), plus Kinetic at a rate of 0.01% v/v.

Thrips potency of each compound is evaluated by using a floral-immersion technique. Each orchid petal is dipped into treatment solution and allowed to dry in Petri dishes. Treated petals are placed into individual re-sealable plastic along with about 20 adult thrips. All test arenas are held under dark condition and a temperature of about 28°C for duration of the assay. The percent mor-tality is recorded 72 hours after treatment.

In this test, compounds I-1-1, 1-1-2, 1-1-3, 1-1-4, 1-1-5, 1-1-6, 1-1-7, I-1-8, I-1-9, 1-1-10, I-1-11, I-2-1, I-3-1, I-3-2, I-3-3, I-3-4, I-3-5, I-3-6, I-3-7, I-3-8, I-3-9, I-3-10, I-3-11, I-3-12, I-3-13, I-3-14, I-4-1, I-4-2, I-4-3, I-4-4, I-4-5, and I-4-6, resp., at 300 ppm showed over 75% mortality in comparison with untreated controls.

### B3. Boll weevil (Anthonomus grandis)

For evaluating control of boll weevil (Anthonomus grandis) the test unit consisted of 96-well-microtiter plates containing an insect diet and 5-10 A. grandis eggs.

The compounds were formulated using a solution containing 75% v/v water and 25% v/v DMSO. Different concentrations of formulated compounds were sprayed onto the insect diet at 5 µl, using a custom built micro atomizer, at two replications.

After application, microtiter plates were incubated at about 25 + 1°C and about 75 + 5 % relative humidity for 5 days. Egg and larval mortality was then visually assessed.

In this test, compounds I-1-1, I-1-2, 1-1-3, 1-1-4, 1-1-5, 1-1-6, 1-1-7, I-1-8, I-1-9, 1-1-10, I-1-11, I-2-1, 1-3-1, I-3-2, I-3-3, I-3-4, I-3-5, I-3-6, I-3-7, I-3-8, I-3-9, I-3-10, I-3-11, I-3-12, I-3-13, I-3-14, I-4-1, I-4-2, I-4-3, I-4-4, I-4-5, and I-4-6, resp., at 2500 ppm showed over 75% mortality in comparison with untreated controls.

### B4. Cowpea aphid (Aphis craccivora)

The active compound is dissolved at the desired concentration in a mixture of 1:1 (vol: vol) distilled water: acetone. Surfactant (Kinetic) is added at a rate of 0.01% (vol/vol). The test solution is prepared at the day of use.

Potted cowpea plants are colonized with approximately 30 - 50 aphids of various stages by man-ually transferring aphid-infested leaf clippings 24 hours before application. Plants are sprayed with the test solutions using a DeVilbiss® hand atomizer at 20- 30 psi (≈ 1.38 to 2.07 bar) after the pest population has been checked. Treated plants are maintained on light carts at about 25- 26°C. Estimate percent mortality is assessed after 72 hours.

In this test, compounds I-1-1, I-1-2, I-1-5, I-1-6, I-1-7, I-1-9, I-1-11, I-2-1, I-3-1, I-3-3, I-3-5, and I-3-11, resp., at 300 ppm showed over 75% mortality in comparison with untreated controls.

### B5. Caenorhabditis elegans

For evaluating control of Caenorhabditis elegans through contact or systemic means the test unit consisted of 96-well-microtiter plates containing a liquid diet.

The compounds or mixtures were formulated using a solution containing 75% water and 25% DMSO. Different concentrations of formulated compounds were sprayed into the microtiter plate wells at 5µl per well, using a custom built micro atomizer, at two replications. Mixed instar 60-100 C. elegans were transferred into the micro-titer plate wells. After application, the nematodes were incubated at 18 + 1°C, 70 + 5 % RH for 4 days. Nematode motility (mortality) was then visually assessed.

In this test, compounds I-1-1, I-1-2, I-1-3, I-1-7, I-1-9, I-1-11, I-2-1, I-3-1, I-3-2, I-3-5, I-3-7, I-3-8, I-3-9, I-3-10, I-3-11, I-3-12, I-3-13, I-3-14, I-4-1, I-4-2, I-4-3, I-4-4, I-4-5, and I-4-6, resp., at 2500 ppm showed over 75% mortality in comparison with untreated controls.

### B6. Tobacco budworm (Heliothis virescens)

For evaluating control of tobacco budworm (Heliothis virescens) the test unit consisted of 96-well-microtiter plates containing an insect diet and 15-25 H. virescens eggs.

The compounds were formulated using a solution containing 75% v/v water and 25% v/v DMSO. Different concentrations of formulated compounds were sprayed onto the insect diet at 10 µl, us-ing a custom built micro atomizer, at two replications.

After application, microtiter plates were incubated at about 28 + 1°C and about 80 + 5 % relative humidity for 5 days. Egg and larval mortality was then visually assessed.

In this test, compounds I-1-1, I-1-2, I-1-3, I-1-4, I-1-5, I-1-6, I-1-7, I-1-8, I-1-9, I-1-10, I-1-11, I-2-1, 1-3-1, I-3-2, I-3-3, I-3-4, I-3-5, I-3-6, I-3-7, I-3-8, I-3-9, I-3-10, I-3-11, I-3-12, I-3-13, I-3-14, I-4-1, I-4-2, I-4-3, I-4-4, I-4-5, and I-4-6, resp., at 2500 ppm showed over 75% mortality in comparison with untreated controls.

### B7. Vetch aphid (Megoura viciae)

For evaluating control of vetch aphid (Megoura viciae) through contact or systemic means the test unit consisted of 24-well-microtiter plates containing broad bean leaf disks.

The compounds were formulated using a solution containing 75% v/v water and 25% v/v DMSO. Different concentrations of formulated compounds were sprayed onto the leaf disks at 2.5 µl, us-ing a custom built micro atomizer, at two replications.

After application, the leaf disks were air-dried and 5 - 8 adult aphids placed on the leaf disks in-side the microtiter plate wells. The aphids were then allowed to suck on the treated leaf disks and incubated at about 23 + 1°C and about 50 + 5 % relative humidity for 5 days. Aphid mortality and fecundity was then visually assessed.

In this test, compounds I-1-1, I-1-2, I-1-3, I-1-4, I-1-5, I-1-6, I-1-7, I-1-8, I-1-9, I-1-10, I-1-11, I-2-1, I-3-1, I-3-2, I-3-3, I-3-4, I-3-5, I-3-6, I-3-7, I-3-8, I-3-9, I-3-11, I-3-12, I-3-13, I-3-14, I-4-1, I-4-2, I-4-3, I-4-4, I-4-5, and I-4-6, resp., at 2500 ppm showed over 75% mortality in comparison with untreated controls.

### B.8 Green Peach Aphid (Myzus persicae)

For evaluating control of green peach aphid (Myzus persicae) through systemic means the test unit consisted of 96-well-microtiter plates containing liquid artificial diet under an artificial membrane.

The compounds were formulated using a solution containing 75% v/v water and 25% v/v DMSO. Different concentrations of formulated compounds were pipetted into the aphid diet, using a cus-tom built pipetter, at two replications.

After application, 5 - 8 adult aphids were placed on the artificial membrane inside the microtiter plate wells. The aphids were then allowed to suck on the treated aphid diet and incubated at about 23 + 1°C and about 50 + 5 % relative humidity for 3 days. Aphid mortality and fecundity was then visually assessed.

In this test, compounds I-1-1, I-1-2, I-1-3, I-1-4, I-1-5, I-1-6, I-1-7, I-1-8, I-1-9, I-1-10, I-1-11, I-2-1, 1-3-1, I-3-2, I-3-3, I-3-4, I-3-5, I-3-6, I-3-7, I-3-8, I-3-9, I-3-10, I-3-11, I-3-12, I-3-13, I-3-14, I-4-1, I-4-2, I-4-3, I-4-4, I-4-5, and I-4-6, resp., at 2500 ppm showed over 75% mortality in comparison with untreated controls.

### B.9 Rice green leafhopper (Nephotettix virescens)

Four to five-week old rice seedlings with cut upper leaf portion are cleaned and washed 24 hours before spraying. The active compounds are formulated in 1:1 acetone:water (vol:vol), and 0.01% vol/vol surfactant (Kinetic) is added. Potted rice seedlings are sprayed with 5-6 ml test solution, air dried, covered with Mylar cages and inoculated with 10 adults. Treated rice plants are kept at about 28-29°C and relative humidity of about 50-60%. Percent mortality is recorded after 72 hours.

In this test, compounds I-1-1, I-1-2, I-1-4, I-1-5, I-1-6, I-1-7, I-1-8, I-1-9, I-1-11, I-2-1, I-3-1, I-3-2, I-3-3, and I-3-5, resp., at 300 ppm showed over 75% mortality in comparison with untreated controls.

### B.10 Green Soldier Stink Bug (Nezara viridula)

The active compound is dissolved at the desired concentration in a mixture of 1:1 (vol:vol) distilled water: aceteone. Surfactant (Kinetic) is added at a rate of 0.01% (vol/vol).The test solution is prepared at the day of use.

Soybean pods are placed in 90 x 50 mm glass Petri dishes lined with moistened filter paper and inoculated with ten late 3rd instar N. viridula. Using a hand atomizer, an approximately 2 ml solution is sprayed into each Petri dish. Treated set-up is kept at about 25-26°C and relative humidity of about 65-70%. Percent mortality is recorded after 5 days.

In this test, compounds I-1-1, I-1-2, I-1-3, I-1-4, I-1-5, I-1-6, I-1-7, I-1-8, I-1-9, I-1-10, I-1-11, I-2-1, I-3-1, I-3-3, and I-3-5, resp., at 300 ppm showed over 75% mortality in comparison with untreated controls.

### B.11 Diamond back moth (Plutella xylostella)

The active compound is dissolved at the desired concentration in a mixture of 1:1 (vol:vol) distilled water: acetone. Surfactant (Kinetic) is added at a rate of 0.01% (vol/vol). The test solution is pre-pared at the day of use.

Cabbage leaf discs (60mm in diameter) are dipped in test solution and air-dried. Treated leaves are placed in petri dishes lined with moistened filter paper and inoculated with ten 3rd instar larvae. Mortality is recorded 72 hours after treatment. Feeding damage is also recorded using a scale of 0-100%.

In this test, compounds I-1-1, I-1-2, I-1-3, I-1-4, I-1-5, I-1-6, I-1-7, I-1-8, I-1-9, I-1-10, I-1-11, I-2-1, I-3-1, I-3-2, I-3-3, I-3-4, I-3-5, I-3-6, I-3-7, I-3-8, I-3-9, I-3-10, I-3-11, I-3-12, I-3-13, I-3-14, I-4-1, I-4-2, I-4-3, I-4-4, I-4-5 and I-4-6, resp., at 300 ppm showed over 75% mortality in comparison with untreated controls.

### B.12 Red spider Mite (Tetranychus kanzawai)

The active compound is dissolved at the desired concentration in a mixture of 1:1 (vol:vol) distilled water: acetone. Add surfactant (Kinetic) is added at a rate of 0.01% (vol/vol). The test solution is prepared at the day of use.

Potted cowpea beans of 4-5 days of age are cleaned with tap water and sprayed with 1-2 ml of the test solution using air driven DeVilbiss® hand atomizer at 20- 30 psi (≈ 1,38 to 2,07 bar). The treated plants are allowed to air dry and afterwards inoculated with 30 or more mites by clipping a cassava leaf section from rearing population. Treated plants are placed inside a holding room at about 25-26°C and about 65-70% relative humidity. Estimate percent mortality is assessed 72 hours after treatment.

In this test, compounds I-1-1, I-1-2, I-1-3, I-1-4, I-1-5, I-1-7, I-1-8, I-1-9, I-1-11, I-2-1, I-3-1, I-3-2, I-3-3, I-3-5, I-3-7, I-3-11, I-3-12, I-3-13, I-3-14, and I-4-4, resp., at 300 ppm showed over 75% mortality in comparison with untreated controls.

Further embodiments of the invention:
A method for combating or controlling invertebrate pests, which method comprises contacting said pest or its food supply, habitat or breeding grounds with a pesticidally effective amount of at least one compound as defined in any one of claims 1 to 12.

A method for protecting growing plants from attack or infestation by invertebrate pests, which method comprises contacting a plant, or soil or water in which the plant is growing, with a pesticidally effective amount of at least one compound as defined in any of claims 1 to 12.

Seed comprising a compound as defined in any of claims 1 to 12, or the enantiomers, diastereomers or salts thereof, in an amount of from 0.1 g to 10 kg per 100 kg of seed.

A method for treating or protecting an animal from infestation or infection by invertebrate pests which comprises bringing the animal in contact with a pesticidally effective amount of at least one compound of the formula I as defined in any of claims 1 to 12, a stereoisomer thereof and/or at least one veterinarily acceptable salt thereof.

## Claims

1. Compounds of formula I 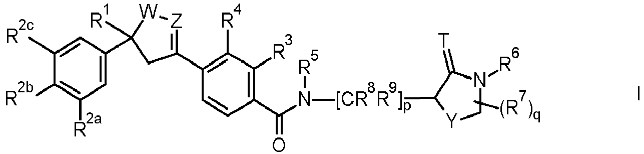 wherein
W-Z is -O-N=, -CH₂-N=, or -CH₂-CH=;
R¹ halomethyl;
R^{2a} halogen, halomethyl, or halomethoxy;
R^{2b}, R^{2c} are independently H, or as defined for R^{2a};
R³ is halogen, CN, NO₂, C₁-C₂-alkyl, halomethyl, C₁-C₂-alkoxy, S(O)ₘ-C₁-C₂-alkyl, C₁-C₂-haloalkoxy, or S(O)ₘ-C₁-C₂-haloalkyl;
R⁴ is H, or as defined for R³; or
R³ and R⁴ form together with the C-atoms they are bound to a 5-, or 6-membered saturated, partially, or fully unsaturated carbo- or heterocyclic ring;
R⁵,R⁶ are independently H, CN, C₁-C₁₀-alkyl, C₃-C₈-cycloalkyl, C₂-C₁₀-alkenyl, C₃-C₈-cycloalkenyl, C₂-C₁₀-alkynyl, OR¹⁰, S(O)ₘR¹⁰, N(R¹⁰)₂, which aliphatic groups are unsubstituted, partially or fully halogenated and/or substituted with one or more R^{a}; phenyl which is unsubstituted or substituted with one or more R^{A}; and 3- to 7-membered saturated, partially or fully unsaturated heterocycle comprising 1, 2 or 3 heteroatoms O, N(O)ₙ or S(O)ₘ as ring members, which heterocycle is unsubstituted or substituted with one or more R^{A},
R¹⁰ is independently H, C₁-C₆-alkyl, C₁-C₆-haloalkyl, C₃-C₈-cycloalkyl, C₃-C₈-cycloalkyl-C₁-C₄-alkyl, C₃-C₈-halocycloalkyl, C₂-C₆-alkenyl, C₂-C₆-haloalkenyl, C₂-C₆-alkynyl, C₂-C₆-haloalkynyl, which groups are unsubstituted or substituted with one or more R^{a},
R^{a} is CN, N3, NO₂, SCN, SF5, Si(C₁-C₄-alkyl)₃, OR^{a1}, OSO₂R^{a1}, S(O)ₙR^{a1}, N(R^{a2})R^{a3}, C(=O)N(R^{a2})R^{a3}, C(=S)N(R^{a2})R^{a3}, C(=O)R^{a1}, C(=O)OR^{a1}, CH=NOR^{a1}, C₃-C₈-cycloalkyl, C₃-C₈-halocycloalkyl, which cyclic moieties may be substituted with R^{a4}; phenyl which is unsubstituted or substituted with one or more R^{A}; and 3- to 7-membered saturated, partially or fully unsaturated heterocycle comprising 1, 2 or 3 heteroatoms O, N(O)ₙ or S(O)ₘ as ring members, which heterocycle is unsubstituted or substituted with one or more R^{A},
m is 0, 1, or 2;
n,p independently are 0, or 1;
R^{a1} H, C₁-C₆-alkyl, C₁-C₆-haloalkyl, C₂-C₄-alkenyl, C₂-C₄-alkynyl, CH₂-CN, C₃-C₆-cycloalkyl, C₃-C₆-halocycloalkyl, C₃-C₆-cycloalkylmethyl, C₃-C₆-halocycloalkylmethyl, phenyl and hetaryl which aromatic rings are unsubstituted or partially or fully substituted with R^{A};
R^{a2} is H, or C₁-C₆-alkyl,
R^{a3} is H, C₁-C₆-alkyl, C₁-C₆-haloalkyl, C₂-C₆-alkenyl, C₂-C₆-haloalkenyl, C₂-C₆-alkynyl, C₂-C₆-haloalkynyl, or C₃-C₆-cycloalkyl, C₃-C₆-halocycloalkyl, C₃-C₆-cycloalkylmethyl, or C₃-C₆-halocycloalkylmethyl which rings are unsubstituted or substituted with a cyano;
R^{a4} is independently OH, CN, C₁-C₆-alkoxy, C₁-C₆-haloalkoxy, S(O)ₘ-C₁-C₆-alkyl, S(O)ₘ-C₁-C₆-haloalkyl, C(=O)N(R^{a2})R^{a3}, C₃-C₆-cycloalkyl, or C₃-C₆-halocycloalkyl which cycles are unsubstituted or substituted with one or more R^{a11}; or phenyl, partially or fully unsaturated heterocycle which rings are unsubstituted or substituted with one or more R^{A};
R^{a11} is independently OH, cyano, C₁-C₂-alkyl, or C₁-C₂-haloalkyl;
R^{A} is independently selected from halogen, CN, NO₂, C₁-C₄-alkyl, C₁-C₄-haloalkyl, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, C₁-C₄-alkoxy, C₁-C₄-haloalkoxy, S(O)ₘ-C₁-C₄-alkyl, S(O)ₘ-C₁-C₄-haloalkyl, N(R^{a2})R^{a3}, N(Ra₂)C(=O)R^{a3}, C₁-C₄-alkylcarbonyl, C₁-C₄-haloalkylcarbonyl, C(=O)N(R^{a2})R^{a3}; or
two R^{A} present on the same carbon atom of a saturated or partially saturated ring may form together =O or =S; or
two R^{A} present on the same S or SO ring member of a heterocyclic ring may together form a group =N(C₁-C₆-alkyl), =NO(C₁-C₆-alkyl), =NN(H)(C₁-C₆-alkyl) or =NN(C₁-C₆-alkyl)₂;
T is O, or S;
Y is NR¹⁰, O, or S;
R⁷ independently from one another are halogen, CN, OH, C₁-C₆-alkyl, C₁-C₆-haloalkyl, C₁-C₄-alkoxy C₁-C₄-alkyl, C₁-C₄-alkoxycarbonyl, C₁-C₄-alkyl, C₁-C₆-alkoxy, C₁-C₆-alkylthio, C₁-C₆-alkylamino, di(C₁-C₄-alkyl)amino, C₁-C₆-alkoxycarbonyl, phenyl, or phenyl partially or fully substituted with R^{A};
two R⁷ present on the same carbon atom may together form a group =O, =S, =C(R^{a2})₂, =NH, =N(C₁-C₆-alkyl), =NO(C₁-C₆-alkyl), =NN(H)(C₁-C₆-alkyl), or =NN(C₁-C₆-alkyl)₂;
R⁸,R⁹ independently are as defined for R¹⁰;
q is 0, 1, 2, or 3;
and the N-oxides, stereoisomers and agriculturally or veterinarily acceptable salts thereof.

2. Compounds of formula I according to claim 1, which correspond to formula I.1.

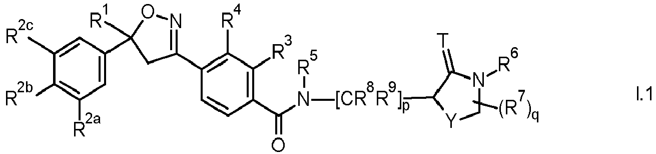

3. Compounds of formula I according to claim 1, which correspond to formula I.2

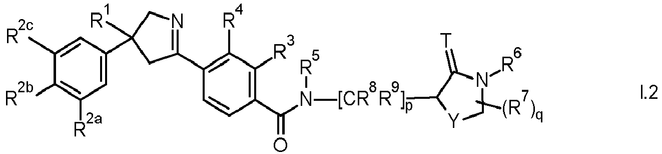

4. Compounds of formula I according to claim 1, which correspond to formula 1.3.

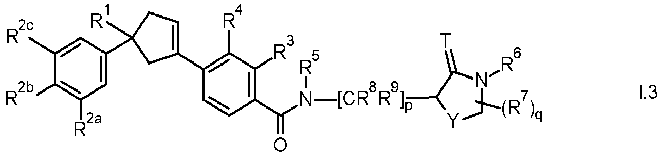

5. Compounds of formula I according to any one of claims 1 to 4, which correspond to formula I.a

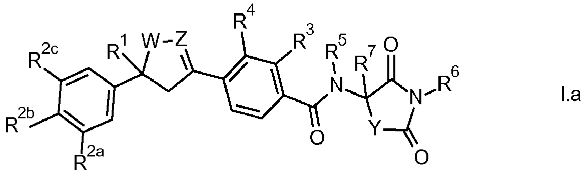

6. Compounds of formula I according to any one of claims 1 to 4, which correspond to formula I.b

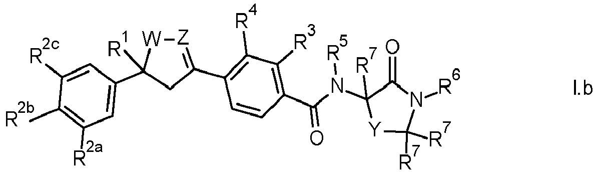

7. Compounds of formula I according to any one of claims 1 to 4, which correspond to formula I.c

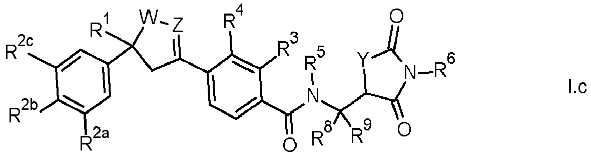

8. Compounds of formula I according to any one of claims 1 to 4, which correspond to formula I.d

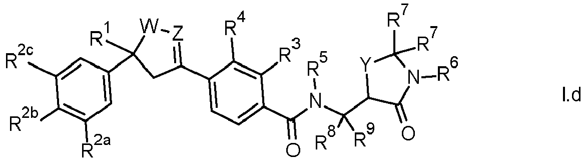

9. Compounds of formula I according to any of claims 1 to 8, wherein R¹ is CF₃.

10. Compounds of formula I according to any of claims 1 to 9, wherein R³ is halogen, NO₂, CN, CH₃, fluoromethyl, CF₃, SCH₃, or OCH₃, and R⁴ is H.

11. Compounds of formula I according to any of claims 1 to 10, wherein R⁵ is H, C₁-C₆-alkyl, C₃-C₆-cycloalkyl, C₂-C₆-alkenyl, C₂-C₆-alkynyl.

12. Compounds of formula I according to any of claims 1 to 11, wherein R⁶ is C₁-C₆-alkyl.

13. An agricultural or veterinary composition comprising at least one compound according to any one of claims 1 to 12 and/or at least one agriculturally or veterinarily acceptable salt thereof, and at least one inert liquid and/or solid agriculturally or veterinarily acceptable carrier.

14. An agricultural composition for combating animal pests comprising at least one compound as defined in any of claims 1 to 12 and at least one inert liquid and/or solid acceptable carrier and, if desired, at least one surfactant.

15. The composition according to claim 13 or 14, comprising additionally a further active substance.
